(19) Europäisches Patentamt
European Patent Office
Office européen des brevets

(11) **EP 2 701 734 B1**

(12) **EUROPEAN PATENT SPECIFICATION**

(45) Date of publication and mention of the grant of the patent:
**25.09.2019 Bulletin 2019/39**

(21) Application number: **12776493.4**

(22) Date of filing: **25.04.2012**

(51) Int Cl.:
***A61K 9/127*** *(2006.01)* ***A61K 39/00*** *(2006.01)*
***A61K 39/145*** *(2006.01)* ***A61K 45/06*** *(2006.01)*
***A61K 39/12*** *(2006.01)*

(86) International application number:
**PCT/US2012/035034**

(87) International publication number:
**WO 2012/149045 (01.11.2012 Gazette 2012/44)**

(54) **LIPOSOMAL FORMULATIONS**

LIPOSOMALE FORMULIERUNGEN

FORMULATIONS LIPOSOMALES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.04.2011 US 201161479302 P**

(43) Date of publication of application:
**05.03.2014 Bulletin 2014/10**

(73) Proprietor: **Molecular Express, Inc.**
**Rancho Dominguez, CA 90220 (US)**


(72) Inventors:
- **FUJII, Gary**
  **Rancho Palos Verdes, CA 90275 (US)**
- **ERNST, William, A.**
  **Rancho Dominguez, CA 90220 (US)**
- **ADLER-MOORE, Jill**
  **Rancho Dominguez, CA 90220 (US)**
- **VICIAN, Linda**
  **Rancho Dominguez, CA 90220 (US)**


(74) Representative: **Schiweck Weinzierl Koch Patentanwälte Partnerschaft mbB**
**Ganghoferstraße 68 B**
**80339 München (DE)**


(56) References cited:

| | |
|---|---|
| **WO-A2-00/16746** | **US-A1- 2006 134 103** |
| **US-A1- 2007 036 626** | **US-A1- 2010 203 099** |
| **US-A1- 2010 226 973** | **US-A1- 2010 226 973** |
| **US-B2- 7 368 537** | **US-B2- 7 368 537** |

- **FUJII GARY ET AL: "The VesiVax system: a method for rapid vaccine development", FRONTIERS IN BIOSCIENCE, vol. 13, January 2008 (2008-01), pages 1968-1980, XP009192074,**
- **ERNST W A ET AL: "Protection against H1, H5, H6 and H9 influenza A infection with liposomal matrix 2 epitope vaccines", VACCINE, ELSEVIER LTD, GB, vol. 24, no. 24, 12 June 2006 (2006-06-12) , pages 5158-5168, XP028010669, ISSN: 0264-410X, DOI: 10.1016/J.VACCINE.2006.04.008 [retrieved on 2006-06-12]**
- **OLSON K ET AL: "Liposomal gD ectodomain (gD1306) vaccine protects against HSV2 genital or rectal infection of female and male mice", VACCINE, ELSEVIER LTD, GB, vol. 28, no. 2, 11 December 2009 (2009-12-11), pages 548-560, XP026791394, ISSN: 0264-410X [retrieved on 2009-10-14]**
- **SHARPE HAYLEY J ET AL: "A Comprehensive Comparison of Transmembrane Domains Reveals Organelle-Specific Properties", CELL, vol. 142, no. 1, 9 July 2010 (2010-07-09), pages 158-169, XP028931092, ISSN: 0092-8674, DOI: 10.1016/J.CELL.2010.05.037**



Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **DATABASE BIOSIS [Online] BIOSCIENCES INFORMATION SERVICE, PHILADELPHIA, PA, US; 1997, WAHLBERG JOHANNA M ET AL: "Multiple determinants direct the orientation of signal-anchor proteins: The topogenic role of the hydrophobic signal domain", Database accession no. PREV199799562423 & WAHLBERG JOHANNA M ET AL: "Multiple determinants direct the orientation of signal-anchor proteins: The topogenic role of the hydrophobic signal domain", JOURNAL OF CELL BIOLOGY, vol. 137, no. 3, 1997, pages 555-562, ISSN: 0021-9525**
- **J. P. ADLER-MOORE ET AL: "Monomeric M2e antigen in VesiVax liposomes stimulates protection against type a strains of influenza comparable to liposomes with multimeric forms of M2e", JOURNAL OF LIPOSOME RESEARCH., vol. 27, no. 3, 3 July 2017 (2017-07-03), pages 210-220, US ISSN: 0898-2104, DOI: 10.1080/08982104.2017.1381708**

## Description

STATEMENT AS TO FEDERALLY SPONSORED RESEARCH

The U.S. Government has a paid-up license in this invention and the right in limited circumstances to require the patent owner to license others on reasonable terms as provided for by the terms of National Institute of Allergy and Infectious Diseases (NIH) Grant Nos. U01AI074508 and 1R43AI078654.

The following discussion of the background of the invention is merely provided to aid the reader in understanding the invention and is not admitted to describe or constitute prior art to the present invention.

Liposomes are vesicles formed from one ("unilamellar") or more ("multilamellar") layers of phospholipid. Because of the amphipathic character of the phospholipid building blocks, liposomes typically comprise a hydrophilic layer presenting a hydrophilic external face and enclosing a hydrophilic core. The versatility of liposomes in the incorporation of hydrophilic/hydrophobic components, their non-toxic nature, biodegradability, biocompatibility, adjuvanticity, induction of cellular immunity, property of sustained release and prompt uptake by macrophages, makes them attractive candidates for the delivery of antigens.

Liposomes have been demonstrated to induce both humoral and cell-mediated immunity to a large variety of bacterial, protozoan, viral and tumour cell antigens. While the widespread use of liposomal vaccines has been long anticipated, few such vaccines have been developed commercially. The immunoadjuvant action of liposomes depends on various structural characteristics. Such characteristics include the three-dimensional conformation of the antigen being presented by the liposome, which may not always mimic the natural conformation of the antigen.

For example, the membrane proximal region (MPR) of HIV gp41, a segment comprised of approximately 35 amino acids N terminal to the transmembrane domain, has been considered a desirable vaccine target because it is well conserved across viral clades and is essential for virus-cell fusion. However, efforts to date have not succeeded in eliciting a useful immune response, and attempts to present structurally constrained epitopes, either conjugated to carrier proteins or grafted on recombinant constructs, have not elicited neutralizing antibodies. In addition to a lack of consensus regarding the epitope structure, the relatively weak immunogenicity of the MPR may result in immune responses to recombinant envelope immunogens directed toward immunodominant regions on gp41 that mask the MPR from antibody recognition.

In addition, methods for associating an antigen with a liposome prior to liposome formation often expose the antigen to detergents and/or organic solvents. In contrast, methods for associating an antigen with a liposome following formation can expose the liposome to unfavorable chemical treatments. Liposomes may also be quickly cleared by the reticuloendothelial system and macrophages, reducing the efficiency of the liposome as a vaccine.

In addition, such characteristics may also include factors which control vesicle fate *in vivo.* Methods for associating an antigen with a liposome prior to liposome formation often expose the antigen to detergents and/or organic solvents. In contrast, methods for associating an antigen with a liposome following formation can expose the liposome to unfavorable chemical treatments. Liposomes may be quickly cleared by the reticuloendothelial system and macrophages, reducing the efficiency of the liposome as a vaccine.

WO 00/16746 describes an immunogenic liposome composition comprising vesicle forming lipids and an antigenic construct comprising one or more antigenic determinants and a hydrophobic domain (HD). Fujii et al 2008, Frontiers in Bioscience, 13:1968-1980 describe the VesiVax system comprising an aqueous soluble hydrophobic domain (HD) and a stably inserted immunogen (e.g. epitopes from HSV2, M2 ectodomain of influenza virus fused to an HD segment). Ernst et al. 2006, Vaccine 24(24):5158-5168 describe the vector pET28a containing the HD gene fused to the M2e peptide. Olson et al. 2009, Vaccine 28(2):548-560 describe a liposome comprising HSV2 gD-HD protein. Sharpe et al. 2010, Cell 142(1):158-169 describe numerous transmembrane domains. US 2010/226973 describes compositions comprising (a) an aqueous vehicle, (b) liposomes comprising (i) DPMC, (ii) DMPG, DTMAP or both, (iii) at least one sterol derivative (e.g. cholesterol) and (c) an immunogenic polypeptide covalently linked to between 1% and 100% of the sterol derivative. US 7368537 describes immunogens comprising a RSV G protein fused to a hydrophobic portion or moiety.

There remains in the art a need for methods and compositions which can provide liposomal vaccines that deliver antigens in a manner useful for stimulating an immune response.

BRIEF SUMMARY OF THE INVENTION

It is an object of the invention to provide liposomal compositions, methods for the manufacture thereof, and methods for the use thereof. Such compositions may be vaccine formulations for use in methods to stimulate an immune response in an animal.

The invention is defined by the appended claims.

Accordingly, the present invention relates to a composition comprising: an aqueous vehicle; and liposomes

comprising (i) dimyristoylphosphatidylcholine ("DMPC"), (ii) dimyristoylphosphatidylglycerol ("DMPG"), or dimyristoyltrimethylammonium propane ("DMTAP"), or both DMPG and DMTAP, (iii) at least one sterol; and (iv) an antigenic polypeptide comprising a first polypeptide sequence comprising at least 10 contiguous residues of an influenza A M2 extracellular domain, the sequence of which has been modified to remove one or more cysteine residues naturally occurring in said M2 extracellular domain, and a second polypeptide sequence comprising a transmembrane sequence of, or derived from, a naturally occurring heterologous membrane protein, said second polypeptide sequence having a number of residues sufficient to cross a lipid bilayer, at least nine contiguous residues of which are predicted to form an alpha helix having a hydrophobicity score of 0.63 or greater, wherein the hydrophobicity score is calculated by the methodology of Eisenberg, wherein said first polypeptide sequence is coupled directly or indirectly to the N- or C- terminus of said second polypeptide sequence to provide an aqueous soluble fusion polypeptide, and wherein the antigenic polypeptide sequence of the fusion polypeptide is displayed on the outer surface of the liposomes.

In one aspect, the disclosure relates to a composition comprising:

a) an aqueous vehicle; and
b) liposomes comprising

(i) dimyristoylphosphatidylcholine ("DMPC"),

(ii) dimyristoylphosphatidylglycerol ("DMPG"), dimyristoyltrimethylammonium propane ("DMTAP"), or both DMPG and DMTAP,

(iii) at least one sterol; and

iv) an antigenic polypeptide sequence, and

a second polypeptide sequence comprising a transmembrane sequence of, or derived from, a naturally occurring heterologous membrane protein, said second polypeptide sequence having a number of residues sufficient to cross a lipid bilayer, at least nine contiguous residues of which are predicted to form an alpha helix having a hydrophobicity score of about 0.7 or greater,
wherein said antigenic polypeptide sequence is coupled directly or indirectly to the N- or C- terminus of said second polypeptide sequence to provide an aqueous soluble fusion polypeptide, and wherein the antigenic polypeptide sequence of the fusion polypeptide is displayed on the outer surface of the liposomes.

The term "about in this context refers to +/- 10% of a given measurement.

For purposes of the invention, alpha helicity and hydrophobicity of a polypeptide sequence is calculated using the methodology of Eisenberg et al., J. Mol. Biol. (1984) 179:125-142, using the following parameters: a window size of 9, relative weight of edges 100%, a linear weight variation, and amino acid scale values as follows: Ala: 0.620 Arg: -2.530 Asn: -0.780 Asp: -0.900 Cys: 0.290 Gln: -0.850 Glu: -0.740 Gly: 0.480 His: -0.400 Ile: 1.380 Leu: 1.060 Lys: -1.500 Met: 0.640 Phe: 1.190 Pro: 0.120 Ser: -0.180 Thr: -0.050 Trp: 0.810 Tyr: 0.260 Val: 1.080.

As noted, suitable hydrophobic sequences typically have a sufficient length to cross a lipid bilayer (from about 15-30 residues; more preferably from about 18-25 residues), at least 9 contiguous residues of which are predicted to form an alpha helix having a hydrophobicity (Eisenberg score) of about + 0.7.

In certain aspects of the foregoing, the disclosure relates to a composition comprising:

a) an aqueous vehicle; and

b) liposomes comprising

(i) dimyristoylphosphatidylcholine ("DMPC"),

(ii) dimyristoylphosphatidylglycerol ("DMPG"), dimyristoyltrimethylammonium propane ("DMTAP"), or both DMPG and DMTAP,

(iii) at least one sterol; and

iv) an antigenic polypeptide comprising a first polypeptide sequence comprising at least 10 contiguous residues of an M2 extracellular domain, the sequence of which has been modified to remove one or more cysteine residues naturally occurring in said M2 extracellular domain, and

a second polypeptide sequence comprising a transmembrane sequence of, or derived from, a naturally occurring heterologous membrane protein, said second polypeptide sequence having a number of residues sufficient to cross a lipid bilayer, at least nine contiguous residues of which are predicted to form an alpha helix having a hydrophobicity score of about 0.7 or greater,
wherein said antigenic polypeptide sequence is coupled directly or indirectly to the N- or C- terminus of said second polypeptide sequence to provide an aqueous soluble fusion polypeptide, and wherein the antigenic polypeptide sequence of the fusion polypeptide is displayed on the outer surface of the liposomes.

The term "M2 extracellular domain" as used herein refers to a polypeptide derived from the IAV M2 protein which contains at least 10 amino acid residues from the extracellular domain of a native or consensus M2 protein sequence, and which lacks all or a portion of the M2 protein transmembrane domain, whereby the resulting M2 extracellular domain polypeptide is soluble in aqueous media.

Examples of native IAV M2 protein sequences include:

Swiss-Prot Q0A2E4 (SEQ ID NO: 1):

```
        10         20         30         40         50         60
MSLLTEVETP TRNGWECKCS DSSDPLVIAA SIIGILHLIL CILDRLFFKC IYRRLKYGLK

        70         80         90
RGPSTEGVPE SMREEYRQEQ QSAVDVDDGH FVNIELE
```

Swiss-Prot P63231 (SEQ ID NO: 2):

```
        10         20         30         40         50         60
MSLLTEVETP IRNEWGCRCN DSSDPLVVAA SIIGILHLIL WILDRLFFKC IYRFFEHGLK

        70         80         90
RGPSTEGVPE SMREEYRKEQ QSAVDADDSH FVSIELE
```

Swiss-Prot P06821 (SEQ ID NO: 3):

```
        10         20         30         40         50         60
MSLLTEVETP IRNEWGCRCN GSSDPLAIAA NIIGILHLIL WILDRLFFKC IYRRFKYGLK

        70         80         90
GGPSTEGVPK SMREEYRKEQ QSAVDADDGH FVSIELE
```

Swiss-Prot P05780 (SEQ ID NO: 4):

```
        10         20         30         40         50         60
MSLLTEVETP IRNEWGCRCN DSSDPLVIAA NIIGILHLIL WILDRLFFKC IYRRFKYGLK

        70         80         90
RGPSTEGVPE SMREEYRKEQ QNAVDVDDGH FVNIELE
```

Swiss-Prot P35938 (SEQ ID NO: 5):

```
      10         20         30         40         50          60
MSLLTEVETP IRNEWGCRCN DSSDPLVVAA SIIGILHLIL WILDRLFFKC IYRLFKHGLK

        70         80         90
RGPSTEGVPE SMREEYRKEQ QNAVDADDSH FVNIELE
```

Swiss-Prot P05778 (SEQ ID NO: 6):

```
        10         20         30         40         50         60
MSLLTEVETP TRNGWECSCS DSSDPLVIAA SIIGILHFIL WILDRLFFKC IYRRLKYGLK

        70         80         90
RGPSTEGVPK SMREEYRQEQ QNAVDVDDGH FVNIELE
```

Swiss-Prot P03492 (SEQ ID NO: 7):

```
        10         20         30         40         50         60
MSLLTEVETP TRNGWECRCN DSSDPLIIAA SIIGILHLIL WILNRLFFKC IYRRLKYGLK

        70         80         90
RGPSTEGVPE SMREEYRQEQ QSAVDVDDGH FVNIELE
```

Swiss-Prot O70632 (SEQ ID NO: 8):

```
        10         20         30         40         50         60
MSLLTEVETL TRNGWGCRCS DSSDPLVVAA SIIGILHLIL WILDRLFFKC IYRRFKYGLK

        70         80         90
RGPSTEGVPE SMREEYRQEQ QNAVDVDDGH FVNIELE
```

Swiss-Prot P63232 (SEQ ID NO: 9):

```
        10         20         30         40         50         60
MSLLTEVETP IRNEWGCRCN DSSDPLVVAA SIIGILHLIL WILDRLFFKC IYRFFEHGLK

        70         80         90
RGPSTEGVPE SMREEYRKEQ QSAVDADDSH FVSIELE
```

In these sequences, residues 2 - 22 represent the extracellular domain (the initiator methionine is typically removed); residues 23 - 43 represent the ransmembrane domain; and residues 44 - 97 represent the cytoplasmic domain.

Consensus IAV M2 extracellular domain sequences have been determined by correlation of numerous IAV isolates. Three consensus sequences for different species-derived viral isolates are described by Betakova, Current Pharmaceutical Design, 2007, 13, 3231-3235:

| | | |
|---|---|---|
| Human | (SEQ ID NO: 10): | SLLTEVETPIRNEWGCRCNDSSD |
| Swine | (SEQ ID NO: 11): | SLLTEVETPIRNGWECKCNDSSD |
| Avian | (SEQ ID NO: 12): | SLLTEVETPTRNGWECKCSDSSD |

In certain embodiments, the cysteine residues are removed from these sequences. This may be done by mutation of the cysteine residues to another residue (e.g., serine), or by truncation of the extracellular domain prior to the cysteine residues. In preferred embodiments, the IAV M2 extracellular domain sequences finding use in the antigenic polypeptides described herein comprises at least 10 contiguous residues, and most preferably all 15 residues from one or more of the following sequences:

| | | |
|---|---|---|
| Human | (SEQ ID NO: 13): | SLLTEVETPIRNEWG; |
| Swine | (SEQ ID NO: 14): | SLLTEVETPIRNGWE; or |
| Avian | (SEQ ID NO: 15): | SLLTEVETPTRNGWE |

In one example of such a preferred IAV M2 extracellular domain sequence, in which cysteines have been mutated to serine, an IAV M2 extracellular domain sequence finding use in the antigenic polypeptides described herein comprises:
(SEQ ID NO: 16): SLLTEVETPIRNEWGSRSNDSSD

Examples of HSV2 antigenic polypeptide sequences include the following (SEQ ID NOS: 60-81):

| Protein name (residues) | (gene name) | Sequence |
|---|---|---|
| Tegument host shutoff protein (261-270) | (UL41) | HTDLHPNNTY |
| Tegument protein VP13/14 (363-371) | (UL47) | RSSLGSLLY |
| Ribonucleotide reductase subunit 1 (517-525) | (UL39) | YMESVFQMY |
| Tegument protein VP13/14 (289-298) | (UL47) | FLVDAIVRVA |
| Envelope glycoprotein B (443-451) | (UL27) | FLIAYQPLL |
| DNA packaging tegument protein (372-380) | (UL25) | FLWEDQTLL |
| Ribonucleotide reductase subunit 2 (181-89) | (UL40) | ILIEGIFFA |
| Ribonucleotide reductase subunit 1 (430-438) | (UL39) | RILGVLVHL |
| Envelope glycoprotein B (275-283) | (UL27) | SVYPYDEFV |
| Nuclear egress lamina protein (291-299) | (UL31) | EYQRLYATF |
| Multifunctional expression regulator (504-512) | (UL54) | KYFYCNSLF |
| Transcriptional regulator (1243-1252) | (ICP4) | LYPDAPPLRL |
| Tegument protein VP13/14 (363-371) | (UL47) | ALATVTLKY |
| Infected Cell Protein 8 (460-468) | (UL29) | ALLAKMLFY |
| Tegument protein VP11/12 (224-234) | (UL46) | LLAYVSVLY |
| Tegument protein VP11/12 (333-341) | (UL46) | SIVHHHAQY |
| DNA packaging tegument protein (175-184) | (UL25) | SSGVVFGTWY |
| Envelope glycoprotein B (290-298) | (UL27) | VYMSPFYGY |
| Single-stranded DNA-binding protein (895-903) | (UL29) | YMANQILRY |
| Capsid maturation protease (22-30) | (UL26) | YVAGFLALY |
| Envelope glycoprotein I (97-105) | (US7) | CPRRPAVAF |
| Envelope glycoprotein I (22-30) | (US7) | VVRGPTVSL |

A polypeptide sequence is "derived" from a particular naturally occurring protein sequence if it is at least 90% identical, more preferably 95% identical, and most preferably at least 97% identical, to the sequence of the naturally occurring protein.

For purposes of the invention, a transmembrane sequence is "heterologous" if the sequence is obtained or derived from a naturally occurring protein that is different from the protein from which the antigenic polypeptide sequence is obtained or derived. Exemplary transmembrane domains of naturally occurring proteins finding use in the present invention are described hereinafter.

For the sake of convenience, the lipid(s) selected in part (ii) above will be referred to below as DMPG/DMTAP, which is intended to mean DMPG, DMTAP, or a mixture of the two. In certain embodiments, the relative percentages of DMPC, DMPG/DMTAP, and sterol are 50% to 98% DMPC : 1% to 25% DMPG/DMTAP : 1% to 25% sterol, and in certain other embodiments 70% to 98% DMPC : 1% to 15% DMPG/DMTAP : 1% to 15% sterol. This is not meant to imply that no other components are present in the liposome; rather, these represent the relative percentages of DMPC, DMPG/DMTAP, and sterol on a molar basis to one another. In certain embodiments, a liposome can also contain one or more additional components which are well known in the art, such as peptidoglycan, lipopeptide, lipopolysaccharide, monophosphoryl lipid A, lipoteichoic acid, resiquimod, imiquimod, flagellin, oligonucleotides containing unmethylated CpG motifs, α-galactosylceramide, muramyl dipeptide, all-trans retinoic acid, double-stranded viral RNA, heat shock proteins, dioctadecyldimethylammonium bromide, cationic surfactants, toll-like receptor agonists, dimyristoyltrimethylammoniumpropane, and nod-like receptor agonists.

In preferred embodiments, these relative percentages are 70% to 85% DMPC : 5% to 15% DMPG/DMTAP : 10% to 15% sterol, and more preferably about 75% DMPC, about 10% DMPG/DMTAP, and about 15% sterol. The term "about" as used herein in this context refers to +/- 10% of a given measurement. DMPG is particularly preferred as the lipid selected in part (ii) above.

The term "sterol" as used herein refers to any molecule having the 4-member ring structure characteristic of steroids and a hydroxyl (-OH) or ester (-OR) substitution at the 3-carbon position:

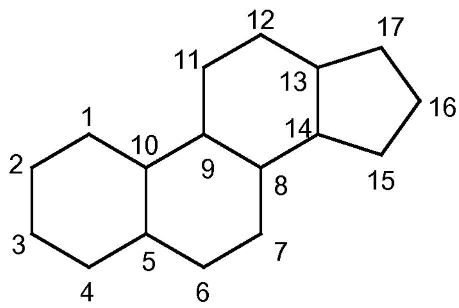

The skilled artisan will understand that a sterol can be further substituted at one or more of the other ring carbons, and may also contain various double bonds in the rings. In certain embodiments, a sterol is selected from the group consisting of cholesterol, cholesteryl chloroformate, stigmasterol, sitosterol, ergosterol, lanosterol, desmosterol, and campesterol.

As described herein, advantageously the antigenic polypeptide is prepared as a fusion protein which is soluble in aqueous solution despite the presence of a hydrophobic transmembrane domain. The provision of an aqueous soluble antigenic construct (1) enables the construct to be produced separately from the liposomes using aqueous systems for expression from an organism as a fusion protein or conventional aqueous synthetic chemical methods; and (2) enables subsequent standard aqueous purification procedures. See WO00/016746.

For purposes of the invention, two polypeptides are "directly coupled" to one another if there are no intervening chemical bonds between the peptide bond between the two polypeptides, while two polypeptides are "indirectly coupled" if a linking chemical structure connects the two polypeptides. An example of an indirect coupling could be one or more amino acid residues, a disulfide linkage, or a bifunctional chemical crosslinking reagent. Methods for covalently coupling two polypeptide sequences are well known in the art. For example, the antigenic polypeptide may be synthesized or expressed as a single "fusion" protein comprising both the M2 extracellular domain residues and the transmembrane domain residues using standard molecular biological techniques. In the case where the M2 extracellular domain residues and the transmembrane domain residues are synthesized or expressed as separate molecules, chemical cross-linkers, such as those discussed in Wong, Chemistry of Protein Conjugation and Cross-linking, CRC Press, Boca Raton, Fla., 1991, may be employed. These reagents typically provide functional groups that couple to amino acid side chains of peptides. Moieties that can be targeted using a cross-linker include primary and ε- amines, sulfhydryls, carbonyls, hydroxyls, and carboxylic acids. In addition, many reactive groups can be coupled nonselectively using a cross-linker such as photoreactive phenyl azides. The two sequences may directly coupled end-to-end, or additional linking atoms or amino acid residues may be included between the two polypeptide sequences of interest.

Suitable methods for preparing liposomes from lipid mixtures are well known in the art. *See, e.g.*, Basu & Basu, Liposome Methods and Protocols (Methods in Molecular Biology), Humana Press, 2002; Gregoriadis, Liposome Technology, 3rd Edition, Informa HealthCare, 2006. Preferred methods include extrusion, homogenization, and sonication methods described therein. An exemplary method for preparing liposomes of the invention, which comprises drying a lipid mixture, followed by hydration in an aqueous vehicle and sonication to form liposomes, is described hereinafter. Preferred steroid derivatives, methods for covalently coupling immunogenic polypeptides or carbohydrates to such derivatives, and covalent linkages are discussed in detail above and hereinafter.

In certain embodiments, the liposome are provided within a particular average size range, as size can affect the efficiency with which liposomes are taken up when delivered mucosally, and/or cleared when delivered intravenously. Liposome size can be determined by methods well known in the art, including photon correlation spectroscopy, dynamic light scattering. In preferred embodiments, the liposomes are substantially between 50 and 500 nm in diameter, more preferably substantially between 50 and 300 nm in diameter. The term "substantially" as used herein in this context means that at least 75%, more preferably 80%, and most preferably at least 90% of the liposomes are within the designated range.

In certain embodiments, the relative percentages of DMPC, DMPG/DMTAP, and sterol are 50% to 98% DMPC : 1% to 25% DMPG/DMTAP : 1% to 25% sterol, and in certain other embodiments 70% to 98% DMPC : 1% to 15% DMPG/DMTAP : 1% to 15% sterol. As discussed above, this is not meant to imply that no other components are present in the lipid mixture (and hence in the liposomes); rather, these represent the relative percentages of DMPC, DMPG/DMTAP, and sterol on a molar basis to one another. In certain embodiments, a lipid mixture can also contain one or more additional components which are well known in the art, such as peptidoglycan, lipopeptide, lipopolysaccharide, monophosphoryl lipid A, lipoteichoic acid, resiquimod, imiquimod, flagellin, oligonucleotides containing unmethylated CpG motifs, α-galactosylceramide, muramyl dipeptide, all-trans retinoic acid, double-stranded viral RNA, heat shock proteins, dioctadecyldimethylammonium bromide, cationic surfactants, toll-like receptor agonists, dimyristoyltrimethylammoniumpropane, and nod-like receptor agonists.

In preferred embodiments, these relative percentages are 70% to 85% DMPC : 5% to 15% DMPG/DMTAP : 10% to 15% sterol, and more preferably about 75% DMPC, about 10% DMPG/DMTAP, and about 15% sterol. The term "about" as used herein in this context refers to +/- 10% of a given measurement. DMPG is particularly preferred as the lipid selected in part (ii) above.

Preferred concentrations of antigenic polypeptide in the final composition are in the range of about 0.05 to 1 mg/mL, and most preferably from about 0.1 to 0.6 mg/mL. The term "about" as used herein in this context refers to +/- 10% of a given measurement.

In certain embodiments, methods further comprise selecting liposomes within a particular average size range. Liposome size can be selected, for example, by extrusion of an aqueous vehicle comprising liposomes through membranes having a preselected pore size and collecting the material flowing through the membrane. In preferred embodiments, the liposomes are selected to be substantially between 50 and 500 nm in diameter, more preferably substantially between 50 and 200 nm in diameter, and most preferably substantially between 50 and 150 nm in diameter. The term "substantially" as used herein in this context means that at least 75%, more preferably 80%, and most preferably at least 90% of the liposomes are within the designated range.

In certain embodiments, the transmembrane sequence is obtained or derived from a heterologous membrane protein which in its native context has a signal-anchor sequence. As used herein, this refers to a polypeptide sequence comprising at least 10 contiguous residues (in certain embodiments at least 15 contiguous residues, in still other embodiments at least 18 contiguous residues, in yet other embodiments at least 20 contiguous residues, and in some embodiments 25 or more contiguous residues) from the membrane-spanning domain of a protein such as a type II or type III membrane protein. Such sequences typically have a sufficient length to cross a lipid bilayer (from about 15-30 residues; more preferably from about 18-25 residues) and are predicted to form an alpha helix having a hydrophobicity (Eisenberg et al. score) of about + 1.5 / residue. The term "about in this context refers to +/- 10% of a given measurement. Type II and type III membrane proteins lack a cleavable N-terminal signal peptide controlling membrane insertion and topology, and instead insert into membranes posttranslationally via the hydrophobic "signal-anchor sequence" which is an uncleavable signal sequence that mediates the translocation of the polypeptide and anchors the protein in the membrane.

One example of such a protein is cytochrome b5. The cytochrome b5 polypeptide consists of two distinct domains linked by a trypsin-sensitive segment. The amino-proximal domain is the catalytic domain, involved in electron transport, and the hydrophobic "carboxyl-proximal" domain serves to anchor the protein in the membrane. Other suitable single-pass membrane proteins known in the art include Mucin-4 beta chain, transferrin receptor, TNFRSF13B, Aminopeptidase N, Dipeptidyl peptidase 4, Tumor necrosis factorsyntaxin 3, BclXL, and R9AP.

In certain alternative embodiments, the transmembrane sequence is obtained or derived from a heterologous membrane protein which is a multi-pass membrane protein having multiple signal-anchor sequences, such as bacteriorhodopsin, the G protein-coupled receptors such as CCRs1-10, CXCR1, acetylcholine receptor, adrenomedullin receptor, cytochrome C oxidase, nicotinic acetylcholine receptor, the mGluRs and aquaporin.

Examples of suitable transmembrane domains comprise 10 or more contiguous residues from one or more of the following sequences. Numerous other such domains are well known in the art, and sequences may be predicted to form a transmembrane domain as calculated using the DAS server (Cserzo, M., E. Wallin, I. Simon, G. von Heijne, and A. Elofsson. 1997. Protein Eng. 10:673-676).

| | |
|---|---|
| R9AP TM (SEQ ID NO: 17): | LIVSLLLCGTALVAITL |
| mGluR1 TM (SEQ ID NO: 18): | IIAIAFSCLGILVTLFVTLIFVLY |
| Cyt b5a TM (SEQ ID NO: 19): | VIPAISAVAVALMY |
| Cyt b5a TM bov (SEQ ID NO: 20): | LIPAISALFVALIY |
| Cyt b5a TM sus (SEQ ID NO: 21): | VIPAISALVVSLMY |
| Cyt b5a TM xen (SEQ ID NO: 22): | LIPAAAVVLLGFMY |
| BclXL TM (SEQ ID NO: 23): | TGMTVAGVVLLGS |
| Stx 3 TM (SEQ ID NO: 24): | IMILICCVILAIVIASTI |
| MGC80327 xen TM (SEQ ID NO: 25): | IIPGISAMIVALMY |

Taking Cyt b5a sequence as an exemplary hydrophobic transmembrane domain, an antigenic fusion protein of the present invention can comprise all, or any contiguous portion, of one of the following sequences, with the proviso that the sequence comprises at least the underlined residues:

Homo sapiens (Swiss-Prot P00167) (SEQ ID NO: 26) NKPPETLITTIDSSSSWWTNWVIPAISAVAVALMYRLYMAED

Ratus norvegicus (Swiss-Prot P00173) (SEQ ID NO: 27) AKPSETLITTVESNSSWWTNWVIPAISALVVALMYRLYMAED

Mus musculus (Swiss-Prot P56395) (SEQ ID NO: 28) AKPSDTLITTVESNSSWWTNWVIPAISALAVALMYRLYMAED

Oryctolagus cuniculus (Swiss-Prot P00169) (SEQ ID NO: 29) SKPMETLITTVDSNSSWWTNWVIPAISALIVALMYRLYMADD

Gallus gallus (Swiss-Prot P00174) (SEQ ID NO: 30) QKPAETLITTVQSNSSSWSNWVIPAIAAIIVALMYRSYMSE

Sus scrufus (Swiss-Prot P00172) (SEQ ID NO: 31) AKPSETLITTVESNSSWWTNWVIPAISALVVSLMYHFYTSEN

Equus Caballus (Swiss-Prot P00170) (SEQ ID NO: 32) AKPVETLITTVDSNSSWWTNWVIPAISAVVVALMYRIYTAED

Mesocricetus auratus (Swiss-Prot P70116) (SEQ ID NO: 33) AKPSESLITTVESNSSWWTNWVIPAVSALAVALMYRLYMGRR

Ovis aires (Swiss-Prot C9E8M7) (SEQ ID NO: 34) TKPSESIITTIDSNSSWWTNWLIPAISALVVALMYHLYTSEN

Xenopus laevis (Swiss-Prot Q28EA4) (SEQ ID NO: 35) KNQGKNDVLLTTSSSSSSWSSWLIPAAAVVLLGFMYRFYMVD

Bombyx mori (Swiss-Prot Q2F5S4) (SEQ ID NO: 36) QYSWEDTAKTSETEASFVNSWKFPVLLGLALTLLYSYIFG

Olea europaea (Swiss-Prot 024651) (SEQ ID NO: 37) KQPHYNQDKTSDFIIKLLQFLVPLFILGVAVGIHFYTKSSA

Rhodopseudomonas palustris (Swiss-Prot Q07P44) (SEQ ID NO: 38) WCGKEATEAYATKTKGRAHTREADELLPKYRIGRFAP

Xenopus tropicalis (Swiss-Prot Q28EA4) (SEQ ID NO: 39) QKPTETFITTTDSDSSWWSNWIIPGISAFIVALMYRFYMASE

Polyandrocarpa misakiensis (Swiss-Prot Q9GV21) (SEQ ID NO: 40) QEEQPQFVTTHESMAETSSWSNWVIPAIVALAVALVYRYYISN

Other similar proteins are also well known in the art. Preferably, such a polypeptide sequence comprises at least 70% sequence identity to one of the cyt b5 protein sequences listed above by Swiss-Prot accession number, and preferably at least 70%, more preferably at least 80%, and most preferably at least 90%, sequence identity to the human sequence recited above as SEQ ID NO: 26 across the entire 42-residue length recited above. Sequence identity is determined using Basic Local Alignment Search Tool (BLAST) (Altschul, S.F. et al. (1990) J. Mol. Biol. 215:403-410), set at default parameters. It is understood that changes in a nucleic acid sequence can be made using degeneracy of the genetic code to produce multiple nucleic acid sequences that all encode substantially the same protein. By way of example, an antigenic fusion protein of the present invention can comprise all, or any contiguous portion, of the following sequences, with the proviso that the sequence comprises at least the underlined residues:

Xenopus laevis MGC80327 protein (Swiss-Prot Q6DE13) (SEQ ID NO: 41)
QKPSETFITTTDSDSSWWSNWIIPGISAMIVALMYRFYMVSE
Macaca mulatta EMBL:CO646551 (SEQ ID NO: 42) SKPPETLITTVDSSSSWWTNWVIPAISAVAVALMYRLYMAED

In related aspects, the present disclosure relates to a purified antigenic polypeptide comprising: a first polypeptide sequence selected to establish a humoral and/or cell-mediated immune response in an animal, the first polypeptide sequence coupled directly or indirectly to the N- or C- terminus of a second polypeptide sequence comprising a transmembrane sequence of, or derived from, a naturally occurring heterologous membrane protein, said second polypeptide sequence having a number of residues sufficient to cross a lipid bilayer, at least nine contiguous residues of which are predicted to form an alpha helix having a hydrophobicity score of about 0.7 or greater, wherein said purified antigenic polypeptide sequence is aqueous soluble.

In certain exemplary aspects, the present disclosure provides purified antigenic polypeptides comprising a first polypeptide sequence comprising at least 10 contiguous residues of an M2 extracellular domain, the sequence of which has been modified to remove one or more cysteine residues naturally occurring in said M2 extracellular domain, optionally comprising a second polypeptide sequence comprising a transmembrane sequence of, or derived from, a naturally occurring heterologous membrane protein, said second polypeptide sequence having a number of residues sufficient to cross a lipid bilayer, at least nine contiguous residues of which are predicted to form an alpha helix having a hydrophobicity score of about 0.7 or greater, wherein said purified antigenic polypeptide sequence is aqueous soluble, wherein said first polypeptide sequence is coupled directly or indirectly to the N- or C- terminus of said second polypeptide sequence.

In other aspects, the invention relates the composition of the present invention for use in methods for immunizing an animal. These methods comprise delivering to said animal by a parenteral or enteral route an effective amount of a composition of the present invention. Preferred materials, methods, and conditions for making such compositions are discussed in detail above and hereinafter.

Preferred enteral routes of administration include delivery by mouth (oral), nasal, rectal, and vaginal routes. Preferred parenteral routes of administration include intravenous, intramuscular, subcutaneous, and intraperitoneal routes.

In certain embodiments, the composition of the present invention for use in the methods of the present invention comprise multiple deliveries of the composition of the present invention, commonly referred to as "prime/boost" immunization protocol. In preferred embodiments, one or more of the prime and boost deliveries comprises delivering to the animal by a parenteral or enteral route a liposomal composition of the present invention. In such immunization protocols, a priming delivery may be via a different route of administration than one or more boost deliveries. For example, a priming delivery may be made by subcutaneous delivery of an immunogen, and a boost delivery may be made by intramuscular delivery.

In addition, the prime and one or more boost deliveries of an antigen of interest may be "homologous," meaning that both the prime and boost comprises delivery of a liposomal composition of the invention; or may be "heterologous," meaning that one of the prime or boost deliveries comprises delivery of a liposomal composition of the present invention, while another delivery may be made by means of a different vaccine platform. Such alternative vaccine platforms include delivery of antigen in a non-liposomal vaccine formulation, delivery of DNA vaccine encoding the antigen, delivery of a recombinant viral vaccine.

In another aspect, the invention relates to methods for preparing a liposomal composition of the present invention. These methods comprise expressing or synthesizing the antigenic polypeptide, wherein the antigenic polypeptide is in monomeric form under non-denaturing conditions; purifying the antigenic polypeptide; adding an aqueous solution comprising the antigenic polypeptide to a lipid mixture comprising (i) DMPC, (ii) DMPG, DMTAP, or both DMPG and DMTAP, and (iii) at least one sterol; drying the antigenic polypeptide and lipid mixture; and sonicating the dried antigenic polypeptide and lipid mixture in the presence of an aqueous vehicle to form liposomes.

It is another object of the present invention to provide nucleic acids configured and arranged for expressing a polypeptide of interest as a fusion protein with a transmembrane sequence of, or derived from, a naturally occurring heterologous membrane protein. Such nucleic acids are isolated or purified, as those terms are defined herein.

In a first aspect, nucleic acid of the present disclosure has a nucleic acid sequence which encodes a fusion protein, the sequence of which comprises:

> a first nucleic acid sequence encoding a thioredoxin;
>
> a second nucleic acid sequence encoding a polypeptide sequence of interest; and
>
> a third nucleic acid sequence encoding a transmembrane sequence of, or derived from, a naturally occurring heterologous membrane protein, said second polypeptide sequence having a number of residues sufficient to cross a lipid bilayer, at least nine contiguous residues of which are predicted to form an alpha helix having a hydrophobicity score of about 0.7 or greater,

wherein said nucleic acid sequence is configured such that, when the nucleic acid is expressed, thioredoxin is expressed N-terminally to the transmembrane sequence.

In certain embodiments, the nucleic acids of the present invention further comprise one or more additional nucleic acid sequences which facilitate use of such nucleic acids in an expression system. Such nucleic acid sequences can include expression control sequences such as a promoter sequence and a ribosome binding site operably linked to such nucleic acids to provide an expression vector. Still other additional nucleic acid sequences include affinity markers, detection markers, and/or protease cleavage sites.

In various embodiments, the order of nucleic acid sequences in such a vector are configured such that the

order of expression, from N-terminal to C-terminal, in the fusion protein is: thioredoxin - polypeptide sequence of interest - transmembrane sequence; or thioredoxin - affinity marker - protease cleavage site - polypeptide sequence of interest - transmembrane sequence; thioredoxin - protease cleavage site - polypeptide sequence of interest - transmembrane sequence; or thioredoxin - transmembrane sequence - polypeptide sequence of interest; or thioredoxin - affinity marker - protease cleavage site - transmembrane sequence - polypeptide sequence of interest; or thioredoxin - protease cleavage site - transmembrane sequence - polypeptide sequence of interest. In each case, such a nucleic acid sequence is preferably in an expression vector and is operably linked to suitable expression control sequences.

As discussed hereinafter, expression of a protein sequence which is heterologous to the expression host can often be improved by replacing rare codons in a sequence to be expressed recombinantly with others that more closely reflect the host system's codon bias without modifying the amino acid sequence (referred to as "codon optimization"). In various embodiments, at least a portion of the nucleic acids of the present invention are codon optimized. By way of example, when *E. coli* is selected as the species for expression, one or more of the first, second, and third nucleic acid sequences described above are codon optimized to account for the codon bias known to exist in *E. coli.*

In various embodiments, the second nucleic acid sequence encoding a polypeptide sequence of interest can encode an antigenic sequence, a polypeptide targeting ligand (e.g., an antibody or other amino acid sequence which binds to a target referred to herein as a "receptor"), a fusogenic polypeptide sequence, which is heterologous to both thioredoxin and the transmembrane sequence.

In related aspects, the present invention relates to a host cell transformed with such a nucleic acid vector. In certain aspects, transformed host cells can be selected using a selectable marker encoded by the vector and the nucleic acid encoding the fusion protein expressed by the host cell. Optionally, the fusion protein may be separated from the host cells and purified in an aqueous medium. This fusion protein may then be incorporated into liposomes as discussed above, such that the polypeptide sequence of interest is displayed on the surface of the liposome.

## BRIEF DESCRIPTION OF THE FIGURES

Figs. 1-10 depict hydrophobicity plots of various polypeptide transmembrane sequences for use in practicing the present invention as calculated using the methodology of Eisenberg et al., J. Mol. Biol. (1984) 179:125-142.

## DETAILED DESCRIPTION OF THE INVENTION

"Administration" as it applies to a human, mammal, mammalian subject, animal, veterinary subject, placebo subject, research subject, experimental subject, cell, tissue, organ, or biological fluid, refers to contact of an exogenous ligand, reagent, placebo, small molecule, pharmaceutical agent, therapeutic agent, diagnostic agent, or composition to the subject, cell, tissue, organ, or biological fluid. "Administration" can refer, *e.g.*, to therapeutic, pharmacokinetic, diagnostic, research, placebo, and experimental methods. Treatment of a cell encompasses contact of a reagent to the cell, as well as contact of a reagent to a fluid, where the fluid is in contact with the cell. "Administration" also encompasses in vitro and ex vivo treatments, *e.g.*, of a cell, by a reagent, diagnostic, binding composition, or by another cell.

An "agonist," as it relates to a ligand and receptor, comprises a molecule, combination of molecules, a complex, or a combination of reagents, that stimulates the receptor. For example, an agonist of granulocyte-macrophage colony stimulating factor (GM-CSF) can encompass GM-CSF, a mutein or derivative of GM-CSF, a peptide mimetic of GM-CSF, a small molecule that mimics the biological function of GM-CSF, or an antibody that stimulates GM-CSF receptor.

An "antagonist," as it relates to a ligand and receptor, comprises a molecule, combination of molecules, or a complex, that inhibits, counteracts, downregulates, and/or desensitizes the receptor. "Antagonist" encompasses any reagent that inhibits a constitutive activity of the receptor. A constitutive activity is one that is manifest in the absence of a ligand/receptor interaction. "Antagonist" also encompasses any reagent that inhibits or prevents a stimulated (or regulated) activity of a receptor. By way of example, an antagonist of GM-CSF receptor includes an antibody that binds to the ligand (GM-CSF) and prevents it from binding to the receptor, or an antibody that binds to the receptor and prevents the ligand from binding to the receptor, or where the antibody locks the receptor in an inactive conformation.

"Conservatively modified variants" applies to both amino acid and nucleic acid sequences. With respect to particular nucleic acid sequences, a conservatively modified variant refers to nucleic acids encoding identical amino acid sequences, or amino acid sequences that have one or more conservative substitutions. An example of a conservative substitution is the exchange of an amino acid in one of the following groups for another amino acid of the same group (U.S. Pat. No. 5,767,063 issued to Lee, et al.; Kyte and Doolittle (1982) J. Mol. Biol. 157:105-132).

(1) Hydrophobic: Norleucine, Ile, Val, Leu, Phe, Cys, Met;
(2) Neutral hydrophilic: Cys, Ser, Thr;
(3) Acidic: Asp, Glu;
(4) Basic: Asn, Gln, His, Lys, Arg;

(5) Residues that influence chain orientation: Gly, Pro;
(6) Aromatic: Trp, Tyr, Phe; and
(7) Small amino acids: Gly, Ala, Ser.

"Effective amount" encompasses an amount that can ameliorate, reverse, mitigate, prevent, or diagnose a symptom or sign of a medical condition or disorder. Unless dictated otherwise, explicitly or by context, an "effective amount" is not limited to a minimal amount sufficient to ameliorate a condition.

"Gene" refers to a nucleic acid sequence encoding an oligopeptide or polypeptide. The oligopeptide or polypeptide can be biologically active, antigenically active, biologically inactive, or antigenically inactive. The term gene encompasses, e.g., the sum of the open reading frames (ORFs) encoding a specific oligopeptide or polypeptide; the sum of the ORFs plus the nucleic acids encoding introns; the sum of the ORFs and the operably linked promoter(s); the sum of the ORFS and the operably linked promoter(s) and any introns; the sum of the ORFS and the operably linked promoter(s), intron(s), and promoter(s), and other regulatory elements, such as enhancer(s). In certain embodiments, "gene" encompasses any sequences required in cis for regulating expression of the gene. The term gene can also refer to a nucleic acid that encodes a peptide encompassing an antigen or an antigenically active fragment of a peptide, oligopeptide, polypeptide, or protein. The term gene does not necessarily imply that the encoded peptide or protein has any biological activity, or even that the peptide or protein is antigenically active. A nucleic acid sequence encoding a non-expressable sequence is generally considered a pseudogene. The term gene also encompasses nucleic acid sequences encoding a ribonucleic acid such as rRNA, tRNA, or a ribozyme.

"Ligand" refers to a small molecule, peptide, polypeptide, or membrane associated or membrane-bound molecule, that is an agonist or antagonist of a receptor. "Ligand" also encompasses a binding agent that is not an agonist or antagonist, and has no agonist or antagonist properties. By convention, where a ligand is membrane-bound on a first cell, the receptor usually occurs on a second cell. The second cell may have the same identity (the same name), or it may have a different identity (a different name), as the first cell. A ligand or receptor may be entirely intracellular, that is, it may reside in the cytosol, nucleus, or in some other intracellular compartment. The ligand or receptor may change its location, e.g., from an intracellular compartment to the outer face of the plasma membrane. The complex of a ligand and receptor is termed a "ligand receptor complex." Where a ligand and receptor are involved in a signaling pathway, the ligand occurs at an upstream position and the receptor occurs at a downstream position of the signaling pathway. A ligand may be an antibody. The term "antibody" as used herein refers to a peptide or polypeptide derived from, modeled after or substantially encoded by an immunoglobulin gene or immunoglobulin genes, or fragments thereof, capable of specifically binding an antigen or epitope. *See, e.g.* Fundamental Immunology, 3rd Edition, W.E. Paul, ed., Raven Press, N.Y. (1993); Wilson (1994; J. Immunol. Methods 175:267-273; Yarmush (1992) J. Biochem. Biophys. Methods 25:85-97. The term antibody includes antigen-binding portions, i.e., "antigen binding sites," (e.g., fragments, subsequences, complementarity determining regions (CDRs)) that retain capacity to bind antigen, including (i) a Fab fragment, a monovalent fragment consisting of the VL, VH, CL and CHI domains; (ii) a F(ab')2 fragment, a bivalent fragment comprising two Fab fragments linked by a disulfide bridge at the hinge region; (iii) a Fd fragment consisting of the VH and CHI domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Single chain antibodies are also included by reference in the term "antibody."

"Nucleic acid" refers to deoxyribonucleotides or ribonucleotides and polymers thereof in either single stranded, double-stranded form, or multi-stranded form. Non-limiting examples of a nucleic acid are a, e.g., cDNA, mRNA, oligonucleotide, and polynucleotide. A particular nucleic acid sequence can also implicitly encompasses "allelic variants" and "splice variants."

"Operably linked" in the context of a promoter and a nucleic acid encoding a mRNA means that the promoter can be used to initiate transcription of that nucleic acid.

The terms "percent sequence identity" and "% sequence identity" refer to the percentage of sequence similarity found by a comparison or alignment of two or more amino acid or nucleic acid sequences. Percent identity can be determined by a direct comparison of the sequence information between two molecules by aligning the sequences, counting the exact number of matches between the two aligned sequences, dividing by the length of the shorter sequence, and multiplying the result by 100. An algorithm for calculating percent identity is the Smith-Waterman homology search algorithm (see, e.g., Kann and Goldstein (2002) Proteins 48:367-376; Arslan, et al. (2001) Bioinformatics 17:327-337).

By "purified" and "isolated" is meant, when referring to a polypeptide, that the polypeptide is present in the substantial absence of the other biological macromolecules with which it is associated in nature. The term "purified" as used herein means that an identified polypeptide often accounts for at least 50%, more often accounts for at least 60%, typically accounts for at least 70%, more typically accounts for at least 75%, most typically accounts for at least 80%, usually accounts for at least 85%, more usually accounts for at least 90%, most usually accounts for at least 95%, and conventionally accounts for at least 98% by weight, or greater, of the polypeptides present. The weights of water, buffers, salts, detergents, reductants, protease inhibitors, stabilizers (including an added protein such as albumin), and excipients,

and molecules having a molecular weight of less than 1000, are generally not used in the determination of polypeptide purity. See, e.g., discussion of purity in U.S. Pat. No. 6,090,611 issued to Covacci, et al.

"Peptide" refers to a short sequence of amino acids, where the amino acids are connected to each other by peptide bonds. A peptide may occur free or bound to another moiety, such as a macromolecule, lipid, oligo- or polysaccharide, and/or a polypeptide. Where a peptide is incorporated into a polypeptide chain, the term "peptide" may still be used to refer specifically to the short sequence of amino acids. A "peptide" may be connected to another moiety by way of a peptide bond or some other type of linkage. A peptide is at least two amino acids in length and generally less than about 25 amino acids in length, where the maximal length is a function of custom or context. The terms "peptide" and "oligopeptide" may be used interchangeably.

"Protein" generally refers to the sequence of amino acids comprising a polypeptide chain. Protein may also refer to a three dimensional structure of the polypeptide. "Denatured protein" refers to a partially denatured polypeptide, having some residual three dimensional structure or, alternatively, to an essentially random three dimensional structure, i.e., totally denatured. The invention encompasses reagents of, and methods using, polypeptide variants, e.g., involving glycosylation, phosphorylation, sulfation, disulfide bond formation, deamidation, isomerization, cleavage points in signal or leader sequence processing, covalent and non-covalently bound cofactors, oxidized variants. The formation of disulfide linked proteins is described (see, e.g., Woycechowsky and Raines (2000) Curr. Opin. Chem. Biol. 4:533-539; Creighton, et al. (1995) Trends Biotechnol. 13:18-23).

"Recombinant" when used with reference, e.g., to a nucleic acid, cell, animal, virus, plasmid, peptide, polypeptide, protein, vector indicates modification by the introduction of an exogenous, non-native nucleic acid, alteration of a native nucleic acid, or by derivation in whole or in part from a recombinant nucleic acid, cell, virus, plasmid, or vector. Recombinant protein refers to a protein derived, e.g., from a recombinant nucleic acid, virus, plasmid, vector. "Recombinant bacterium" encompasses a bacterium where the genome is engineered by recombinant methods, e.g., by way of a mutation, deletion, insertion, and/or a rearrangement. "Recombinant bacterium" also encompasses a bacterium modified to include a recombinant extra-genomic nucleic acid, e.g., a plasmid or a second chromosome, or a bacterium where an existing extra-genomic nucleic acid is altered.

A "selectable marker" encompasses a nucleic acid that allows one to select for or against a cell that contains the selectable marker. Examples of selectable markers include e.g.: (1) A nucleic acid encoding a product providing resistance to an otherwise toxic compound (e.g., an antibiotic), or encoding susceptibility to an otherwise harmless compound (e.g., sucrose); (2) A nucleic acid encoding a product that is otherwise lacking in the recipient cell (e.g., tRNA genes, auxotrophic markers); (3) A nucleic acid encoding a product that suppresses an activity of a gene product; (4) A nucleic acid that encodes a product that can be readily identified (e.g., phenotypic markers such as beta-galactosidase, green fluorescent protein (GFP), cell surface proteins, an epitope tag, a FLAG tag); (5) A nucleic acid that can be identified by hybridization techniques, for example, PCR or molecular beacons.

"Specifically" or "selectively" binds, when referring to a ligand/receptor, nucleic acid/complementary nucleic acid, antibody/antigen, or other binding pair (e.g., a cytokine to a cytokine receptor) indicates a binding reaction which is determinative of the presence of the protein in a heterogeneous population of proteins and other biologics. Thus, under designated conditions, a specified ligand binds to a particular receptor and does not bind in a significant amount to other proteins present in the sample. Specific binding can also mean, e.g., that the binding compound, nucleic acid ligand, antibody, or binding composition derived from the antigen-binding site of an antibody, of the contemplated method binds to its target with an affinity that is often at least 25% greater, more often at least 50% greater, most often at least 100% (2-fold) greater, normally at least ten times greater, more normally at least 20-times greater, and most normally at least 100-times greater than the affinity with any other binding compound.

The term "subject" as used herein refers to a human or non-human organism. Thus, the methods and compositions described herein are applicable to both human and veterinary disease. In certain aspects of the disclosure, subjects are "patients," i.e., living humans that are receiving medical care for a disease or condition. This includes persons with no defined illness who are being investigated for signs of pathology. Preferred are subjects who have an existing plasmodium infection.

"Therapeutically effective amount" is defined as an amount of a reagent or pharmaceutical composition that is sufficient to show a patient benefit, i.e., to cause a decrease, prevention, or amelioration of the symptoms of the condition being treated. When the agent or pharmaceutical composition comprises a diagnostic agent, a "diagnostically effective amount" is defined as an amount that is sufficient to produce a signal, image, or other diagnostic parameter. Effective amounts of the pharmaceutical formulation will vary according to factors such as the degree of susceptibility of the individual, the age, gender, and weight of the individual, and idiosyncratic responses of the individual (see, e.g., U.S. Pat. No. 5,888,530 issued to Netti, et al.).

"Treatment" or "treating" (with respect to a condition or a disease) is an approach for obtaining beneficial or desired results including and preferably clinical results. For purposes of this invention, beneficial or desired results with respect to a disease include one or more of the following: improving a condition associated with a disease, curing a disease, lessening severity of a disease, delaying progression of a disease, alleviating one or more symptoms associated

with a disease, increasing the quality of life of one suffering from a disease, and/or prolonging survival. Likewise, for purposes of this invention, beneficial or desired results with respect to a condition include one or more of the following: improving a condition, curing a condition, lessening severity of a condition, delaying progression of a condition, alleviating one or more symptoms associated with a condition, increasing the quality of life of one suffering from a condition, and/or prolonging survival.

"Vaccine" encompasses preventative vaccines. Vaccine also encompasses therapeutic vaccines, e.g., a vaccine administered to a mammal that comprises a condition or disorder associated with the antigen or epitope provided by the vaccine.

The goal of vaccine formulation is provide a combination of antigens and adjuvants capable of generating a sufficient population of memory T cells and B cells to react quickly to a pathogen, tumor cell, bearing an antigen of interest. The present invention relates to methods for providing liposomal vaccine compositions, methods for the manufacture thereof, and the liposomal vaccine compositions for use in stimulating an immune response in an animal, which can meet this goal.

The annual flu (also called "seasonal flu" or "human flu") in the U.S. reportedly results in approximately 36,000 deaths and more than 200,000 hospitalizations each year. It is referred to as being "seasonal" because influenza epidemics typically emerge during each winter. There are two flu seasons which occur at different times in the Northern and Southern Hemispheres. Worldwide, seasonal influenza kills an estimated 250,000 to 500,000 people each year. The majority of deaths in the industrialized world occur in adults age of 65 and over. Seasonal flu is the seventh leading cause of death in the U.S., and its economic costs in the U.S. alone have been estimated at over $80 billion. There are three types of seasonal influenza - A, B and C. Perhaps the most lethal seasonal flu outbreak was the 1918 flu pandemic (Spanish flu pandemic; type A influenza, H1N1 subtype), which lasted from 1918 to 1919. Estimates of the number of individuals killed in this outbreak range from 20 to 100 million people.

Influenza A virus ("IAV") is a member of the Orthomyxoviridae family of viruses. Influenza A viruses contain a single-stranded, segmented, negative-sense RNA genome. The eight RNA segments are:

"HA", which encodes the viral hemagglutinin (about 500 molecules of hemagglutinin are needed to make one virion);

"NA", which encodes the viral neuraminidase (about 100 molecules of neuraminidase are needed to make one virion);

"NP", which encodes the viral nucleoprotein;

"M", which encodes two matrix proteins M1 and M2 (the latter being an ion channel in the viral envelope) through the use of different reading frames from the same RNA segment (about 3000 matrix protein molecules are needed to make one virion);

"NS", which encodes non-structural proteins NS1 and NEP through the use of different reading frames from the same RNA segment;

"PA", which encodes a viral RNA polymerase;

"PB1", which encodes another RNA polymerase, together with PB1-F2 protein through the use of different reading frames from the same RNA segment; and

"PB2", which encodes an RNA polymerase.

Thus, the IAV genome encodes eleven proteins (HA, NA, NP, M1, M2, NS1, NEP, PA, PB1, PB1-F2, PB2). IAV is typically classified into subtypes, the nomenclature of which arises according to an H (haemagglutinin) number and an N (neuraminidase) number. New influenza viruses are constantly evolving by mutation or by genomic reassortment. The IAV genome is highly plastic, due to an inherent high mutation rate, together with the segmented nature of the genome which allows for the exchange of entire genes between different viral strains. This results in a virus exhibiting continual antigenic variation, which requires the annual reformulation of IAV vaccines.

The protective immune response generated by current vaccines is believed to be based largely on viral hemagglutinin (HA) and neuraminidase (NA) proteins. Influenza vaccines used in the United States and around world are manufactured by growing virus in fertilized chicken eggs, then either killing the virus or attenuating live virus. This egg-based technology for producing IAV vaccine was created in the 1950s. To achieve current vaccine production targets, millions of 11-day old fertilized eggs must be available every day of production. Because of the continual antigenic variation in IAV, vaccines based on a current seasonal flu cannot be depended upon to work in the case of a future seasonal flu. While there can be some cross-protection against related flu strains, the best protection would be from a

vaccine specifically produced for any future pandemic flu virus strain.

Problems with H5N1 vaccine production include a lack of overall production capacity, and lack of surge production capacity (it is impractical to develop a system that depends on hundreds of millions of 11-day old specialized eggs on a standby basis).

Some IAV vaccines based on modern molecular biology techniques have started early stage clinical trials. While several such vaccines directed to viral hemagglutinin (HA) and neuraminidase (NA) proteins have been described, novel targets such as antigens from the NP and M2 proteins also been explored.

Herpes Simplex Virus is a member of the Herpesviridae family of viruses. Genital herpes caused by Herpes Simplex Virus Type 2 (HSV2) is one of the most common sexually transmitted diseases in humans. Epidemiological studies indicate that as many as 1 out of every 6 American adults is infected with HSV2. The disease affects both normal and immunosuppressed adults, and is associated with increased susceptibility to infection by the human immunodeficiency virus (HIV). Serious clinical disease can occur in neonates following transmission of HSV2 from their infected mother and these mothers are more likely to develop cervical cancer than non-infected women. A major factor contributing to HSV2 transmission is the shedding of the virus from the genital tract in the absence of clinical symptoms. Currently, an effective vaccine to prevent spread of the disease is not available. Several HSV2 vaccine candidates containing subunit protein-based antigens have failed to achieve the goal of providing protective immunity against infection or clinical disease by HSV2. In light of the unsuccessful results of the tested vaccine candidates, the realization that people who are infected with HSV2 are more susceptible to infection by HIV, and the fact that viral shedding often occurs in "clinically asymptomatic" carriers of HSV2, the effort to develop new approaches to HSV2 disease management, including new immunization strategies, remains an important healthcare objective.

Two different HSV2 vaccines containing recombinant forms of the glycoproteins B and D (gB and gD, respectively) have been tested in Phase 3 clinical trials. One of these vaccines contained recombinant HSV2 gB and gD, combined with the adjuvant MF59, to create an oil-in-water emulsion of the proteins. The vaccine stimulated high levels of HSV2-specific neutralizing antibodies in vaccinated recipients, but no significant effects on the duration of the first clinical episode or subsequent frequency of reactivation of infection were observed, suggesting that this vaccine was not particularly effective. The second HSV2 vaccine was consisted of HSV2 gD and an adjuvant system, AS04, containing aluminum hydroxide and 3-deacylated monophosphoryl lipid A. Although preliminary results suggested that this vaccine was effective in HSV1 seronegative women (but not men) for preventing the development of symptomatic HSV2 genital herpes, recent results from a larger study did not confirm the initial findings. The unexpected failure of the GSK HSV2 gD-based vaccine will undoubtedly lead to further speculation on the role of the gD antigen as a target for developing an effective HSV2 vaccine.

There are many possible factors that might account for the clinical failure of these vaccine formulations. One of the most obvious is that the gD antigen alone does not contain the epitopes necessary to provide complete protective immunity to a heterologous human population. Studies in humans and mice indicate that CD8 and CD4 T-cells are important in the control of HSV2 infection. While gD epitopes are recognized by human CD4 T cells, no gD specific CD8 T cells have been observed. Recently, 47 new CD8 T cell epitopes from HSV1 seropositive individuals were identified, with none of these CD8 T cells responding to gD. These studies indicate that while the gD protein may produce neutralizing antibodies and stimulate CD4 T-cells, it does not induce a strong CD8 T cell response, which is most relevant for clearance or control of latently infected cells. Without CD8 T cells, the gD vaccine alone appears incapable of stimulating a protective immune response to an HSV2 infection in humans. A second possibility is that the adjuvant system used might not have been optimal for stimulating protective immune responses. The formulations tested clinically have not incorporated a strong cellular immune response adjuvant. Lastly, other factors, such as the potential lack of cross-protection caused by slight differences in strains of HSV2 endemic in a particular region, might also play a role in the overall efficacy of a gD-based vaccine. Taken together, studies aimed at addressing all of the possible reasons for the failure of HSV2 vaccine candidates to date will require significant effort to identify a truly effective HSV2 vaccine.

Previously, a liposomal HSV2 $gD_{1-306}$ vaccine (L-$gD_{1-306}$-HD) was tested in an acute murine HSV2 infection model of intravaginal (female) or intrarectal (male or female) challenge (Olson et al., Vaccine 28: 548-560, 2009. Two doses of L-$gD_{1-306}$-HD containing 60$\mu$g $gD_{1-306}$-HD and 15$\mu$g monophosphoryl lipid A (MPL) per dose reportedly provided protection against HSV2 intravaginal challenge (86-100% survival, $P \leq 0.0003$ vs control liposomes; P=0.06 vs L-$gD_{1-306}$-HD without MPL). Both male and female mice (BALB/c and C57BL/6) immunized with L-$gD_{1-306}$-HD/MPL were significantly protected against HSV2 intrarectal challenge, with higher survival rates compared to controls (71-100%, $P \leq 0.007$). L-$gD_{1-306}$-HD/MPL also provided increased survival when compared to a liposomal peptide vaccine, L-$gD_{264-285}$-HD/MPL (male BALB/c, $P \leq 0.001$; female BALB/c and male C57BL/6, P=0.06). Mice given L-$gD_{1-306}$-HD/MPL also had minimal disease signs, reduced viral burden in their spinal cords and elevated neutralizing antibody titers in the females. The vaccine also stimulated $gD_{1-306}$-HD specific splenocytes of both male and female mice with significantly elevated levels of IFN-$\gamma$ compared to IL-4 ($P \leq 0.01$) indicating that there was an enhanced Th1 response.

As discussed above, the liposomal formulations of the present invention comprise liposomes prepared using dimyristoylphosphatidylcholine ("DMPC"); together with dimyristoylphosphatidylglycerol ("DMPG"), dimyristoyltrimethylammonium propane ("DMTAP"), or both DMPG and DMTAP; and one or more sterols. The components of the liposomes may be naturally occurring or synthetic. Sterols are also known as steroid alcohols. They are a subgroup of steroids with a hydroxyl group at the 3-position of the A-ring.

In addition, these liposomal formulations comprise an antigenic polypeptide. The terms "antigenic polypeptide" as used herein refers to a polypeptide comprising at least one antigenic epitope that is foreign to a recipient animal and that, and upon delivery to the animal using, in whole or part, the liposomal formulations described herein, stimulates the formation of antigen specific antibodies and/or an antigen-specific T-cell response. Antigenic polypeptides which may be used in practicing the present disclosure may be derived from, by way of example only, viral pathogens, bacterial toxins, bacterial pathogens, fungal pathogens, cancer cells. The antigenic peptides comprise residues derived from IAV M2 protein extracellular domain.

As described herein, the antigenic polypeptides of the present invention comprise, in addition to an antigenic sequence of interest, a suitable hydrophobic domain. Examples of suitable antigenic sequences include polypeptide sequences which comprises one or more T-cell epitopes an IAV M2 protein sequence, an HSV2 protein sequence obtained from a protein selected from the group consisting of Tegument host shutoff protein, Tegument protein VP13/14, Ribonucleotide reductase subunit 1, Ribonucleotide reductase subunit 2, Tegument protein VP11/12, Envelope glycoprotein B, DNA packaging tegument protein, Nuclear egress lamina protein, Multifunctional expression regulator, Transcriptional regulator, Infected Cell Protein 8, Single-stranded DNA-binding protein, Capsid maturation protease, Envelope glycoprotein I. Figs. 1-10 depict Eisenberg et al. (J. Mol. Biol. 179:125-142(1984) hydrophobicity profiles of a number of exemplary hydrophobic domains from naturally occurring proteins which preferably find use in the present invention. These hydrophobic domain sequences, and the others provided by way of example herein, are exemplary in nature only. These or other hydrophobic sequences can me modified using conservative amino acid substitutions to arrive at additional synthetic sequences which have a hydrophobic profile similar to that of human cytochrome b5 transmembrane domain for example. For purposes of the invention, a hydrophobicity profile is calculated using the methodology of Eisenberg et al., using the following parameters: a window size of 9, relative weight of edges 100%, and a linear weight variation. Amino acid scale values are as follows:

Ala: 0.620 Arg: -2.530 Asn: -0.780 Asp: -0.900 Cys: 0.290 Gln: -0.850 Glu: -0.740 Gly: 0.480 His: -0.400 Ile: 1.380 Leu: 1.060 Lys: -1.500 Met: 0.640 Phe: 1.190 Pro: 0.120 Ser: -0.180 Thr: -0.050 Trp: 0.810 Tyr: 0.260 Val: 1.080

Suitable sequences for the hydrophobic domain portion typically have a sufficient length to cross a lipid bilayer (from about 15-30 residues; more preferably from about 18-25 residues), at least 9 contiguous residues of which are predicted to form an alpha helix having a hydrophobicity (Eisenberg score) of about + 0.7. The term "about in this context refers to +/- 10% of a given measurement. Type II and type III membrane proteins lack a cleavable N-terminal signal peptide controlling membrane insertion and topology, and instead insert into membranes posttranslationally via the hydrophobic "signal-anchor sequence" which is an uncleavable signal sequence that mediates the translocation of the polypeptide and anchors the protein in the membrane.

Fusion proteins:

As discussed above, methods for covalently linking an immunogenic polypeptide sequence to heterologous transmembrane sequence are well known in the art. The antigenic polypeptides of the present invention may be chemically synthesized or expressed using recombinant DNA methodology. In various embodiments, one or more antigenic epitopes are expressed as part of a fusion protein with a transmembrane sequence from a heterologous single-pass membrane protein having a signal-anchor sequence. Such recombinant proteins may comprise antigenic sequences selected from the group consisting of a viral envelope protein.

Fusion proteins may be expressed in cell-based and cell-free systems. For large-scale protein expression and purification, bacterial expression is often used, typically under control of either a bacterial or T7 promoter, depending on the bacterial strain, and an RBS (prokaryotic ribosome binding sequence). Nucleic acids encoding polypeptide sequences of interest operably connected to suitable expression control sequences, proteolytic cleavage sites, optionally flanked or separated by spacer residues are inserted into an appropriate vector by standard recombinant DNA techniques (see generally, Sambrook et al., Molecular Cloning, A Laboratory Manual, 2d ed., Cold Spring Harbor Laboratory Press, Cold Spring Harbor, N.Y., 1989). The nucleic acids of interest are ultimately expressed as antigenic polypeptides (with or without spacer or framework residues).

*E. coli* is one prokaryotic host useful particularly for expression of the antigenic polypeptides of the present invention. Other microbial hosts suitable for use include bacilli, such as *Bacillus subtilis*, and other *enterobacteriaceae*, such as *Salmonella, Serratia*, and various *Pseudomonas* species. In these prokaryotic hosts, one can also make expression vectors, which will typically contain expression control sequences compatible with the host cell (*e.g.*, an origin of replication). In addition, any number of a variety of well-known promoters will be present, such as the lactose promoter system, a tryptophan (trp) promoter system, a beta-lactamase promoter system, or a promoter system from phage lambda. The promoters typically control expression, optionally with an operator sequence, and have ribosome binding

site sequences, for initiating and completing transcription and translation.

Other microbes, such as yeast, are also be used for expression. *Saccharomyces* is a preferred host, with suitable vectors having expression control sequences, such as promoters, including 3-phosphoglycerate kinase or other glycolytic enzymes, and an origin of replication, and termination sequences as desired.

Mammalian tissue cell culture can also be used to express and produce the antigenic polypeptides of the present invention (see Winnacker, From Genes to Clones (VCH Publishers, N.Y., N.Y., 1987). A number of suitable host cell lines capable of secreting intact immunoglobulins have been developed including insect cells for baculovirus expression, CHO cell lines, various Cos cell lines, HeLa cells, myeloma cell lines, transformed B-cells and hybridomas. Expression vectors for these cells can include expression control sequences, such as an origin of replication, a promoter, and an enhancer (Queen et al., Immunol. Rev. 89: 49-68 (1986)), and necessary processing information sites, such as ribosome binding sites, RNA splice sites, polyadenylation sites, and transcriptional terminator sequences. Preferred expression control sequences are promoters derived from immunoglobulin genes, SV40, adenovirus, bovine papilloma virus, or cytomegalovirus.

Different organisms often display "codon bias"; that is, the degree to which a given codon encoding a particular amino acid appears in the genetic code varies significantly between organisms. In general, the more rare codons that a gene contains, the less likely it is that the heterologous protein will be expressed at a reasonable level within that specific host system. These levels become even lower if the rare codons appear in clusters or in the N-terminal portion of the protein. Replacing rare codons in a sequence to be expressed recombinantly with others that more closely reflect the host system's codon bias without modifying the amino acid sequence (referred to as "codon optimization") can increase the levels of functional protein expression. In various embodiments, at least one percent of any non-optimal codons are changed to provide optimal codons, more normally at least five percent are changed, most normally at least ten percent are changed, often at least 20% are changed, more often at least 30% are changed, most often at least 40%, usually at least 50% are changed, more usually at least 60% are changed, most usually at least 70% are changed, optimally at least 80% are changed, more optimally at least 90% are changed, most optimally at least 95% are changed, and conventionally 100% of any non-optimal codons are codon-optimized for expression. Gene optimization tools such as Gene Designer 2.0 (DNA2.0), OptimumGene™ (GenScript), OPTIMIZER (Puigbo et al., Nucl. Acids Res. 35 (Suppl 2): W126-31, 2007), GeneOptimizer® (GeneArt), Gene Composer (Lorimer et al., BMC Biotechnology 2009, 9:36 doi:10.1186/1472-6750-9-36), and the methods disclosed in U.S. Patents 7,561,973, 7,561,972, are known in the art and may be used to optimize expression of the desired fusion protein.

Methods for introducing vectors containing the polynucleotide sequences of interest vary depending on the type of cellular host. For example, calcium chloride transfection is commonly utilized for prokaryotic cells, whereas calcium phosphate treatment or electroporation may be used for other cellular hosts. (See generally Sambrook *et al.*, *supra*).

In certain embodiments, a fusion protein may comprise an additional peptide sequence, such as an affinity marker, detection marker, and/or protease cleavage site. Examples of such affinity/detection markers include strep-tag, glutathione S-transferase (GST), maltose binding protein (MBP), thioredoxin (Trx), calmodulin binding peptide (CBP), poly-His, FLAG, c-myc, and hemagglutinin (HA). GST, MBP, Trx, CBP, and poly-His enable purification of their cognate fusion proteins on immobilized glutathione, maltose, phenylarsine oxide, calmodulin, and metal-chelate resins, respectively. Strep-tag, FLAG, c-myc, and hemagglutinin (HA) enable immunoaffinity purification of fusion proteins using commercially available monoclonal and polyclonal antibodies that specifically recognize these epitope tags. Other suitable tag sequences will be apparent to those of skill in the art. Likewise, numerous proteolytic enzymes (e.g., trypsin, subtilisin, thermolysin, chymotrypsin, papain) and corresponding protease cleavage sites are known to those of skill in the art. In preferred embodiments, one or more proteolytic enzymes for use in the present methods are selected from the group consisting of factor Xa, enterokinase, thrombin, tobacco etch virus protease, and human rhinovirus 3C protease. In yet other preferred embodiments, an introduced protease cleavage site is selected from the group consisting of IEGR (SEQ ID NO: 55), DDDDK (SEQ ID NO: 56), LVPRGS (SEQ ID NO: 57), ENLYFQG (SEQ ID NO: 58), and LEVLFQGP (SEQ ID NO: 59).

Such fusion proteins may also comprise chemical structures between the polypeptides said to be fused. Two polypeptides are "directly" fused if the last residue of the sequence of one of the peptides is joined to the first residue of the other sequence. Two polypeptides are "indirectly" fused if additional chemical structures (such as additional amino acids, sometimes referred to as "linker" residues) lie between the last residue of the sequence of one of the peptides and the first residue of the other sequence.

Chemical protein coupling

As an alternative to fusion proteins, one or more antigenic epitopes may be chemically synthesized or expressed separately from a transmembrane sequence from a heterologous single-pass membrane protein having a signal-anchor sequence, and the separate peptides coupled using chemical linkages. Chemical cross-linkers are discussed in numerous

books and catalogues. *See, e.g.*, Wong, Chemistry of Protein Conjugation and Cross-linking, CRC Press, Boca Raton, Fla., 1991. These reagents often employ functional groups that couple to amino acid side chains of peptides. Designing a cross-linker involves selection of the functional moieties to be employed. The choice of functional moieties is entirely dependent upon the target sites available on the species to be crosslinked. Some species (*e.g.*, proteins) may present a number of available sites for targeting (*e.g.*, lysine ε-amino groups, cysteine sulfhydryl groups, glutamic acid carboxyl groups), and selection of a particular functional moiety for inclusion in a sterol may be made empirically in order to best preserve a biological property of interest (e.g., binding affinity of an antibody, catalytic activity of an enzyme)

Coupling through Amine Groups:

Imidoester and N-hydroxysuccinimidyl ("NHS") esters are typically employed as amine-specific functional moieties. NHS esters yield stable products upon reaction with primary or secondary amines. Coupling is efficient at physiological pH, and NHS-ester cross-linkers are more stable in solution than their imidate counterparts. Homobifunctional NHS-ester conjugations are commonly used to cross-link amine-containing proteins in either one-step or two-step reactions. Primary amines are the principle targets for NHS-esters. Accessible α-amine groups present on the N-termini of proteins react with NHS-esters to form amides. However, because α-amines on a protein are not always available, the reaction with side chains of amino acids become important. While five amino acids have nitrogen in their side chains, only the ε-amino group of lysine reacts significantly with NHS-esters. A covalent amide bond is formed when the NHS-ester cross-linking agent reacts with primary amines, releasing N-hydroxysuccinimide.

Coupling through Sulfhydryl Groups:

Maleimides, alkyl and aryl halides, α-haloacyls, and pyridyl disulfides are typically employed as sulfhydryl-specific functional moieties. The maleimide group is specific for sulfhydryl groups when the pH of the reaction mixture is kept between pH 6.5 and 7.5. At pH 7, the reaction of the maleimides with sulfhydryls is 1000-fold faster than with amines. Maleimides do not react with tyrosines, histidines or methionines. When free sulfhydryls are not present in sufficient quantities, they can often be generated by reduction of available disulfide bonds.

Coupling Through Carboxyl Groups:

Carbodiimides couple carboxyls to primary amines or hydrazides, resulting in formation of amide or hydrazone bonds. Carbodiimides are unlike other conjugation reactions in that no cross-bridge is formed between the carbodiimide and the molecules being coupled; rather, a peptide bond is formed between an available carboxyl group and an available amine group. Carboxy termini of proteins can be targeted, as well as glutamic and aspartic acid side chains. In the presence of excess cross-linker, polymerization may occur because proteins contain both carboxyls and amines. No cross-bridge is formed, and the amide bond is the same as a peptide bond, so reversal of the cross-linking is impossible without destruction of the protein.

Nonselective reactive groups:

A photoaffinity reagent is a compound that is chemically inert but becomes reactive when exposed to ultraviolet or visible light. Arylazides are photoaffinity reagents that are photolyzed at wavelengths between 250-460 nm, forming a reactive aryl nitrene. The aryl nitrene reacts nonselectively to form a covalent bond. Reducing agents must be used with caution because they can reduce the azido group.

Coupling Through Arginines:

Glyoxals are useful compounds for targeting the guanidinyl portion of arginine residues. Glyoxals will target arginines at mildly alkaline pH. There is some cross-reactivity (the greatest at higher pH) with lysines.

Coupling Through Carbonyl Groups:

Carbonyls (aldehydes and ketones) react with amines and hydrazides at pH 5-7. The reaction with hydrazides is faster than with amines, making this useful for site-specific cross-linking. Carbonyls do not readily exist in proteins; however, mild oxidation of sugar moieties using sodium metaperiodate will convert vicinal hydroxyls to aldehydes or ketones. For carbohydrates with reducing end(s), the carbonyl group(s) can be reactive towards a hydrazine moiety to form a hydrazone bond. S-HyNic is a heterobifunctional linker used to incorporate HyNic (6-hydrazinonicotinamide) moieties into molecules through a free amino group via an activated ester (i.e. NHS). The addition of a HyNic hydrazine

linker permits formation of a conjugate in slightly acidic buffer (100mM NaPO4, pH6). For carbohydrates without a reducing end, CDAP specific activation may be used. Under mild conditions (pH 9.5 for activation and pH 7 for conjugation), 1-cyano-4-dimethylaminopyridinium tetrafluoroborate ("CDAP") converts hydroxyl groups to cyanyl esters which will then form carbamates in the presence of amine groups.

The polymeric substances optionally included in the linkage chemistry are preferably poly(alkylene oxides). As used herein, the term "alkylene oxide" refers to the structure, -X-O-, where X is an alkylene moiety covalently linked to oxygen O; thus poly(alkylene oxide) refers to the structure $-(X-O-)_m)-$. It is preferred that the poly(alkylene oxide) polymer be a nonbranched homopolymer (*i.e.*, a polymer of the structure $-((CH_2)_n-O-)_m)-$ in which n does not vary) such as poly(ethylene oxide) derived from ethylene glycol. Alternative polymers such as other polyalkylene oxide homopolymers (*e.g.*, methylene oxide, propylene oxide, isopropylene oxide, and butylene oxide polymers) and co-polymers or block co-polymers of poly(alkylene oxides) may also be used. In those aspects of the invention where PEG-based polymers are used, it is preferred that they have average length n of between 40 and 1000 monomeric units. Molar equivalent amounts of the other alkylene oxides may be determined readily by those of ordinary skill in the art to arrive at preferred average molecular weights for other homopolymers and copolymers.

Average molecular weights of the present invention are measured using the "number-average" method. In a mixture of polymer molecules with different molecular weights in which the number of molecules having a particular molecular weight, $M_i$, is given by $N_i$, the "number-average" probability of a given mass being present is

$$P_i = \frac{N_i}{\sum_{j=0}^{\infty} N_j}$$

and the number-average molecular weight is given by the formula

$$\overline{M_n} = \sum_{i=0}^{\infty} \left( \frac{N_i}{\sum_{j=0}^{\infty} N_j} \right) M_i = \frac{\sum_{i=0}^{\infty} N_i M_i}{\sum_{j=0}^{\infty} N_j}$$

The number average is the simple arithmetic mean, representing the total weight of the molecules present divided by the total number of molecules. The number-average molecular weight of a polymer may be measured by vapor pressure osmometry using methods and apparatuses well known to those of skill in the art.

Alternative polymeric substances which may be used in place of poly(alkylene oxides) include materials such as dextran, polyvinyl pyrrolidones, polysaccharides, starches, polyvinyl alcohols, polyacryl amides or other similar polymers.

"Administration" as used herein with respect to an animal, including preferably a mammal and most preferably a human, refers to delivery of an exogenous reagent to a cell, tissue, organ, or biological fluid of the subject.

"Effective amount" as used herein refers to an amount of a reagent that can ameliorate, reverse, mitigate, or prevent a symptom or sign of a medical condition or disorder. Unless dictated otherwise, explicitly or otherwise, an "effective amount" is not limited to a minimal amount sufficient to ameliorate a condition, or to an amount that results in an optimal or a maximal amelioration of the condition. "Effective amount" within the context of administration of a vaccine is that which causes an immune response in the mammal. Such an effective amount may not be, in and of itself, sufficient to cause such an immune response, but may be used together with previous or subsequent delivery of additional reagents (e.g. a prime-boost vaccination). An "immunological response" or "immune response" as used herein encompasses at least one or more of the following effects: the production of antibodies by B- cells; and/or the activation of suppressor T-cells and/or T-cells directed specifically to an antigen or antigens present in the vectors, composition or vaccine of interest.

A variety of *in vitro* and *in vivo* assays are known in the art for measuring an immune response, including measuring humoral and cellular immune responses, which include standard immunoassays, such as RIA, ELISA assays; intracellular staining; T cell assays including for example, lymphoproliferation (lymphocyte activation) assays, CTL cytotoxic cell assays, or by assaying for T-lymphocytes specific for the antigen in a sensitized subject. Such assays are well known in the art.

Liposomal preparation:

The preparation of liposomes is well known in the prior art. In general, liposomes have been made by a number of different techniques including ethanol injection (Batzri et al., Biochem. Biophys. Acta. 298:1015, 1973); ether infusion (Deamer et al., Biochem. Biophys. Acta. 443:629, 1976; Schieren et al., Biochem. Biophys. Acta. 542:137, 1978); detergent removal (Razin, Biochem. Biophys. Acta. 265:24 1972); solvent evaporation (Matsumato et al., J. Colloid Interface Sci. 62:149, 1977); evaporation of organic solvents from chloroform in water emulsions (REV's) (Szoka Jr. et al., Proc. Natl. Acad. Sci. USA, 75:4194, 1978); extrusions of MLVs or EUV's through a nucleopore polycarbonate membrane (Olson et al., Biochem. Biophys. Acta. 557:9, 1979); freezing and thawing of phosopholipid mixtures (Pick, Arch. Biochem. Biophys., 212:186, 1981), as well as sonication and homogenization. By convention, liposomes are categorized by size, and a 3-letter acronym is used to designate the type of liposome being discussed. Multilamellar vesicles are generally designated "MLV." Small unilamellar vesicles are designated "SUV," and large unilamellar vesicles are designated "LUV." These designations are sometimes followed by the chemical composition of the liposome. For a discussion of nomenclature and a summary of known types of liposomes, see Storm et al., PSIT, 1: 19-3, 1998.

The liposomal compositions of the invention may further comprise, either as part of the liposome itself or as part of the vehicle in which the liposomes are suspended, various excipients, adjuvants, carriers, auxiliary substances, modulating agents.

A carrier, which is optionally present, is a molecule that does not itself induce the production of antibodies harmful to the individual receiving the composition. Suitable carriers are typically large, slowly metabolized macromolecules such as proteins, polysaccharides, polylactic acids, polyglycollic acids, polymeric amino acids, amino acid copolymers, lipid aggregates (such as oil droplets or liposomes), and inactive virus particles. Examples of particulate carriers include those derived from polymethyl methacrylate polymers, as well as microparticles derived from poly(lactides) and poly(lactide-co-glycolides), known as PLG. *See, e.g.*, Jeffery et al., Pharm. Res. 10:362, 1993; McGee et al., J. Microencapsul. 14: 197, 1997; O'Hagan et al., Vaccine 11:149, 1993. Such carriers are well known to those of ordinary skill in the art.

Adjuvants include: (1) aluminum salts (alum), such as aluminum hydroxide, aluminum phosphate, aluminum sulfate; (2) oil-in-water emulsion formulations (with or without other specific immunostimulating agents such as muramyl peptides (see below) or bacterial cell wall components), such as for example (a) MF59 (International Publication No. WO 90/14837), containing 5% Squalene, 0.5% Tween 80, and 0.5% Span 85 (optionally containing various amounts of MTP-PE (see below), although not required) formulated into submicron particles using a microfluidizer such as Model 11OY microfluidizer (Microfluidics, Newton, Mass.), (b) SAF, containing 10% Squalane, 0.4% Tween 80, 5% pluronic-blocked polymer LI 21, and MDP either microfluidized into a submicron emulsion or vortexed to generate a larger particle size emulsion, and (c) Ribi™ adjuvant system (RAS), (Ribi Immunochem, Hamilton, MT); (3) one or more bacterial cell wall components from the group consisting of monophosphorylipid A (MPL), trehalose dimycolate (TDM), and cell wall skeleton (CWS), preferably MPL+CWS (Detoxu); (4) saponin adjuvants, such as Stimulon™ (Cambridge Bioscience, Worcester, Mass.); (5) Complete Freunds Adjuvant (CFA) and Incomplete Freunds Adjuvant (IFA); (6) cytokines, such as interleukins (IL-I, IL-2), macrophage colony stimulating factor (M-CSF), tumor necrosis factor (TNF), beta chemokines (MIP, 1-alpha, 1-beta Rantes); (7) detoxified mutants of a bacterial ADP-ribosylating toxin such as a cholera toxin (CT), a pertussis toxin (PT), or an E. coli heat-labile toxin (LT), particularly LT- K63 (where lysine is substituted for the wild-type amino acid at position 63) LT-R72 (where arginine is substituted for the wild-type amino acid at position 72), CT-S109 (where serine is substituted for the wild-type amino acid at position 109), and PT-K9/G129 (where lysine is substituted for the wild-type amino acid at position 9 and glycine substituted at position 129) (see, e.g., International Publication Nos. WO93/13202 and WO92/19265); and (8) other substances that act as immunostimulating agents to enhance the effectiveness of the composition.

Preferred adjuvants include pathogen-associated molecular patterns (PAMPs), which mediate innate immune activation via Toll-like Receptors (TLRs), (NOD)-like receptors (NLRs), Retinoic acid inducible gene-based (RIG)-I-like receptors (RLRs), and/or C-type lectin receptors (CLRs). Examples of PAMPs include lipoproteins, lipopolypeptides, peptidoglycans, zymosan, lipopolysaccharide, neisserial porins, flagellin, profillin, $\alpha$-galactosylceramide, muramyl dipeptide. Peptidoglycans, lipoproteins, and lipoteichoic acids are cell wall components of Gram-positive. Lipopolysaccharides are expressed by most bacteria, with MPL being one example. Flagellin refers to the structural component of bacterial flagella that is secreted by pathogenic and commensal bacterial. $\alpha$-Galactosylceramide ($\alpha$-GalCer) is an activator of natural killer T (NKT) cells. Muramyl dipeptide is a bioactive peptidoglycan motif common to all bacteria

Other preferred adjuvants include viral double-stranded RNA, which is sensed by the intracellular receptor TLR3; CpG motifs present on bacterial or viral DNA or ssRNA, which are sensed by TLR7, 8, and 9; all-trans retinoic acid; and heat shock proteins such as HSP70 and Gp96, which are highly effective carrier molecules for cross-presentation. Pharmaceutical adjuvants include resiquimod, a TLR7/8 agonists, and imiquimod, a TLR7 agonist.

Pharmaceutical compositions:

The liposomes of the present invention are preferably formulated as pharmaceutical compositions for use in parenteral or enteral delivery. A typical pharmaceutical composition for administration to an animal comprises a pharmaceutically acceptable vehicle such as aqueous solutions, non-toxic excipients, including salts, preservatives, buffers. See, e.g., Remington's Pharmaceutical Sciences, 15th Ed., Easton ed. , Mack Publishing Co., pp 1405-1412 and 1461-1487 (1975); The National Formulary XIV, 14th Ed., American Pharmaceutical Association, Washington, DC (1975) . Examples of non-aqueous solvents are propylene glycol, polyethylene glycol, vegetable oil and injectable organic esters such as ethyloleate. Aqueous carriers include water, alcoholic/aqueous solutions, saline solutions, parenteral vehicles such as sodium chloride, Ringer's dextrose. Intravenous vehicles include fluid and nutrient replenishers. Preservatives include antimicrobial agents, anti-oxidants, chelating agents and inert gases. The pH and exact concentration of the various components the pharmaceutical composition are adjusted according to routine skills in the art.

Repeated administrations of a particular vaccine (homologous boosting) have proven effective for boosting humoral responses. Such an approach may not be effective at boosting cellular immunity because prior immunity to the vector tends to impair robust antigen presentation and the generation of appropriate inflammatory signals. One approach to circumvent this problem has been the sequential administration of vaccines that use different antigen-delivery systems (heterologous boosting).

In a heterologous boosting regimen, at least one prime or boost delivery comprises delivery of the liposomal formulations described herein. The heterologous arm of the regimen may comprise delivery of antigen using one or more of the following strategies:

attenuated and/or inactivated bacteria or viruses comprising the antigen of interest, which are particles that have been treated with some denaturing condition to render them ineffective or inefficient in mounting a pathogenic invasion;

purified antigens, which are typically naturally-produced antigens purified from a cell culture of the pathogen or a tissue sample containing the pathogen, or a recombinant version thereof;

live viral or bacterial delivery vectors recombinantly engineered to express and/or secrete antigens in the host cells of the subject. These strategies rely on genetically engineering the viral vectors to be non-pathogenic and non-toxic;

antigen presenting cell (APC) vectors, such as a dendritic cell (DC) vector, which comprise cells that are loaded with an antigen, or transfected with a composition comprising a nucleic acid encoding the antigen;

tumor cells, for example, autologous and allogeneic tumor cells; and

naked DNA vectors and naked RNA vectors which may be administered by a gene gun, electroporation, bacterial ghosts, microspheres, microparticles, liposomes, polycationic nanoparticles.

A prime vaccine and a boost vaccine can be administered by any one or combination of the following routes. In one aspect, the prime vaccine and boost vaccine are administered by the same route. In another aspect, the prime vaccine and boost vaccine are administered by different routes. The term "different routes" encompasses different sites on the body, for example, a site that is oral, non-oral, enteral, parenteral, rectal, intranode (lymph node), intravenous, arterial, subcutaneous, intramuscular, intratumor, peritumor, intratumor, infusion, mucosal, nasal, in the cerebrospinal space or cerebrospinal fluid, as well as by different modes, for example, oral, intravenous, and intramuscular.

An effective amount of a prime or boost vaccine may be given in one dose, but is not restricted to one dose. Thus, the administration can be two, three, four, five, six, seven, eight, nine, ten, eleven, twelve, thirteen, fourteen, fifteen, sixteen, seventeen, eighteen, nineteen, twenty, or more, administrations of the vaccine. Where there is more than one administration of a vaccine the administrations can be spaced by time intervals of one minute, two minutes, three, four, five, six, seven, eight, nine, ten, or more minutes, by intervals of about one hour, two hours, three, four, five, six, seven, eight, nine, ten, 11, 12, 13, 14, 15, 16, 17, 18, 19, 20, 21, 22, 23, 24 hours, and so on. In the context of hours, the term "about" means plus or minus any time interval within 30 minutes. The administrations can also be spaced by time intervals of one day, two days, three days, four days, five days, six days, seven days, eight days, nine days, ten days, 11 days, 12 days, 13 days, 14 days, 15 days, 16 days, 17 days, 18 days, 19 days, 20 days, 21 days, and combinations thereof. The invention is not limited to dosing intervals that are spaced equally in time, but encompass doses at non-equal intervals, such as a priming schedule consisting of administration at 1 day, 4 days, 7 days, and 25 days, just to provide an example.

Examples

The following examples serve to illustrate the present invention. These examples are in no way intended to limit the scope of the invention.

Example 1. Influenza A Virus Materials and Methods

A. IAVM2e1-HD pET28a vector

The IAVM2e1-HD pET28a plasmid design has been previously described [Ernst et al., 2006].

B. IAVM2e1-HD pEV vector

Table 1. Peptide and oligonucleotide sequences of the different IAVM2e1 sequences evaluated

| Construct | Amino Acid Sequence | Oligonucleotide sequence |
|---|---|---|
| IAVM2e1 5' | SLLTEVETPIRNEWGCRCNDSSD (SEQ ID NO: 43) | ATCAGATCT CGAAAACCTGTACTTCCAGTCCCTGCTGACCGAAGTTGAGA (SEQ ID NO: 44) |
| IAVM2e1 3' (C16S) | SLLTEVETPIRNEWGSRCNDSSD (SEQ ID NO: 45) | CGCAAGCTTGTCGACGTCGGAGGAGTCGTTACAACGAGAACCC (SEQ ID NO: 46) |
| IAVM2e1 3' (C18S) | SLLTEVETPIRNEWGCRSNDSSD (SEQ ID NO: 47) | CGCAAGCTTGTCGACGTCGGAGGAGTCGTTAGAACGAC (SEQ ID NO: 48) |
| IAVM2e1 3' (C16S, C18S) | SLLTEVETPIRNEWGSRSNDSSD (SEQ ID NO: 49) | CGCAAGCTTGTCGACGTCGGAGGAGTCGTTACTACGACTACCCCA (SEQ ID NO: 50) |
| IAVM2e1 (1-15) 3' | SLLTEVETPIRNEWG (SEQ ID NO: 51) | ACAAAGCTTACCCCATTCGTTACGGATCGGGGTCTCAACTTCGGT (SEQ ID NO: 52) |
| IAVM2e(1-15)-HD | SLLTEVETPIRNEWGKLAAANKPPETLITTIDSSSSWWTNWVIPAISAVAVALMYRLYMAED(SEQ ID NO: 53) | |

(continued)

| Construct | Amino Acid Sequence | Oligonucleotide sequence |
|---|---|---|
| IAVM2e(1-15)-XD | SLLTEVETPIRNEWGKLQKPS ETFITTTDSDSSWWSNWIIPGI SAMIVALMYRFYMVSE (SEQ ID NO: 54) | |

To generate the IAVM2e1-HD protein, a 5' oligonucleotide primer was synthesized encoding the IAVM2e1 gene containing a Bgl II restriction enzyme site and an enterokinase (EK) site (Integrated DNA Technologies). To generate the LDR-IAVM2e1-HD protein, a 5' oligonucleotide primer was synthesized encoding the IAVM2e1 gene containing a Bgl II restriction enzyme site, an EK site and the LDR sequence of the pET28a vector (Integrated DNA Technologies). Similarly, a 3' oligonucleotide primer encoding the gene for the hydrophobic domain was synthesized containing an Xho I restriction enzyme site. To generate the IAVM2e1-HD gene, the primers were used to polymerase chain reaction (PCR) amplify the IAVM2e1-HD pET28a vector template, previously constructed by Molecular Express. Briefly, to a PCR tube, 7.5 μL of 10X Pfx (Invitrogen) buffer, 1 μL of 50 mM MgCl2, 1.5 μL of 10 mM dNTP, 1 μL of a 20 μM 5' and 3' primer, 10 ng of IAVM2e1-HD pET28a plasmid, 1 μL of Pfx polymerase and qs. to 50 μL. The reaction mix was cycled 25 times (94°C for 1 minute, 58°C for 1 minute, 72°C for 1 minute) and then a final extension at 72°C for 7 minutes. The PCR product was purified using a PCR purification kit (Qiagen, Chatsworth, CA). The PCR product and pEV1 vector were enzymatically digested with Bgl II and Xho I (Invitrogen). The pEV1 vector is a composite vector of pET32b and pET28a, in which, the thioredoxin-6His-multiple cloning site of pET32b was inserted into the pET28a. The pEV1 vector provides the thioredoxin-6His and thrombin cleavage site of the pET32b and under the kanamycin resistance of the pET28a vector. The digested DNA was gel purified (Qiagen, Chatsworth, CA) and then ligated and transformed into DHS-α *E. coli.* The plasmid was purified from the transformed *E. coli* and sequenced to ensure proper sequence, frame and orientation of the gene.

C. Truncated and cysteine mutation IAVM2e1-HD constructs

To generate the mutated IAVM2e1-HD proteins, a 5' primer was synthesized encoding a Bgl II restriction enzyme site, a tobacco etch virus (TEV) protease site and the first 22 bases of the IAVM2e1 sequence (Integrated DNA Technologies). Similarly, 3' primers encoding the sequence for the various mutated IAVM2e1 genes and the truncated IAVM2e1(1-15) were synthesized containing a Hind III restriction enzyme site (Table 1). The vector for these constructs contains the HD from a previous construct, which has a HinD III site 5' of the region that encodes the HD.

For each cysteine to serine mutant, a reaction was set up using the 5' primer and one of the 3' primers to amplify the IAVM2e1 region from the pEV1-IAVM2e1 template previously constructed by Molecular Express. Briefly, to a PCR tube, 7.5 μL of 10X Pfx polymerase buffer, 1 μL of 50 mM MgCl2, 1.5 μL of 10 mM dNTP, 1 μL each of 20 μM 5' and 3' primer, 10 ng of IAVM2e1-HD pEV1, 0.75 ul of Pfx (Invitrogen) were added and qs. to 50 μL with distilled water. The reaction mix was heated to 94°C for 5 min to activate the polymerase. It was then subjected to 30 cycles of 95.5°C for 20 sec, followed by 70°C for 40 sec. After cycling, it was incubated for a final extension period of 10 minutes at 68°C, then held at 4°C. The PCR product was purified using the Qiaquick Spin Kit (Qiagen). It was then digested using 5 units each of Bgl II and HinD III in IX React 2 buffer (Invitrogen) for 30 min at 37°C. Each of the products was gel purified on a 3.5% Nuseive agarose gel. The bands were excised from the gel and the DNA fragments were purified using the Qiaquick Gel Extraction kit. The DNA concentrations were determined by analysis on a 2% agarose gel next to a known amount of Low Mass DNA standards with varying amounts of DNA.

For the deletion mutant which includes only amino acids 1-15, the insert is short enough that the lower primer overlaps the upper primer by 13 bases. In this case, a similar reaction was set up in a PCR tube with the two primers, but no template DNA was added. After the 5 minute denaturation phase to activate the polymerase, the reaction was incubated at 48°C for 2 min, then the temperature was increased to 70°C at 20% ramp speed and held there for 5 minutes. The 48°C and 70°C steps were repeated once more, followed by a 10 minute extension step at 70°C. The resulting product was gel purified and analyzed by the same procedures as the slightly longer, mutated IAVM2e1 PCR products.

The pEV1 vector with an HD was prepared by digesting 3 μg of Molecular Express Plasmid (MEP)-180 in 55 μL of IX React 2 overnight at 37°C with 24 units Bgl II and 10 units HinD III The vector was gel purified on a 1% agarose gel. The excised band was purified using the Qiagen Gel Purification Kit. The DNA concentration was determined by running it on a 1% agarose gel next to a known amount of High Mass DNA Standards.

Each of the digested, purified PCR products was then ligated to the vector in a 10μL reaction containing a 3-fold molar ratio of PCR product to vector, and 1x ligation mix (Novagen Clonables Ligation/Transformation Kit) at 16°C for 80 min. One microliter of this reaction was then transformed into Novablue chemically competent cells, plated on LB-agarose plates containing 30 mg/L Kanamycin and incubated overnight. Because the IAVM2e1 sequence is so short, colonies were analyzed using both colony PCR and restriction analysis of the PCR products. The PCR reaction was carried out using Molecular Express oligonucleotide (MEO) primers MEO-248 and MEO-250. The MEO-248 primer site begins 540 bp upstream of the Bgl II site and MEO-250 ends 250 bp downstream of the HinD III site at the 5' end of the HD. The 23 amino acid sequence of IAVM2e1 plus the 7 amino acids of the TEV site require 90 bp. PCRs that rendered a fragment of approximately 900 bp were digested with Bgl II and HinD III in two separate additional reactions and analyzed on 3.5% agarose gels. If the gel indicated that both cut sites were present, the colony was grown up overnight in 16 ml of LB with 30 mg/L kanamycin, 225 rpm, 37°C. Plasmid DNA (Qiagen Plasmid Miniprep Kit) was purified for verification by sequencing using primers MEO-302 and MEO-250 (Davis Sequencing). The sequencing results were compared to the intended DNA sequence using the "Align 2 or more Sequences using BLAST.

SEQ ID NOS: 53 and 54 depict IAVM2e1-HD constructs which contain hydrophobic domains obtained from human cytochrome b5 and Xenopus MGC80327 protein, respectively (underlined). A short linker sequence of amino acids (double underline) resulting from the restriction sites employed during preparation of the construct links the cytochrome b5 or Xenopus MGC80327 residues to the IAVM2e1 sequence. The IAVM2e1 sequence (no underline) is thus indirectly fused to the amino terminus of the hydrophobic domain via bridging amino acid residues.

D. Expression of IAVM2e1-HD constructs

To express the IAVM2e1-HD protein, the IAVM2e1-HD containing pEV1 plasmids or the IAVM2e1-HD-pET28a plasmids were transformed into the *E. coli* expression strain C-43 (Lucigen). A starter culture was grown in animal-free LB broth (AthenaES) supplemented with 30 mg/L kanamycin overnight in a G-25 environmental shaker (New Brunswick Scientific) at 37°C. To 4-2 L sterile shaker flasks containing 0.5 L of animal-free LB broth supplemented with 30 mg/L kanamycin and 1% glycerol, the starter culture was added to an optical density (O.D.) of 0.09-0.12 (A600nm). The cultures were incubated at 37°C with orbital shaking (225 rpm) to 0.5-0.7 O.D. (A600nm), at which time the culture was induced with 0.75 mM isopropyl β-D-1-thiogalactopyranoside (IPTG) (BioWorld). The culture was incubated for an additional 5 hours at 37°C shaking. The bacteria were collected by centrifugation (4000xg) for 25 minutes at 4°C and the pellet weighed.

E. Purification of 6His-IAVM2e1-HD

To purify the 6His-IAVM2e1-HD protein, the bacteria were lysed in 8 M urea, 20 mM imidazole, 50 mM Tris pH 8.0 buffer and homogenized three times at 15,000 psi in a microfluidizer M-110L (Microfluidics) to shear the bacterial DNA and rupture the bacteria. The lysed bacteria were then centrifuged (30,000xg) to remove cellular debris. Purification was achieved by incubating washed nickel-charged chelating agarose resin (Qiagen) with the supernatant containing the IAVM2e1-HD protein. The resin was washed with 5 column volumes of urea buffer containing 20 mM imidazole then washed with five column volumes of 0.3% sodium deoxycholate and finally eluted with 250 mM imidazole, 50 mM sodium phosphate pH 7.9. Protein fractions were electrophoresed on a 4%-12% SDS-PAGE and stained with Coomassie Blue to identify the fractions that contained the 6His-IAVM2e1-HD protein. The 250 mM imidazole elution fractions containing the 6His-IAVM2e1-HD protein were pooled and incubated with 0.1 ml of packed polymyxin B-agarose to remove residual endotoxin. After polymyxin B-agarose treatment, the protein was dialyzed against 10 mM sodium phosphate, pH 7.2 to remove residual imidazole, urea and sodium deoxycholate. The protein concentration and endotoxin concentration were determined by BCA and Limulus Amoebacyte Assay (Charles River Lab.), respectively.

F. Purification of IAVM2e1-HD from the pEV1 plasmid

The *E. coli* pellet was suspended with 4 volumes (wt/vol) of 10 mM Tris buffer, pH 8.1; 20% sucrose; 1 mM ethylenediaminetetraacetate (EDTA). Once the bacteria were fully suspended, lysozyme powder (Sigma) was added to a concentration of 1 mg/ml and stirred at room temperature for 20 minutes. The spheroplasts were collected by centrifugation at 30,000xg for 30 minutes at 4°C. The pellet was resuspended in ice cold distilled water containing 1 mM phenylmethanesulphonylfluoride (PMSF), DNase (10 units/ml) and stirred for 10 minutes. The membranes were collected by centrifugation at 30,000xg for 30 minutes at 4°C. The pelleted membranes were resuspended in 300 ml of 10 mM Tris buffer pH 8.1; 1 mM PMSF. While stirring, 3-[(3-Cholamidopropyl)dimethylammonio]-1-propanesulfonate (CHAPS) (Sigma) (20% stock) was added drop-wise to a final concentration of 1%. The mixture was homogenized three times at 15,000psi in a microfluidizer M-110L (Microfluidics) and then stirred for an additional 16 hours at 4°C. The mixture was then centrifuged at 30,000xg for 30 minutes at 4°C to remove particulates. To the cleared lysate, sodium chloride and

imidazole were added to a final concentration of 500 mM and 20 mM, respectively. The lysate was then incubated with packed Ni-NTA resin (Qiagen) for 45 minutes at 4°C with rotation. The resin was collected by centrifugation at 225xg for 5 minutes and transferred to a Kontes 2.5 cm x 25 cm column and washed with 10 column volumes of 10 mM Tris buffer pH 8.1, 20 mM imidazole, 500 mM NaCl and 1% CHAPS. The protein was eluted with 250 mM imidazole, 20 mM Tris pH 8.0 and fractions collected and analyzed by Coomassie Blue stained SDS-PAGE. Fractions containing the target protein were pooled and dialyzed against 2 mM Tris, 10 mM NaCl, pH 8.1 with a 15 kDa molecular weight cut off (MWCO) (Spectra-Por).

G. SDS-PAGE

Identity of the IAVM2e1-HD protein was determined by demonstrating the appropriate MW on SDS PAGE gels stained with coomassie blue. Concentration of impurities in the test sample was estimated by comparing staining intensity of these bands to the staining intensity of the reference protein of known concentration.

H. HPLC analysis of Trx-6-His-IAVM2e1-HD

HPLC analysis of protein samples were performed using a Dionex GP50 HPLC system. The system consisted of a GP50 gradient pump, GM-3 gradient mixer, AS50 autosampler, and PDA-100 photodiode array detector. Components were separated by RP-HPLC on a Dionex Acclaim 120 C8 column (4.6x250 mm; 5 μm; 120 A) using acetonitrile gradient elution at 1 ml/min, 25°C : solvent A (10% acetonitrile, 0.1% trifluoroacetic acid); solvent B (90% acetonitrile, 0.1% trifluoroacetic acid); (0 min, 40% B); (1 min, 40% B); (25 min, 100% B). Detection of components was by UV absorbance at 220 nm. Typically, 20 μL of a sample was injected per analysis. The thioredoxin-IAVM2e(1-15)-HD fusion protein elutes at approximately 11-12 min. A conversion factor of 1.17x10-4 mg/mAU220nm*min was used to estimate the content of fusion protein from the 11-12 min peak area. IA VM2e(1-15)-HD elutes at approximately 14 min; a conversion factor of 7.27x10-5 mg/mAU220nm*min was used to estimate the content of IAVM2e(1-15)-HD from the 14 min peak area.

I. Protease cleavage of the Ni-NTA purified protein

The recombinant proteins containing the enterokinase cleavage site were cleaved with 10 ng of enterokinase per milligram of protein (New England Biolabs) or with 10 units of TEV per milligram of protein (Invitrogen).

J. Ultra-filtration to concentrate IAVM2e1-HD and remove thioredoxin

The cleaved protein solution was concentrated by ultrafiltration through an Amicon UF cell (Millipore) using a 30 kDa MWCO PES membrane (Millipore) to an IAVM2e1-HD concentration of approximately 20mg/ml. The concentrated sample was then washed 4 times (1/10 dilution each) with 10 mM sodium phosphate buffer, pH 7.0 to remove the bulk of the thioredoxin component in the flow-through.

K. HPLC purification IAVM2e1-HD from pEV1

HPLC purification of IAVM2e1-HD and LDR-IAVM2e1-HD was performed using a Dionex Ultimate 3000 HPLC system. The system consists of an Ultimate 3000 pump and an Ultimate 3000 variable wavelength detector. The protein was separated by RP-HPLC on a Vydac C8 column (22x250 mm; 10 μm; 300 A) using acetonitrile gradient elution: solvent A (water, 0.1% trifluoroacetic acid); solvent B (acetonitrile, 0.1% trifluoroacetic acid); (0 min, 5% B); (1 min, 5% B); (60 min, 100% B); (65 min, 100% B); (70 min 5% B); (75 min, 5% B). Detection of components was by UV absorbance at 220 nm.

L. Ultrafiltration to concentrate purified IAVM2e1-HD

HPLC fractions containing IAVM2e1-HD was concentrated to approximately 20 mg/ml working concentration by ultra-filtration through an Amicon UF cell (Millipore) using a 5 kDa MWCO regenerated cellulose membrane (Millipore) for monomeric IAVM2e1-HD and 10 kDa MWCO regenerated cellulose membrane (Millipore) for dimeric IAVM2e1-HD. The concentrated protein sample was buffer exchanged to 10 mM sodium phosphate buffer, pH 7.0 by a series of 4 washes (1/10 dilution each). The final samples were stored at 4°C.

M. Endotoxin concentration

The endotoxin concentration was determined by the Limulus Amoebacyte Lysate (LAL) assay (Charles River

Lab). Eight 10-fold dilutions of the IAVM2e1-HD protein preparations and eight 2-fold dilutions of a control endotoxin standard were prepared using endotoxin-free sterile water. The control and IAVM2e1-HD samples were aliquoted into sterile endotoxin-free 10x75mm borosilicate glass tubes and added with equal volume (100 μE) of the LAL. In addition, 100 μL of LAL were added to 100 μL of endotoxin-free sterile water for injection as a negative control. The endotoxin concentration (EU/mL) in a sample was calculated by multiplying the LAL labeled sensitivity by the reciprocal of the first dilution that did not show a clot.

N. IAVM2e1-HD purity and sequence

The purity of the protein was assessed by ion spray mass spectroscopy (LC/MS/MS) (Midwest Bio Services). The sequence of the protein was verified by trypsin cleavage of the IAVM2e1-HD followed by ion trap mass spectroscopy (Midwest Bio Services).

O. Western Blot analysis

To verify the purified protein was the target recombinant protein (IAVM2e1-HD), a sample of the purified protein (2μg) was run on a 4%-20% SDS-PAGE (Invitrogen). The proteins were transferred to a 0.2μM nitrocellulose membrane (NCM) (BioRad) in a Tris-Glycine-Methanol buffer for 1.5hr at 100mAmps. The NCM was blocked with 3% bovine serum albumin (BSA) (EMD, PN 2960) for 16 hours at 4°C. To detect the IAVM2e1-HD, the NCM was incubated with a 1:1000 dilution of anti-M2e monoclonal antibody (mAb) (Pierce Cat #MA1082) diluted in 1.5% BSA in Tris buffered saline containing 0.2% Tween-20 pH 7.4 (TTBS). The blot was incubated with a secondary antibody, goat anti mouse IgG-HRP conjugated (1:7500) in TTBS. The blot was incubated for an additional 1 hour with rocking. The blot was washed three times with 20 ml of TTBS for 15 minutes. The blot was then developed by adding 10 ml of tetramethyl benzidine (TMB) solution (Pierce, Cat # 34108) and incubated for 15-30 minutes without shaking. The reaction was stopped by gently rinsing the NCM with distilled water.

P. Liposome preparation

Dimyristoylphosphatidyl choline (DMPC, Lipoid), cholesterol (Chol, Nippon Oil and Fat), dimyristoylphosphatidyl glycerol (DMPG, Nippon Fine Chemicals), monophosphoryl lipid A (MPL) were dissolved in chloroform, methanol (1:1 ratio) and the IAVM2e1-HD protein added to a 65:1 lipid to protein concentration. Since three different doses were given for the test samples, three different preparations with different concentrations were prepared. In this way, the same volume of dose could be given to each animal. An identical sample of lipids and MPL was prepared without protein as a control (L-control) (Table 2). The lipids and proteins were dried under a stream of nitrogen. The dried lipid films were placed under vacuum for 48 hours to remove residual organic solvents. To the dried lipid films, an appropriate amount of buffer (sodium phosphate (10mM) buffer, 9% sucrose pH 7.4) was added that resulted in a concentration of 0.6, 0.3 or 0.1 mg/ml IAVM2e1-HD. The lipid protein mixture was heated at 60°C for -10 minutes, vortexed for 15 seconds then probe sonicated at 10-20% power on a Branson Sonifier for 3-5 minutes, until translucent to prepare the liposomes. The liposomes were sterile filtered through a 0.2 μM polyethersulfone syringe filter into sterile, pyrogen free tubes and labeled in a laminar flow hood. The liposome preparations were transferred from Molecular Express, Inc. to Dr. Jill Adler-Moore (Cal Poly Pomona) in person and signed for as receipt of the liposomes. The liposomes were transported on ice and stored at 4°C at Cal Poly Pomona. Liposome size distribution was determined by Microtrac (Microtrac-UPA150, Honeywell). Protein concentration of each liposomal preparation was confirmed by HPLC assay.

Table 2. L-IAVM2e1-HD formulations

| | **[Lipid] (mg/ml)** | **[Protein] (mg/ml)** | **[MPL] (mg/ml)** |
|---|---|---|---|
| LDR-IAVM2e1-HD(pET28a) | 19.5 | 0.3 | 0.3 |
| LDR-IAVM2e1-HD(pEV1) | 19.5 | 0.3 | 0.3 |
| IAVM2e1(C16S, C18S)-HD | 39 | 0.6 | 0.3 |
| IAVM2e1(C16S, C18S)-HD | 19.5 | 0.3 | 0.3 |
| IAVM2e1(C16S, C18S)-HD | 6.5 | 0.1 | 0.3 |
| IAVM2e(1-15)-HD | 39 | 0.6 | 0.3 |
| IAVM2e(1-15)-HD | 19.5 | 0.3 | 0.3 |

(continued)

| | [Lipid] (mg/ml) | [Protein] (mg/ml) | [MPL] (mg/ml) |
|---|---|---|---|
| IAVM2e(1-15)-HD) | 6.5 | 0.1 | 0.3 |
| IAVM2e1(C16S)-HD | 39 | 0.6 | 0.3 |
| IAVM2e1(C16S)-HD | 19.5 | 0.3 | 0.3 |
| IAVM2e1(C16S)-HD) | 6.5 | 0.1 | 0.3 |
| IAVM2e1(C18S)-HD) | 39 | 0.6 | 0.3 |
| IAVM2e1(C18S)-HD) | 19.5 | 0.3 | 0.3 |
| IAVM2e1(C18S)-HD) | 6.5 | 0.1 | 0.3 |
| L-control | 19.5 | 0.3 | 0.3 |

Q. Influenza virus infection model

In the influenza challenge model, groups (n=17/group) of BALB/c mice were immunized subcutaneously week 0 and intranasally week 8 with L-IAVM2e1-HD vaccine or an equivalent dose of L-control (liposomes with all components except the protein). Five of the unchallenged mice were sacrificed on day 6 and sera collected for ELISA assays (described below). The remaining twelve of the immunized mice were challenged intranasally (40μL per challenge dose) at one week post-boost (week 9) with 10 LD50 of IAV (H1N1 (A/PR/8/34) isolated from infected lung homogenates. Viral challenge was performed by first anesthetizing the animals intraperitoneally with a mixture of Ketamine (80 mg/kg) and Xylazine (16 mg/kg). Using a 200 μL micropipette tip, virus was administered directly onto the nares of the mice, allowing them to naturally inhale the virus. Seven animals were monitored over 28 days for signs of morbidity (weight loss, lack of grooming and reduced activity) and mortality. Five mice were sacrificed on day 5 post-challenge and their lungs assayed for viral burden by the foci assay (described below).

R. Antibody titers and isotype characterization by ELISA

From five mice/group, the serum was collected 6 days after the last immunization. Briefly, mice were deeply sedated by intraperitoneal injection with a mixture of Ketamine (120 mg/kg) and Xylazine (20 mg/kg) and the blood was removed by cardiac puncture. The blood was transferred to a microcentrifuge tube that had been rinsed with Heparin. The blood was centrifuged at 1500 rpm for 20 minutes to separate the serum from the blood cells. The collected serum was aliquoted and stored at -80°C until use.

The serum collected from the mice by cardiac puncture was used to assay for the antibody isotype titer to IAVM2e1 by OptEIA ELISA assays (PharMingen, Inc.). Briefly, the procedure involves using flat-bottom 96-well microtiter Immulon plates incubated at 4°C overnight with 100μL/well coating buffer (65mM Na2CO3, 100mM NaHCO3, pH 9.5) containing 5μg/well of the IAVM2e1 protein (UCLA Peptide Synthesis Core Facility). The plates were rinsed 3X with 275μL wash buffer (PBS with 0.05% Tween-20) and blocked by adding 275μL blocking buffer (1% fetal bovine serum in PBS) to each well and incubating the plate for 2h at room temperature. The plate was then washed 7X with 275μL wash buffer/well and 100μL diluted mouse serum samples (diluted in blocking buffer) were added to each well and incubated for 2h at room temperature. Five minutes before the end of the 2h incubation, the biotin-labeled detection antibody (anti-mouse immunoglobulin isotype antibody: anti-IgG1, anti-IgG2a, anti-IgG2b, IgG3, anti-IgA) was prepared in blocking buffer. The wells were washed 7X with 275μL/well of wash buffer, and 100μL of biotin anti-mouse immunoglobulin isotype antibody was added. The plate was sealed and incubated for 1h at room temperature. The wells were washed 7X with 275μL wash buffer, allowing the buffer to remain in the well for one minute for each wash prior to its removal. Streptavidin-HRP diluted with blocking buffer to 1:1000 was added to each well (100μL/well) and incubated for 1h at room temperature, followed by washing 7X with 275μL wash buffer. The substrate reagent (Peroxidase detection reagent) was added to each well (100μL/well), and incubated in the wells for 40 minutes at room temperature in the dark. The Stop solution (1M H3PO4) was added to each well (100μL/well) and the plates were read with a Spectramax plate reader at 450nm. The antibody titer was calculated as the reciprocal of the last serum dilution giving an OD reading above background (wells not coated with IAVM2e1).

S. Virus neutralization assay

Sera from immunized mice was diluted in PBS and 50μL of each serum dilution was added to 50μL of a

1:100-1:120 dilution of H1N1 virus in quadruplicate wells in a 96-well round bottom plate. The plate was incubated in a humidity chamber at 37°C with 5% CO2 for 8h. To a 24-well flat-bottom plate, 200 µL of 1x106 MDCK cells/ml, and 400 µL of virus/serum dilution mixture (pooled material from four wells of the 96-well plate) was added to each well. The 24-well plate was incubated at 37°C with 5% CO2 for 48h. The cells were then fixed with 250ml of 100% methanol and incubated at room temperature for 20 minutes. The methanol was then decanted and the cells stained for 10 minutes at room temperature with 250 ml of 0.1% crystal violet in 20% ethanol solution. The cells were rinsed with distilled water and dried at room temperature. The neutralization titer was determined as the lowest dilution capable of preventing 50% MDCK cytolysis by the H1N1 virus. To determine the 50% MDCK cytolysis, the density of each well in the plate was calculated using a UVP Gel DOC system.

T. In vitro viral foci (or "plaque") assay

To assess the viral titer in the lungs, the lungs from five mice in each group were collected on day five post-challenge, homogenized and ten-fold serial dilutions of the homogenized lungs were prepared on ice in CMEM (MEM, 10% FBS and 1% penicillin-streptomycin). A 200 µL aliquot of the lung sample was mixed with 200µL of MDCK cells (7x105 - 1x106 cells/ml) in 24 well plates and incubated at 37°C, 5% CO2 to allow the MDCK cells to adhere to the bottom of the plates. After 6h, 300 µL of 3% methylcellulose diluted in CMEM was added to each well. The plates were incubated for 40-48 h at 37°C, 5% CO2 incubator. To visualize formation of the viral foci, an immunohistochemical staining procedure was performed. The 3% methylcellulose was removed and plates were washed 1X with PBS. Then, 400µL of 4% formaldehyde in PBS was added to each well to fix the cells. The plates were incubated for 30 minutes at room temperature and the fixative was removed and the cells permeabilized with 400 µL per well of 0.5% Triton X-100 in PBS. Plates were washed 2X with PBS and then incubated with PBS, 10% FBS for 90 minutes at room temperature. After the incubation, plates were washed 1X with PBS and 150 µL per well of monoclonal anti-NP-influenza antibody (diluted 1:100 in PBS) was added. After incubating for 90 minutes at room temperature, the plates were washed 7X with PBS and 150 µL of horse-radish peroxidase-labeled anti-IgG (diluted 1:1000 in PBS, 10%FBS) were added to each well. The plates were incubated for 90 minutes, washed 7X with PBS and 300 µL of the developing substrate added (25.7 ml Na2HPO4 (0.1M) mixed with 24.3 ml citric acid (0.05 M) and 20 mg diaminobenzidine and 20 µL of H2O2). Plates were incubated for 10 to 30 min and the reaction stopped by washing the plates with distilled water. The number of foci forming units (i.e., number of localized areas of brown-colored cells in each well) were counted using the inverted microscope. The number of foci in each well were multiplied by the dilution factor and used to determine the foci or "plaque" forming units/ml.

U. Statistical analysis.

Statistical analysis utilized the ranked ANOVA, Kaplan-Meier, Mann-Whitney rank sum test or Wilcoxon's test. The particular test(s) selected will depend on the type of data/sample being analyzed.

Example 2. Results

In this study, we constructed four new IAVM2e1-HD constructs in addition to the pET28a-IAVM2e1-HD; (i) pEV1 IAVM2e1-HD with the LDR sequence described above, (ii) pEV1-IAVM2e1(C16S,C18S)-HD (iii) pEV1-IAVM2e1(C16S)-HD, (iv) pEV1-IAVM2e1(C18S)-HD and (v) pEV1-IAVM2e1(1-15)-HD. All new plasmids were sequenced and verified to be in-frame and contain the correct sequence. The PCR products for each of the IAVM2e1-HD constructs indicates that each of the PCR products correspond with the expected size of the respective IAVM2e1-HD insert. The plasmids were transformed into the *E. coli* strain C-43 and expressed. Small-scale expression studies were done to verify that the constructs were expressing the protein. Unlike the original pET28a-IAVM2e1-HD construct, the pEV containing IAVM2e-HD constructs showed high levels of protein expression compared to the respective uninduced controls. To verify that the target M2e protein was expressed, a Western blot analysis was performed. The IAVM2e1 proteins were developed with the anti-M2e mAb. In contrast, the negative control B. anthracis Protective Antigen was not detected as expected. Multiple bands were seen in several of the IAVM2e1 samples indicating that even in the presence of SDS and β-mercaptoethanol, the IAVM2e1-HD can form multimers.

The membrane fractions were further processed then subjected to Ni-NTA affinity purification. Thioredoxin-IAVM2e-HD fusion protein fractions were eluted with 20 mM Tris, 250 mM imidazole, pH 8. The fractions were evaluated by SDS-PAGE and the fractions containing the target protein were pooled and further purified. Processing of the various recombinant thioredoxin-IAVM2e1-HD fusion proteins and purification of the various IAVM2e1-HD peptides were similarly performed. The purification of the IAVM2e1(1-15)-HD peptide is described in detail here as a representative data set. Samples of the Ni-NTA affinity purified fusion proteins, in elution buffer (20 mM Tris, 250 mM imidazole, pH 8), were analyzed by HPLC to assess protein contents. A representative HPLC chromatogram, of the thioredoxin-IAVM2e1(1-15)-HD fusion protein sample showed two major components eluting at approximately 9 and 12 min; the large peak at the solvent front (3 min) comprised mainly of residual imidazole and more polar components. The 9 min component was a major contaminant with an apparent molecular weight of approximately 17 kDa (data not shown). The 12 min component was the target thioredoxin-IAVM2e1(1-15)-HD fusion protein.

To concentrate the samples and deplete contaminating small molecules such as imidazole and/or other components, the Ni-NTA affinity purified fusion protein samples were ultra-filtered and buffer exchanged into 10 mM sodium phosphate buffer, pH 8. Ultra-filtration was accomplished by use of an Amicon ultra-filtration stirred-cell, 50 ml capacity, with a 50 kDa MWCO PES membrane. Typically, 4 or more ultra-filtration cycles were performed as described above, resulting in 10,000 or greater fold dilution of the elution buffer and its components. The flow-through (pooled) and retentate fractions from an ultrafiltration process were analyzed by HPLC. By this method, most of the contaminating component at 9 min can be removed. The retentate fraction retained most of the 12 min fusion protein component. The ultrafiltration process took advantage of a previously observed, but uninvestigated phenomenon, in which low molecular weight HD proteins can be retained by high MWCO membrane. In this case, monomeric fusion protein of ~24.0 kDa can be retained by the 50 kDa MWCO PES membrane, while other components, including the ~17 kDa contaminant, flowed through. It is proposed that the - HD component of the protein assembles into stable multi-subunit complexes or aggregates larger than 50 kDa, enabling retention of -HD proteins in the retentate fraction.

The fusion protein was subsequently cleaved with TEV protease to detach the thioredoxin component from the IAVM2e1(1-15)-HD peptide by use of 5 units of TEV protease per 1 mg of fusion protein over 18 hr at 25°C, in the presence of 0.5 mM EDTA, 10 mM β-mercaptoethanol. After cleavage of the fusion protein by TEV protease to yield the thioredoxin and IAVM2e(1-15)-HD components, the sample was subjected to Amicon stirred-cell an ultrafiltration through a 50 kDa MWCO PES membrane to partially deplete the thioredoxin components. The ultra-filtered sample showed that the thioredoxin component at 8-9 min has been depleted by greater than 85% with near 100% retention of the residual fusion protein at 11-12 min and IAVM2e1(1-15)-HD at 14 min.

The retentate from the ultra-filtration process, containing the putative IAVM2e1(1-15)-HD and significantly depleted of the thioredoxin components, was diluted to approximately 10 mg IAVM2e1(1-15)-HD per mL of 10 mM sodium phosphate, pH 8, then subjected to preparative RP-HPLC to purify IAVM2e1(1-15)-HD from the remaining contaminating components. A 2 mL sample containing approximately 20 mg of IAVM2e1(1-15)-HD was injected onto a preparative C18 column (22x250 mm) and eluted with an acetonitrile gradient containing 0.1% TFA. This method elutes residual thioredoxin components between 30-35 min and uncleaved fusion protein between 35-40 min. Fractions from 40-42 min were typically collected as the IAVM2e1(1-15)-HD peptides. The method appeared to effectively resolve the thioredoxin and fusion protein components from the putative IAVM2e1(1-15)-HD component, anticipated to be highly pure.

The various recombinant IAVM2e1-HD fusion proteins were processed similarly. The TEV protease cleaved samples were subjected to preparative HPLC and monomeric forms of the peptides recovered similarly. Fractions containing various HPLC purified IAVM2e1-HD peptides were analyzed by HPLC to assess purity. Analytical HPLC chromatograms ndicate that relatively pure IAVM2e1-HD peptides eluting between 11 to 15 min were recovered from preparative HPLC fractions, without detectable levels of any other contaminating protein components including thioredoxin and fusion protein.

The IAVM2e1(1-15)-HD and IAVM2e1(C16S,C18S)-HD peptides were designed without cysteine residues and hence, will not be able to form disulfide linked dimers or multimers. As such, these peptides were recovered as monomers by Amicon stirred-cell ultra-filtration through a 5 kDa MWCO regenerated cellulose membrane to retain and buffer exchange the peptides from the acetonitrile/TFA solvent into 10 mM sodium phosphate, pH 8.

The IAVM2e1(C16S)-HD and IAVM2e1(C18S)-HD peptides were designed to contain one cysteine and hence, were able to form dimmers. These peptides were buffer exchanged into 10 mM sodium phosphate, pH 8, then allowed to oxidize at ambient temperature in the presence of air over 72 hr. After oxidation, dimers were purified from the remaining monomers by preparative HPLC. Dimers typically eluted approximately 2-3 min later than monomers, enabling distinct fractions to be collected as purified dimers. Dimer fractions were buffer exchanged by stirred-cell ultra-filtration through 5 kDa MWCO regenerated cellulose membrane. When buffer exchanged into 10 mM sodium phosphate, pH 8, dimers readily precipitated. The final samples were exchanged and recovered into 80% ethanol, which more readily solubilized the dimer peptides.

The LDR-6His-IAVM2e1-HD peptide (from pEV vector) was designed to mimic the pET28a generated IAVM2e1-HD peptide. The pET28a-IAVM2e1-HD peptides were typically purified then lyophilized and reconstituted in buffer at pH 8. Due to the presence of 2 cysteines, it was observed that the pET28a-IAVM2e1-HD peptides readily assume dimeric and multimeric forms as the predominant molecular species. Therefore, purified LDR-6His-IAVM2e1-HD monomers were similarly processed to mimic the formation of dimers and multimers. HPLC purified LDR-6His-IAVM2e1-HD monomers were ultrafiltered to exchange the buffer into 10 mM sodium phosphate, pH 8, then lyophilized and reconstituted with water, maintaining buffering capacity at 10 mM sodium phosphate, pH 8. HPLC analysis of the reconstituted LDR-6His-IAVM2e1-HD suggested significant oxidation of monomers to other species.

The purified IAVM2e1-HD peptides, reconstituted in sodium phosphate buffer or 80% ethanol, were analyzed

by SDS-PAGE under non-reducing conditions (without β-mercaptoethanol to avoid reduction of disulfide linked multimers into monomers) to assess the migration patterns of the peptides. The calculated molecular weights for each peptide are shown in Table 3, column 3.

Table 3. Properties of purified IAVM2e1-HD proteins. NaPi (sodium phosphate). Mass spectrometry by ESI-MS. Calculated mass based on average isotope. Endotoxin level by LAL assay.

| Peptide Construct | Reconstitution solution | Calculated Mass (a.m.u.) | Measured Mass (a.m.u.) | Endotoxin (EU/15 μg) |
|---|---|---|---|---|
| IAVM2e1(1-15)-HD | 10 mM NaPi pH 8 | 6923 | 6925 | 6.9 |
| IAVM2e1(C16S,C18S)-HD | 10 mM NaPi pH 8 | 7986 | 7988 | 0.12 |
| IAVM2e1(C16S)-HD | 80% Ethanol | 16002 (dimer) | 16002 | 0.052 |
| IAVM2e1(C18S)-HD | 80% Ethanol | 16002 (dimer) | 16004 | 0.050 |
| LDR-6His-IAVM2e1-HD | 10 mM NaPi pH 8 | 10312 (monomer)<br>20623 (dimer) | 10314<br>20627 | 0.20 |

The IAVM2e1(1-15)-HD peptide, anticipated to be monomeric, elutes as a singular band with an apparent molecular weight of approximately 5 kDa, consistent with the calculated size of the monomeric species of 6.9 kDa. The IAVM2e1(C16S,C18S)-HD peptide, also anticipated to be monomeric, elutes as a singular band with an apparent molecular weight of approximately 6 kDa, consistent with the calculated size of the monomeric species of 8.0 kDa. The IAVM2e1(C16S)-HD and IAVM2e1(C18S)-HD peptides, anticipated to be dimeric, elute as singular bands between the 14 and 22 kDa markers, consistent with the calculated size of their dimeric form of 16 kDa. Reduction of these samples shows singular bands migrating near the 6 kDa marker, consistent with monomeric forms. The LDR-6His-IAVM2e1-HD peptide, anticipated to contain multiple forms, shows five distinct bands under non-reducing conditions . Reduction of the sample shows only 2 distinct bands, a major one above the 6 kDa marker, consistent with the 10.3 kDa monomeric form, and a minor band just above the 14 kDa marker . The minor band above the 14 kDa marker is likely to be negligible levels of contaminating thioredoxin component. The bands at approximately 20, 30, and 40 kDa are consistent with the molecular size of dimers, trimers, and tetramers respectively. Based on the migration pattern in Figure 6, lane 4, the LDR-6His-IAVM2e1-HD peptide in this preparation appears to be predominantly in monomeric and dimeric forms. Overall, SDS-PAGE analysis indicated relatively pure IAVM2e1-HD peptides of the appropriate molecular species.

To accurately verify the mass of the IAVM2e1-HD peptides, samples were subjected to SEC-ESI-MS. The results are summarized in Table 3, column 3 and 4. Overall, the mass spectrometry data verified that the measured mass of the purified peptides were the same as the calculated mass of the various IAVM2e1-HD peptides, confirming that the targeted IAVM2e1-HD peptides were recovered.

To verify that the purification process could remove endotoxin to negligible levels, the various IAVM2e1-HD samples were assayed for endotoxin levels by the LAL assay. The results are shown in Table 3, column 5, in EU/15 μg protein (anticipated amount of protein per dose in later animal studies). Overall, the levels of endotoxin in the various samples were negligible, with the IAVM2e1(1-15)-HD sample showing highest levels at 6.9 EU/15 μg.

Due to the large number of mice used to test the new constructs, two separate studies of the different L-IAVM2e1-HD formulations were conducted. In each group, the original L-LDR-IAVM2e1-HD (pET28a), L-LDR-IAVM2e1-HD (pEV1, this is an identical protein to the pET28a version, but in a high expressing plasmid) were used as positive controls, and L-control as a negative control. The first group contained the L-IAVM2e1(C16S, C18S)-HD and the L-IAVM2e1(1-15)-HD each of which were predicted to produce monomeric forms of the IAVM2e1-HD protein.

The size distribution varied for each of the different liposome formulations. Overall, the liposomes showed bimodal distributions with one peak ranging from 41.8 nm to 84.5 nm and a second peak ranging from 189.9 nm to 298.2 nm. In contrast, the L-LDR-IAVM2e1-HD (pEV1) preparation showed a unimodal distribution of 188 nm (Table 4). The bimodal size distribution for the liposomal protein samples may be due to the intra-membrane movement of the IAVM2e1-HD at the aqueous surface during the UPA measurement. In contrast, the L-control which contained no protein, has a unimodal, Gaussian size distribution of 57.8 nm.

Table 4. First mouse study of L-IAVM2e1-HD formulations

| | Expected [protein] (mg/ml) | HPLC [protein] (mg/ml) | Size Distribution | |
|---|---|---|---|---|
| | | | Peak #1 nm / % | Peak #2 nm / % |
| L-LDR-IAVM2e1-HD (pET28a) | 0.3 | ND | 68.4 / 80 | 212.0 / 20 |
| L-LDR-IAVM2e1-HD(pEV1) | 0.3 | ND | 188.8 / 100 | |
| L-IAVM2e1(C16S, C18S)-HD | 0.6 | 0.504 | 84.5 / 56 | 298.2 / 44 |
| L-IAVM2e1(C16S, C18S)-HD | 0.3 | 0.276 | 60.5 / 67 | 189.8 / 33 |
| L-IAVM2e1(C16S, C18S)-HD | 0.1 | 0.086 | 73.5 / 48 | 204.9 / 52 |
| L-IAVM2e1(1-15)-HD | 0.6 | 0.541 | 70.1 / 48 | 226.6 / 52 |
| L-IAVM2e1(1-15)-HD | 0.3 | 0.230 | 59.8 / 54 | 241.9 / 46 |
| L-IAVM2e1(1-15)-HD | 0.1 | 0.102 | 41.8 / 65 | 213.3 / 35 |
| L-control | NA | NA | 57.8 / 100 | |
| NA- Not applicable<br>ND- Not done | | | | |

The protein concentration was determined for each of the L-IAVM2e1(C16S, C18S)-HD and the L-IAVM2e1(1-15)-HD preparations. The RP-HPLC analysis of the protein concentration showed that the samples contained 80% to 100% of the original starting material. The loss of the protein could be attributed to the protein adhering to the sides of the tubes, losses due to probe sonication and losses during the sterile filtration. Due to the multimeric form of the IAVM2e1-HD construct from the pET28 and the pEV and the lack of resolution, the protein concentrations were not determined.

The second group contained the two single mutant batches (i.e., C16S and C18S IAVM2e peptides) which were predicted to produce a dimeric form of the protein. The liposomes produced a typical translucent solution with varying size distributions. As seen with the monomeric IAVM2e1-HD constructs in the first group, there were some preparations showing a unimodal distribution and some showing a bimodal distribution (Table 5). The variability in this distribution does not seem to correlate with protein type or protein concentration. Typically, the first peak ranged from 48 nm to 173 nm and the second peak from 149 nm to 254 nm. The protein concentration of the final liposome preparation was determined by RP-HPLC. The data indicate that very little protein was lost during the preparation of these samples (Table 5).

Table 5. Second mouse study of L-IAVM2e1-HD formulations

| | Expected [protein] (mg/ml) | HPLC [protein] (mg/ml) | Size Distribution | |
|---|---|---|---|---|
| | | | Peak #1 nm / % | Peak #2 nm / % |
| L-LDR-IAVM2e1-HD (pET28a) | 0.3 | ND | 67.9 / 100 | |
| L-LDR-IAVM2e1-HD(pEV1) | 0.3 | ND | 173.7 / 100 | |
| L-IAVM2e1(C16S)-HD | 0.6 | 0.57 | 83.8 / 100 | |
| L-IAVM2e1(C16S)-HD | 0.3 | 0.30 | 58.8 / 58 | 191.5 / 42 |
| L-IAVM2e1(C16S)-HD | 0.1 | 0.094 | 66.1 / 79 | 254.2 / 21 |
| L-IAVM2e1(C18S)-HD | 0.6 | 0.54 | 83.8 / 100 | |
| L-IAVM2e1(C18S)-HD | 0.3 | 0.28 | 84.3 / 100 | |

(continued)

| | Expected [protein] (mg/ml) | HPLC [protein] (mg/ml) | Size Distribution | |
|---|---|---|---|---|
| | | | Peak #1 nm / % | Peak #2 nm / % |
| L-IAVM2e1(C18S)-HD | 0.1 | 0.10 | 48.3 / 57 | 149.1 / 43 |
| L-control | NA | NA | 67.0 / 100 | |
| NA- Not applicable<br>ND- Not done | | | | |

Previously, we have shown that a L-IAVM2e1-HD vaccine could induce a strong protective immune response in mice against a lethal influenza virus challenge [Ernst et al., 2006]. This response was linked to a humoral immune response since L-IAVM2e1-HD immunized mouse sera could protect naive mice against a lethal influenza challenge. Due to the total number of mice used to test the new constructs, two separate studies were conducted. In the first study, BALB/c mice were immunized, as describe in the materials and methods section, with the original L-LDR-IAVM2e1-HD (pET28a), L-LDR-IAVM2e1-HD (pEV; this is an identical protein to the pET28a version but in a high expressing plasmid), L-IAVM2e1(1-15)-HD and L-IAVM2e1(C16S, C18S)-HD. In this experiment, the truncated (IAVM2e1-15) and double mutant IAVM2e1(C16S, C18S) proteins were compared to the original IAVM2e1 protein. Mice that were immunized with 15 μg of the original formulation (from pET28a) and the original formulation (from pEV) showed a good immune protective response of 72% survival. When mice were immunized with 5 μg, 15 μg or 30 μg of L-IAVM2e1(1-15)-HD, 86% of the mice survived a lethal influenza challenge ($p < 0.05$ compared to L-control). All of the mice that received L-control and subsequently challenged, died by day 9 (Figure 7A). Additionally, immunized mice showed minimal signs of disease and weight loss compared to the control treated group. In contrast to the mice immunized with L-IAVM2e1(1-15)-HD, of the mice that were immunized with 5 μg, 15 μg or 30 μg of L-IAVM2e1(C16S, C18S)-HD, only the mice that received the 30 μg dose showed a significant difference to that of the controls ($p = 0.006$, 86% survival). While the mice that received 5 μg or 15 μg of L-IAVM2e1(C16S, C18S)-HD showed only 57% and 42% survival, respectively (Figure 8A). The mice were also evaluated for weight loss and signs of disease, which correlated with the percent survival.

In the second study, mice were immunized with single cysteine IAVM2e1 mutants (C16S or C18S) as a liposome preparation. These mutants yielded dimeric forms of the IAVM2e1-HD and were compared in vivo to the monomeric IAVM2e1-HD forms described above. Groups of mice were immunized with 5 μg, 15 μg or 30 μg of L-IAVM2e1(C16S)-HD and subsequently challenged with 10 LD50 of A/PR/8/34. The immune protective response varied between the three doses tested. The mice that were immunized with 5 μg, 15 μg or 30 μg doses showed 86%, 71% and 57% survival, respectively. Only the 5 μg ($p=0.004$) and 15 μg ($p=0.032$) doses showed significant differences compared to the L-control, in which, all the mice treated with L-control died by day 9 post-challenge. Mice were treated with 5 μg, 15 μg or 30 μg of L-IAVM2e1(C18S)-HD showed a much better response than the C16S mutant. All of the mice that received the 15 μg dose survived the lethal challenge and all but one of the seven mice (86%) mice that received the 5 μg and 30 μg dose survived. Each of these doses was significantly better than the mice that received the L-control ($p < 0.005$). Interestingly, both of the single cysteine mutation constructs yielded better protection than the unmutated L-LDRIAVM2e1-HD vaccines. Further evaluation of the immunized mice showed minimal signs of disease and weight loss compared to the control treated group.

Example 3. Conclusions

Since the IAVM2e1 segment contains two cysteines (at the 16th and 18th position; see Table 2) and the peptide can disulfide bond to form dimers to multimers, the purification of the IAVM2e1-HD protein has been difficult. Since the protein is expressed as various forms, the resolution of purification is lower than desired. By expressing and purifying an IAVM2e1-HD with only one cysteine or without any cysteines, the present invention yields a more uniform protein molecule. Our data indicate that the single cysteine IAVM2e1-HD mutations did form dimers and when expressed downstream of thioredoxin, yielded comparable amounts of the protein compared to the original IAVM2e1-HD. In order to test the monomeric forms of the IAVM2e1-HD, two additional constructs were prepared. One construct was made in which both cysteines were mutated and a second construct in which only the first 15 residues of the IAVM2e1 were used. In both cases, the protein was formed as a monomer and was expressed equally well as the original and the single cysteine mutated forms. These data indicated that the cysteines play a definitive role in the protein structure of the dimerization and multimerization of the protein when placed in an oxidizing environment (i.e., buffers used in preparation of liposomes). Purification of the monomeric forms of the protein was straightforward and purification was achieved to high purity. Further, it was clear that there was no difference in the expression of the IAVM2e1-HD proteins when the

proteins were fused to the C-terminus of the thioredoxin.

The preclinical results described herein provide proof that the IAVM2e1 antigen segment is a viable candidate for a universal influenza vaccine. Although there was variability between the different forms of the peptide used, nearly all of the constructs stimulated a significant protective immune response with at least one dose amount compared to the controls. Unexpectedly, it is clear from these data that the cysteines play no role in the protective immune response since both the double cysteine mutant and the truncated IAVM2e1(1-15) protein stimulated a protective immune response.

Example 4. References

Ernst WA, Kim HJ, Tumpey TM, et al. Protection against H1, H5, H6 and H9 influenza A infection with liposomal matrix 2 epitope vaccines. Vaccine 2006;24(24):5158-68.

Fan J, Liang X, Horton MS, et al. Preclinical study of influenza virus A M2 peptide conjugate vaccines in mice, ferrets, and rhesus monkeys. Vaccine 2004;22(23-24):2993-3003.

Liu W, Li H, Chen YH. N-terminus of M2 protein could induce antibodies with inhibitory activity against influenza virus replication. FEMS Immunol.Med Microbiol. 2003;35(2):141-6.

Neirynck S, Deroo T, Saelens X, Vanlandschoot P, Jou WM, Fiers W. A universal influenza A vaccine based on the extracellular domain of the M2 protein. Nat.Med 1999;5(10):1157-63.


Example 5. HSV2

To demonstrate the effectiveness of an HSV2 protein vaccine, a suitable HSV antigen-containing liposomal formulation is made as described in Example 1, with a polypeptide comprising, and preferably consisting of, one or more of the following HSV2 sequences substituting for IAVM2e1:

HTDLHPNNTY, RSSLGSLLY, YMESVFQMY, FLVDAIVRVA, FLIAYQPLL, FLWEDQTLL, ILIEGIFFA, RILGVLVHL, SVYPYDEFV, EYQRLYATF, KYFYCNSLF, LYPDAPPLRL, ALATVTLKY, ALLAKMLFY, LLAYVSVLY, SIVHHHAQY, SSGVVFGTWY, VYMSPFYGY, YMANQILRY, YVAGFLALY, CPRRPAVAF, and VVRGPTVSL (SEQ ID NOS: 60-81)

human HLA A2:01 transgenic (Tg) mice (n=20/group) are used as the animal model to demonstrate the feasibility of the HSV2 vaccine to stimulate protective immune responses in humans. Of the five HLA haplotypes represented in this vaccine, HLA A2:01 is the best model since this HLA haplotype represents the most common HLA haplotype amongst all human ethnic groups. As above, liposomal formulations of the HSV2 sequence(s) fused to an appropriate hydrophobic domain as described herein are prepared with MPL or CpG adjuvant. The vaccinated mice (N=20/group) are evaluated for their response to each of the HSV2 peptides through Luminex multi-bead ELISA and ELISPOT analysis (5 mice/group), and the mice intravaginally challenged to monitor the degree of protection, using the procedures described above.

One skilled in the art readily appreciates that the present invention is well adapted to carry out the objects and obtain the ends and advantages mentioned, as well as those inherent therein. The examples provided herein are representative of preferred embodiments, are exemplary, and are not intended as limitations on the scope of the invention.

All patents and publications mentioned in the specification are indicative of the levels of those of ordinary skill in the art to which the invention pertains.

Other embodiments are set forth within the following claims.

## Claims

**1.** A composition comprising:

an aqueous vehicle; and
liposomes comprising

(i) dimyristoylphosphatidylcholine ("DMPC"),
(ii) dimyristoylphosphatidylglycerol ("DMPG"), or dimyristoyltrimethylammonium propane ("DMTAP"), or both DMPG and DMTAP,
(iii) at least one sterol; and
(iv) an antigenic polypeptide comprising a first polypeptide sequence comprising at least 10 contiguous residues of an influenza A M2 extracellular domain, the sequence of which has been modified to remove one or more cysteine residues naturally occurring in said M2 extracellular domain,
and
a second polypeptide sequence comprising a transmembrane sequence of, or derived from, a naturally occurring heterologous membrane protein, said second polypeptide sequence having a number of residues sufficient to cross a lipid bilayer, at least nine contiguous residues of which are predicted to form an alpha helix having a hydrophobicity score of 0.63 or greater,
wherein the hydrophobicity score is calculated by the methodology of Eisenberg, wherein said first polypeptide sequence is coupled directly or indirectly to the Nor C- terminus of said second polypeptide sequence to provide an aqueous soluble fusion polypeptide, and wherein the antigenic polypeptide sequence of the fusion polypeptide is displayed on the outer surface of the liposomes.

**2.** A composition according to claim 1, wherein the relative percentages of DMPC, DMPG/DMTAP, and sterol are 50% to 98% DMPC : 1% to 25% DMPG/DMTAP : 1% to 25% sterol; or 70% to 98% DMPC : 1% to 15% DMPG/DMTAP : 1% to 15% sterol; or 70% to 85% DMPC : 5% to 15% DMPG/DMTAP : 10% to 15% sterol; or
wherein the relative percentages of DMPC, DMPG/DMTAP, and sterol are 75% DMPC, 10% DMPG/DMTAP, and 15% sterol.

**3.** A composition according to claims 1 or 2, wherein the liposomes comprise DMPG, wherein optionally the liposomes do not comprise DMTAP.

**4.** A composition according to one of claims 1-3, further comprising one or more additional components selected from the group consistin of peptidoglycan, lipopeptide, lipopolysaccharide, monophosphoryl lipid A, lipoteichoic acid, resiquimod, imiquimod, flagellin, oligonucleotides containing unmethylated CpG motifs, $\alpha$-galactosylceramide, muramyl dipeptide, all-trans retinoic acid, double-stranded viral RNA, heat shock proteins, dioctadecyldimethylammonium bromide, cationic surfactants, toll-like receptor agonists, dimyristoyltrimethylammoniumpropane, and nod-like receptor agonists; and/or
wherein the composition comprises a sterol selected from the group consisting of cholesterol, cholesteryl chloroformate, stigmasterol, sitosterol, ergosterol, lanosterol, desmosterol, and campesterol.

**5.** A composition according to one of claims 1-4, wherein the first polypeptide sequence comprises at least 10 contiguous residues from residues 2 - 22 of one of SEQ ID NOS: 1-9; or at least 10 contiguous residues from one of SEQ ID NOS: 10-12; or at least 10 contiguous residues from one of SEQ ID NOS: 13-15, or
wherein the first polypeptide sequence comprises SEQ ID NO: 16.

**6.** A composition according to one of claims 1-5, wherein one or more cysteine residues are removed from the influenza A M2 extracellular domain sequence by mutation of the cysteine residue(s) of interest to another residue or by truncation of the extracellular domain sequence prior to the cysteine residue(s) of interest.

**7.** A composition according to one of claims 1-6, wherein the second polypeptide sequence comprises

(a) a transmembrane sequence encoded by a protein selected from the group consisting of Cytochrome b5, Mucin-4 beta chain, transferrin receptor, TNFRSF13B, Aminopeptidase N, Dipeptidyl peptidase 4, Tumor necrosis factorsyntaxin 3, BclXL, and R9AP,
wherein optionally the second polypeptide sequence comprises a transmembrane sequence encoded by one of SEQ ID NOS: 26-42; or
(b) at least 10 contiguous residues from one of SEQ ID NOS: 17-25,
wherein optionally the second polypeptide sequence comprises a transmembrane sequence encoded by a Cytochrome b5 protein;

**8.** A composition according to one of claims 1 to 7 for use in immunizing an animal, the use comprising administration of the composition via a parenteral route to said animal.

**9.** A method for preparing a liposomal composition according to claim 1, comprising:

recombinantly expressing or synthesizing by chemical methods the antigenic polypeptide,
wherein the antigenic polypeptide is in monomeric form under non-denaturing conditions;
purifying the antigenic polypeptide into an aqueous solution;
adding the aqueous solution comprising the antigenic polypeptide to a lipid mixture comprising (i) DMPC, (ii) DMPG, DMTAP, or both DMPG and DMTAP, and (iii) at least one sterol;
drying the antigenic polypeptide and lipid mixture; and
sonicating the dried antigenic polypeptide and lipid mixture in the presence of the aqueous vehicle to form the liposomes.

**10.** An isolated nucleic acid sequence which encodes a fusion protein, the sequence of which comprises:

a first nucleic acid sequence encoding a thioredoxin;
a second nucleic acid sequence encoding a polypeptide sequence comprising at least 10 contiguous residues of an influenza A M2 extracellular domain, the sequence of which has been modified to remove one or more cysteine residues naturally occurring in said M2 extracellular domain; and
a third nucleic acid sequence encoding a transmembrane sequence of, or derived from, a naturally occurring heterologous membrane protein, said second polypeptide sequence having a number of residues sufficient to cross a lipid bilayer, at least nine contiguous residues of which are predicted to form an alpha helix having a hydrophobicity score of 0.63 or greater, wherein the hydrophobicity score is calculated by the methodology of Eisenberg,
wherein said nucleic acid sequence is configured such that, when the nucleic acid is expressed, thioredoxin is expressed N-terminally to the transmembrane sequence.

**11.** An isolated nucleic acid sequence according to claim 10, further comprising expression control sequences operably linked to the nucleic acid sequence encoding the fusion protein, and/or
one or more additional nucleic acid sequences encoding an affinity marker, and/or
one or more additional nucleic acid sequences encoding a protease cleavage site.

**12.** An isolated nucleic acid sequence according to one of claims 10 or 11,

(a) wherein at least a portion of the isolated nucleic acid sequence is codon optimized for expression in a host cell, wherein optionally at least a portion of the isolated nucleic acid sequence is codon optimized for expression in *E. coli,* and/or
(b) wherein the second nucleic acid sequence encodes an antigenic sequence, a polypeptide targeting ligand, or a fusogenic polypeptide sequence, and/or
(c) wherein the third nucleic acid sequence encodes a transmembrane sequence encoded by a protein selected from the group consisting of Cytochrome b5, Mucin-4 beta chain, transferrin receptor, TNFRSF13B, Aminopeptidase N, Dipeptidyl peptidase 4, Tumor necrosis factorsyntaxin 3, BclXL, and R9AP,
wherein optionally the third nucleic acid sequence comprises a transmembrane sequence encoded by a Cytochrome b5 protein or by one of SEQ ID NOS: 26-42, and/or
(d) wherein the third nucleic acid sequence comprises at least 10 contiguous residues from one of SEQ ID NOS: 17-25.

**13.** A host cell transformed with an isolated nucleic acid sequence according to one of claims 10-12.

**14.** The use of a host cell according to claim 13 for obtaining a polypeptide sequence of interest, comprising:

expression of the fusion protein encoded by the isolated nucleic acid sequence from the host cell, and optionally purifying all or a portion of the expressed fusion protein into an aqueous solution, wherein
optionally the method further comprises
adding the aqueous solution comprising the purified protein to a lipid mixture comprising (i) DMPC, (ii) DMPG, DMTAP, or both DMPG and DMTAP, and (iii) at least one sterol;
drying the purified protein and lipid mixture; and

sonicating the dried purified protein and lipid mixture in the presence of the aqueous vehicle to form liposomes.

## Patentansprüche

1. Zusammensetzung umfassend:

   ein wässriges Lösungsmittel; und
   Liposomen umfassend

   (i) dimyristoylphosphatidylcholin ("DMPC")
   (ii) dimyristoylphosphatidylglycerin ("DMPG"), oder dimyristoyltrimethylammoniumpropan ("DMTAP"), oder beide DMPG und DMTAP,
   (iii) zumindest ein Sterin; und
   (iv) ein Antigenpolypeptid umfassend eine erste Polypeptidsequenz umfassend zumindest 10 zusammenhängende Reste einer Influenza A M2 extrazellulären Domäne, deren Sequenz modifiziert wurde, um ein oder mehrere in der M2 extrazellulären Domäne natürlich vorkommende Cysteinreste zu entfernen,
   und
   eine zweite Polypeptidsequenz umfassend eine Transmembransequenz eines, oder abstammend von einem, natürlich vorkommenden heterologen Membranprotein(s), wobei die zweite Polypeptidsequenz eine Anzahl von Resten aufweist, die ausreichend ist, um eine Lipid-Doppelschicht zu durchqueren, zumindest neun zusammenhängende Reste, von denen vorhergesagt wird, dass sie eine Alpha-Helix formen, die einen Wasserabweisungswert von 0,63 oder größer aufweist,
   wobei der Wasserabweisungswert mit der Methodik von Eisenberg berechnet wird,
   wobei die erste Polypeptidsequenz direkt oder indirekt mit dem N- oder C-Terminus der zweiten Polypeptidsequenz verbunden wird, um ein wässriges lösliches Fusionspolypeptid bereitzustellen, und wobei die Antigenpolypeptidsequenz des Fusionspolypeptids an der äußeren Oberfläche der Liposomen angezeigt wird.

2. Zusammensetzung gemäß Anspruch 1, wobei die relativen Prozentsätze von DMPC, DMPG/DMTAP, und Sterin 50% bis 98% DMPC : 1% bis 25% DMPG/DMTAP : 1% bis 25% Sterin, oder 70% bis 98% DMPC : 1% bis 15% DMPG/DMTAP : 1% bis 15% Sterin, oder 70% bis 85% DMPC : 5% bis 15% DMPG/DMTAP : 10% bis 15% Sterin sind; oder
   wobei die relativen Prozentsätze von DMPC, DMPG/DMTAP, und Sterin 75% DMPC, 10% DMPG/DMTAP, und 15% Sterin sind.

3. Zusammensetzung gemäß den Ansprüchen 1 oder 2, wobei die Liposomen DMPG umfassen, wobei gegebenenfalls die Liposomen nicht DMTAP umfassen.

4. Zusammensetzung gemäß einem der Ansprüche 1-3, weiterhin umfassend ein oder mehrere zusätzlichen Bestandteile ausgewählt aus der Gruppe bestehend aus Peptidoglykan, Lipopeptid, Lipopolysaccharid, Monophosphoryllipid A, Lipoteichonsäure, Resiquimod, Imiquimod, Flagellin, Oligonukleotiden, die unmethylierte CpG-Motive enthalten, α-Galactosylceramid, Muramyldipeptid, all-trans-Retinsäure, doppelsträngiger viraler RNA, Hitzeschockproteinen, Dioctadecyldimethylammoniumbromid, kationischen Tensiden, Toll-like-Rezeptor-Agonisten, Dimyristoyltrimethylammoniumpropan, und Nod-like-Rezeptor-Agonisten;
   und/oder
   wobei die Zusammensetzung ein Sterin umfasst ausgewählt aus der Gruppe bestehend aus Cholesterin, Cholesterylchlorformiat, Stigmasterin, Sitosterin, Ergosterin, Lanosterin, Desmosterin und Campesterin.

5. Zusammensetzung gemäß einem der Ansprüche 1-4, wobei die erste Polypeptidsequenz zumindest 10 zusammenhängende Reste von den Resten 2-22 von einer der SEQ ID NOS: 1-9; oder zumindest 10 zusammenhängende Reste von einer der SEQ ID NOS: 10-12, oder zumindest 10 zusammenhängende Reste von einer der SEQ ID NOS: 13-15 umfasst, oder
   wobei die erste Polypeptidsequenz SEQ ID NO: 16 umfasst.

6. Zusammensetzung gemäß einem der Ansprüche 1-5, wobei ein oder mehrere Cysteinreste von der Influenza A M2 extrazellulären Domänensequenz durch Mutation des/der Cysteinrests/Cyteinreste von Interesse zu einem anderen Rest oder durch Verkürzung der extrazellulären Domänensequenz vor dem/den Cysteinrest/Cysteinresten von

Interesse entfernt werden.

**7.** Zusammensetzung gemäß einem der Ansprüche 1-6, wobei die zweite Polypeptidsequenz

(a) eine Transmembransequenz kodiert von einem Protein ausgewählt aus der Gruppe bestehend aus Cytochrom b5, Mucin-4-beta-Kette, Transferrinrezeptor, TNFRSF13B, Aminopeptidase N, Dipeptidylpeptidase 4, Tumornekrosefaktorsyntaxin 3, BclXL, und R9AP umfasst,
wobei gegebenenfalls die zweite Polypeptidsequenz eine Transmembransequenz kodiert von einer der SEQ ID NOS: 26-42 umfasst; oder
(b) zumindest 10 zusammenhängende Reste von einer der SEQ ID NOS: 17-25 umfasst,
wobei gegebenenfalls die zweite Polypeptidsequenz eine Transmembransequenz kodiert von einem Cytochrom b5 Protein umfasst.

**8.** Zusammensetzung gemäß einem der Ansprüche 1 bis 7 zur Verwendung bei dem Immunisieren eines Tiers, die Verwendung umfassend Verabreichung der Zusammensetzung über eine parenterale Applikation an das Tier.

**9.** Verfahren zum Herstellen einer liposomalen Zusammensetzung nach Anspruch 1, umfassend:

rekombinantes Exprimieren oder Synthetisieren des Antigenpolypeptids durch chemische Methoden, wobei das Antigenpolypeptid unter nicht-denaturierenden Bedingungen in monomerer Form vorliegt;
Reinigen des Antigenpolypeptids in eine wässrige Lösung;
Zugeben der wässrigen Lösung umfassend das Antigenpolypeptid zu einer Lipidmischung umfassend (i) DMPC, (ii) DMPG, DMTAP, oder beide DMPG und DMTAP, und (iii) zumindest ein Sterin;
Trocknen des Antigenpolypeptids und der Lipidmischung; und
Beschallen des getrockneten Antigenpolypeptids und der Lipidmischung in der Anwesenheit des wässrigen Lösungsmittels, um Liposomen zu formen.

**10.** Isolierte Nukleinsäuresequenz, die ein Fusionsprotein kodiert, dessen Sequenz umfasst:

eine erste Nukleinsäuresequenz, die Thioredoxin kodiert;
eine zweite Nukleinsäuresequenz, die eine Polypeptidsequenz kodiert umfassend zumindest 10 zusammenhängende Reste einer Influenza A M2 extrazellulären Domäne, deren Sequenz modifiziert wurde, um ein oder mehrere in der M2 extrazellulären Domäne natürlich vorkommende Cysteinreste zu entfernen; und
eine dritte Nukleinsäuresequenz, die eine Transmembransequenz eines, oder abstammend von einem, natürlich vorkommenden heterologen Membranprotein(s) kodiert, wobei die zweite Polypeptidsequenz eine Anzahl von Resten aufweist, die ausreichend ist, um einen Lipid-Doppelschicht zu durchqueren, zumindest neun zusammenhängende Reste, von denen vorhergesagt wird, dass sie eine Alpha-Helix formen, die einen Wasserabweisungswert von 0,63 oder größer aufweist, wobei der Wasserabweisungswert mit der Methodik von Eisenberg berechnet wird,
wobei die Nukleinsäuresequenz so konfiguriert ist, dass wenn die Nukleinsäure exprimiert wird, Thioredoxin N-terminal zu der Transmembransequenz exprimiert wird.

**11.** Isolierte Nukleinsäuresequenz gemäß Anspruch 10, weiterhin umfassend Expressions-Kontrollsequenzen, die funktionsfähig mit der Nukleinsäuresequenz, die das Fusionsprotein kodiert, verknüpft sind,
und/oder
ein oder mehrere zusätzliche Nukleinsäuresequenzen, die einen Affinitätsmarker kodieren, und/oder
ein oder mehrere zusätzliche Nukleinsäuresequenzen, die eine Protease-Spaltstelle kodieren.

**12.** Isolierte Nukleinsäuresequenz gemäß einem der Ansprüche 10 oder 11,

(a) wobei zumindest ein Teil der isolierten Nukleinsäuresequenz für Expression in einer Wirtszelle codonoptimiert ist,
wobei gegebenenfalls zumindest ein Teil der isolierten Nukleinsäuresequenz für Expression in *E. coli* codonoptimiert ist, und/oder
(b) wobei die zweite Nukleinsäuresequenz eine Antigensequenz, ein Polypeptid, das auf einen Liganden abzielt, oder eine fusogene Polypeptidsequenz kodiert, und/oder
(c) wobei die dritte Nukleinsäuresequenz eine Transmembransequenz kodiert von einem Protein ausgewählt aus der Gruppe bestehend aus Cytochrom b5, Mucin-4-beta-Kette, Transferrinrezeptor, TNFRSF13B, Aminopeptidase N, Dipeptidylpeptidase 4, Tumornekrosefaktorsyntaxin 3, BclXL und R9AP kodiert,
wobei gegebenenfalls die dritte Nukleinsäuresequenz eine Transmembransequenz kodiert von einem Cytochrom b5 Protein oder kodiert von einer der SEQ ID NOS: 26-42 umfasst, und/oder
(d) wobei die dritte Nukleinsäuresequenz zumindest 10 zusammenhängende Reste von einer der SEQ ID NOS: 17-25 umfasst.

**13.** Wirtszelle transformiert mit einer isolierten Nukleinsäuresequenz gemäß einem der Ansprüche 10-12.

**14.** Verwendung einer Wirtszelle gemäß Anspruch 13 zum Erhalten einer Polypeptidsequenz von Interesse, umfassend:

Expression des Fusionsproteins kodiert von der isolierten Nukleinsäuresequenz aus der Wirtszelle, und gegebenenfalls
Reinigen des gesamten oder eines Teils des exprimierten Fusionsproteins in eine wässrige Lösung, wobei gegebenenfalls das Verfahren weiterhin umfasst Zugeben der wässrigen Lösung umfassend das aufgereinigte Protein zu einer Lipidmischung umfassend (i) DMPC, (ii) DMPG, DMTAP, oder beide DMPG und DMTAP, und (iii) zumindest Sterin;
Trocknen des aufgereinigten Proteins und der Lipidmischung; und
Beschallen des getrockneten aufgereinigten Proteins und der Lipidmischung in der Anwesenheit des wässrigen Lösungsmittels, um Liposomen zu formen.

## Revendications

**1.** Composition comportant:

un véhicule aqueux; et
des liposomes comportant

(i) de la dimyristoyl phosphatidylcholine ("DMPC"),
(ii) du dimyristoyl phosphatidylglycérol ("DMPG") ou du dimyristoyl triméthylammonium propane ("DMTAP") ou à la fois le DMPG et le DMTAP,
(iii) au moins un stérol et
(iv) un polypeptide antigénique comportant une première séquence polypeptidique comportant au moins 10 résidus contigus d'un domaine extracellulaire d'influenza A M2, dont la séquence a été modifié pour éliminer un ou plusieurs résidus de cystéine se produisant naturellement dans ledit domaine extracellulaire M2,
et
une seconde séquence polypeptidique comportant une séquence transmembranaire d'une ou provenant d'une protéine hétérologue membranaire se produisant naturellement, ladite seconde séquence polypeptidique ayant un nombre de résidus suffisant pour traverser une bicouche lipidique, dont au moins neuf résidus contigus sont prévus comme formant une hélice alpha ayant un score d'hydrophobie de 0,63 ou plus, le score d'hydrophobie étant calculé par la méthodologie d'Eisenberg, la première séquence polypeptidique étant couplée directement ou indirectement à la terminaison N ou C de ladite seconde séquence polypeptidique pour fournir une polypeptide de fusion aqueuse soluble, et la séquence polypeptidique antigénique de la polypeptide de fusion étant présentée sur la surface externe des liposomes.

**2.** Composition selon la revendication 1, les pourcentages relatifs de DMPC, DMPG/DMTAP et de stérol étant de 50% à 98% de DMPC : 1% à 25% de DMPG/DMTAP : 1% à 25% de stérol; ou 70% à 98% de DMPC : 1% à 15% de DMPG/DMTAP : 1% à 15% de stérol; ou 70% à 85% de DMPC : 5% à 15% de DMPG/DMTAP : 10 à 15% de stérol; ou les pourcentages relatifs de DMPC, DMPG/DMTAP et de stérol étant de 75% de DMPC, 10% de DMPG/DMTAP et 15% de stérol.

**3.** Composition selon la revendication 1 ou 2, les liposomes comportant le DMPG, les liposomes facultativement ne comportant pas le DMTAP.

**4.** Composition selon l'une quelconque des revendications 1 à 3,
comportant en outre un ou plusieurs composants supplémentaires choisi parmi le groupe constitué de peptidoglycane, de lipopeptide, de lipopolysaccharide, de monophosphoryl lipide A, de l'acide lipotéichoique, de résiquimod,

d'imiquimod, de flagelline, d'oligonucléotides contenant des motifs CpG non méthylés, de l'a-galactosylcéramide, de dipeptide de muramyle, de l'acide tout-trans rétinoique, de l'ARN viral double brin, de protéines de choc thermique, de bromure de dioctadécyl diméthylammonium, de surfactants cationiques, d'agonistes de récepteur de type Toll, de dimyristoyl triméthylammoniumpropane, et d'agonistes de récepteur de type NOD; et/ou
la composition comportant un stérol choisi parmi le groupe constitué de cholésterol, de cholésteryle chloroformate, de stigmastérol, de sitostérol, d'ergostérol, de lanostérol, de desmostérol, et de campestérol.

**5.** Composition selon l'une quelconque des revendications 1 à 4, la première séquence polypeptidique comportant au moins 10 résidus contigus de résidus de 2 à 22 de l'une des SEQ-ID N°: 1 à 9, ou au moins 10 résidus contigus de l'une des SEQ-ID N°: 10 à 12, ou au moins 10 résidus contigus de l'une des SEQ-ID N°: 13 à 15, ou la première séquence polypeptidique comportant SEQ-ID N°: 16.

**6.** Composition selon l'une quelconque des revendications 1 à 5, un ou plusieurs résidus de cystéine étant éliminés de la séquence du domaine extracellulaire d'influenza A M2 par mutation du (des) résidu(s) de cystéine d'intérêt à un autre résidu ou par troncature de la séquence du domaine extracéllulaire avant le (les) résidu(s) de cystéine d'intérêt.

**7.** Composition selon l'une quelconque des revendications 1 à 6, la seconde séquence polypeptidique comportant

(a) une séquence transmembranaire codée par une protéine choisie parmi le groupe constitué de cytochrome b5, de mucine-4 chaîne bêta, de récepteur de la transferrine, de TNFRSF13B, d'aminopeptidase N, de dipeptidyl peptidase 4, de nécrose tumorale de facteur syntaxine 3, de BclXL, et de R9AP;
facultativement la seconde séquence polypeptidique comportant une séquence transmembranaire codée par une des SEQ-ID N°: 26 à 42; ou
(b) au moins 10 résidus contigus de l'une des SEQ-ID N°: 17 à 25, la seconde séquence polypeptidique facultativement comportant une séquence transmembranaire codée par une protéine b5 de cytochrome.

**8.** Composition selon l'une quelconque des revendications 1 à 7 servant à immuniser un animal, l'utilisation comportant l'administration de la composition par une voie parentérale audit animal.

**9.** Procédé de préparation d'une composition liposomale selon la revendication 1 comportant:

exprimer de manière recombinante ou synthétiser par des méthodes chimiques le polypeptide antigénique,
le polypeptide antigénique étant sous forme monomère dans des conditions non dénaturantes;
purifier le polypeptide antigénique dans une solution aqueuse;
ajouter la solution aqueuse comportant le polypeptide antigénique à un mélange lipidique comportant (i) la DMPC, (ii) le DMPG, le DMTAP ou à la fois le DMPG et le DMTAP, et (iii) au moins un stérol;
sécher le polypeptide antigénique et le mélange lipidique; et
soniquer le polypeptide antigénique séché et le mélange lipidique en présence du véhicule aqueux pour former les liposomes.

**10.** Séquence d'acide nucléique isolée qui code pour une protéine de fusion, dont la séquence comporte:

une première séquence d'acide nucléique codant pour un thiorédoxine;
une seconde séquence d'acide nucléique codant pour une séquence polypeptidique comportant au moins 10 résidus contigus d'un domaine extracellulaire d'influenza A M2, dont la séquence a été modifié pour éliminer un ou plusieurs résidus de cystéine se produisant naturellement dans ledit domaine extracellulaire M2, et
une troisième séquence d'acide nucléique codant pour une séquence transmembranaire d'une ou provenant d'une protéine hétérologue membranaire se produisant naturellement, ladite seconde séquence polypeptidique ayant un nombre de résidus suffisant pour traverser une bicouche lipidique, dont au moins neuf résidus contigus sont prévus comme formant une hélice alpha ayant un score d'hydrophobie de 0,63 ou plus, le score d'hydrophobie étant calculé par la méthodologie d'Eisenberg,
ladite séquence d'acide nucléique étant configurée de telle sorte que lorsque l'acide nucléique est exprimé, le thiorédoxin est exprimé à terminaison N à la séquence transmembranaire.

**11.** Séquence d'acide nucléique isolée selon la revendication 10,
comportant en outre des séquences de contrôle d'expression fonctionnellement liées à la séquence d'acide nucléique codant pour la protéine de fusion

et/ou
une ou plusieurs séquences d'acide nucléique supplémentaires codant pour un marqueur d'affinité, et/ou
une ou plusieurs séquences d'acide nucléique supplémentaires codant pour un site de clivage de protéase.

**12.** Séquence d'acide nucléique isolée selon l'une quelconque des revendications 10 ou 11,

(a) au moins une partie de la séquence d'acide nucléique isolée étant à codon optimisé pour l'expression dans une cellule hôte, facultativement au moins une partie de la séquence d'acide nucléique isolée étant à codon optimisé pour l'expression dans *E. coli,* et/ou
b) la seconde séquence d'acide nucléique codant pour une séquence antigénique, un ligand polypeptique de ciblage ou une séquence polypeptidique fusogénique, et/ou
c) la troisième séquence d'acide nucléique codant pour une séquence transmembranaire codée par une protéine choisie parmi le groupe constitué de cytochrome b5, de mucine-4 chaîne bêta, de récepteur de la transferrine, de TNFRSF13B, d'aminopeptidase N, de dipeptidyl peptidase 4, de nécrose tumorale de facteur syntaxine 3, de BclXL, et de R9AP;
facultativement la troisième séquence d'acide nucléique comportant une séquence transmembranaire codée par une protéine de cytochrome b5 ou par une des SEQ-ID N°: 26 à 42, et/ou
d) la troisième séquence d'acide nucléique comportant au moins 10 résidus contigus de l'une des SEQ-ID N°: 17 à 25.

**13.** Cellule hôte transformée avec une séquence d'acide nucléique isolée selon l'une quelconque des revendications 10 à 12.

**14.** Utilisation d'une cellule hôte selon la revendication 13 pour obtenir une séquence polypeptidique d'intérêt comportant:

l'expression de la protéine de fusion codée par la séquence d'acide nucléique isolée de la cellule hôte, et facultativement
purifier toute ou une partie de la protéine de fusion exprimée dans une solution aqueuse, le procédé comportant facultativement en outre ajouter la solution aqueuse comportant la protéine purifiée à un mélange lipidique comportant (i) de la DMPC, (ii) du DMPG, du DMTAP ou à la fois le DMPG et le DMTAP, et (iii) au moins un stérol;
sécher la protéine purifiée et le mélange lipidique; et
soniquer la protéine purifiée séchée et le mélange lipidique en présence du véhicule aqueux pour former les liposomes.

Fig. 1

**Sequence:Homo sapiens**

```
        10         20         30         40
NKPPETLITT IDSSSSWWTN WVIPAISAVA VALMYRLYMA ED
```

SEQUENCE LENGTH: 42

Using the scale **Hphob. / Eisenberg et al.**, the individual values for the 20 amino acids are:

```
Ala:  0.620  Arg: -2.530  Asn: -0.780  Asp: -0.900  Cys:  0.290
Gln: -0.850
Glu: -0.740  Gly:  0.480  His: -0.400  Ile:  1.380  Leu:  1.060
Lys: -1.500
Met:  0.640  Phe:  1.190  Pro:  0.120  Ser: -0.180  Thr: -0.050
Trp:  0.810
Tyr:  0.260  Val:  1.080  : -0.840  : -0.795  : -0.000
```

Weights for window positions 1,..,9, using **linear weight variation model:**

```
   1     2     3     4     5     6     7     8     9
 1.00  1.00  1.00  1.00  1.00  1.00  1.00  1.00  1.00
 edge                   center                   edge
```

MIN: -0.092
MAX: 0.769

**Fig. 1 cont.**

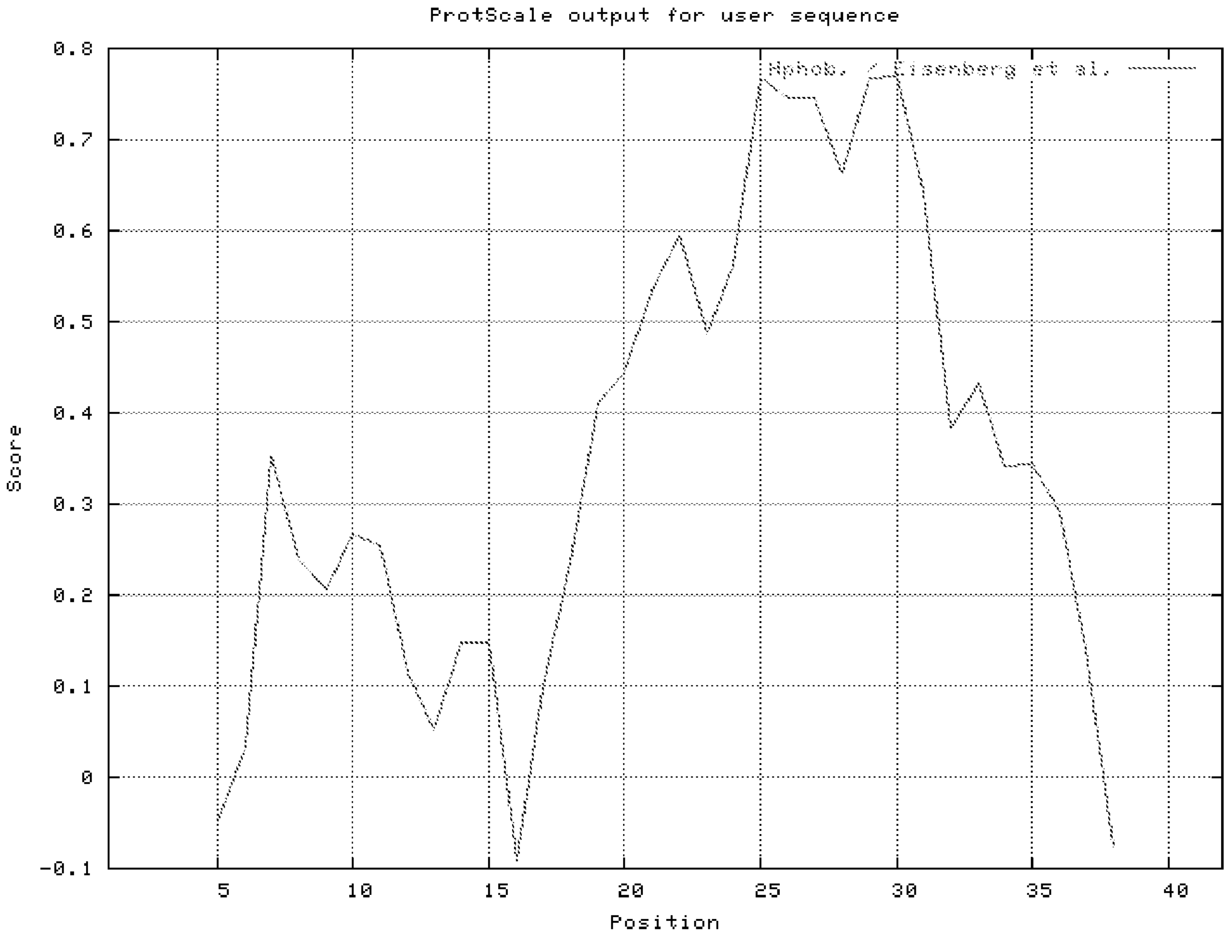
ProtScale output for user sequence
Hphob. / Eisenberg et al.
Score
Position
0.8
0.7
0.6
0.5
0.4
0.3
0.2
0.1
0
-0.1
5
10
15
20
25
30
35
40

**Fig. 2**

**Sequence:**Ratus

```
       10         20         30         40
AKPSETLITT VESNSSWWTN WVIPAISALV VALMYRLYMA ED
```

SEQUENCE LENGTH: 42

Using the scale **Hphob. / Eisenberg et al.**, the individual values for the 20 amino acids are:

```
Ala:  0.620  Arg: -2.530  Asn: -0.780  Asp: -0.900  Cys:  0.290
Gln: -0.850
Glu: -0.740  Gly:  0.480  His: -0.400  Ile:  1.380  Leu:  1.060
Lys: -1.500
Met:  0.640  Phe:  1.190  Pro:  0.120  Ser: -0.180  Thr: -0.050
Trp:  0.810
Tyr:  0.260  Val:  1.080  : -0.840  : -0.795  : -0.000
```

Weights for window positions 1,..,9, using **linear weight variation model:**

| 1 | 2 | 3 | 4 | 5 | 6 | 7 | 8 | 9 |
|---|---|---|---|---|---|---|---|---|
| 1.00 | 1.00 | 1.00 | 1.00 | 1.00 | 1.00 | 1.00 | 1.00 | 1.00 |
| edge | | | | center | | | | edge |

MIN: -0.141
MAX: 0.818

**Fig. 2 cont.**

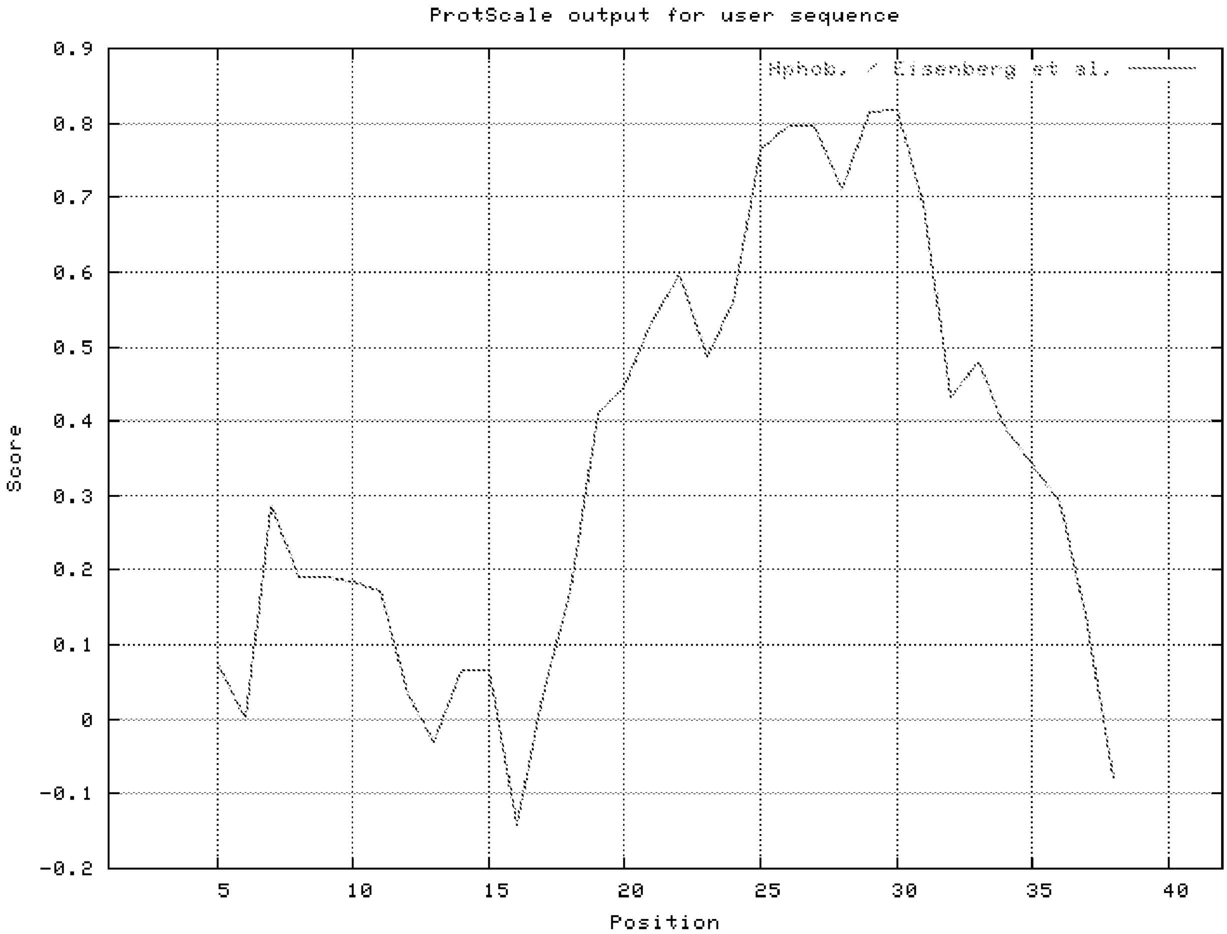
ProtScale output for user sequence
Hphob. / Eisenberg et al.
Score
Position
0.9
0.8
0.7
0.6
0.5
0.4
0.3
0.2
0.1
0
-0.1
-0.2
5
10
15
20
25
30
35
40

**Fig. 3**

**Sequence:Mus musculus**

```
       10         20         30         40
AKPSDTLITT VESNSSWWTN WVIPAISALA VALMYRLYMA ED
```

SEQUENCE LENGTH: 42

---

Using the scale **Hphob. / Eisenberg et al.**, the individual values for the 20 amino acids are:

```
Ala:  0.620  Arg: -2.530  Asn: -0.780  Asp: -0.900  Cys:  0.290
Gln: -0.850
Glu: -0.740  Gly:  0.480  His: -0.400  Ile:  1.380  Leu:  1.060
Lys: -1.500
Met:  0.640  Phe:  1.190  Pro:  0.120  Ser: -0.180  Thr: -0.050
Trp:  0.810
Tyr:  0.260  Val:  1.080  : -0.840  : -0.795  : -0.000
```

---

Weights for window positions 1,..,9, using **linear weight variation model:**

| 1 | 2 | 3 | 4 | 5 | 6 | 7 | 8 | 9 |
|---|---|---|---|---|---|---|---|---|
| 1.00 | 1.00 | 1.00 | 1.00 | 1.00 | 1.00 | 1.00 | 1.00 | 1.00 |
| edge | | | | center | | | | edge |

---

MIN: -0.141
MAX: 0.767

**Fig. 3 cont.**

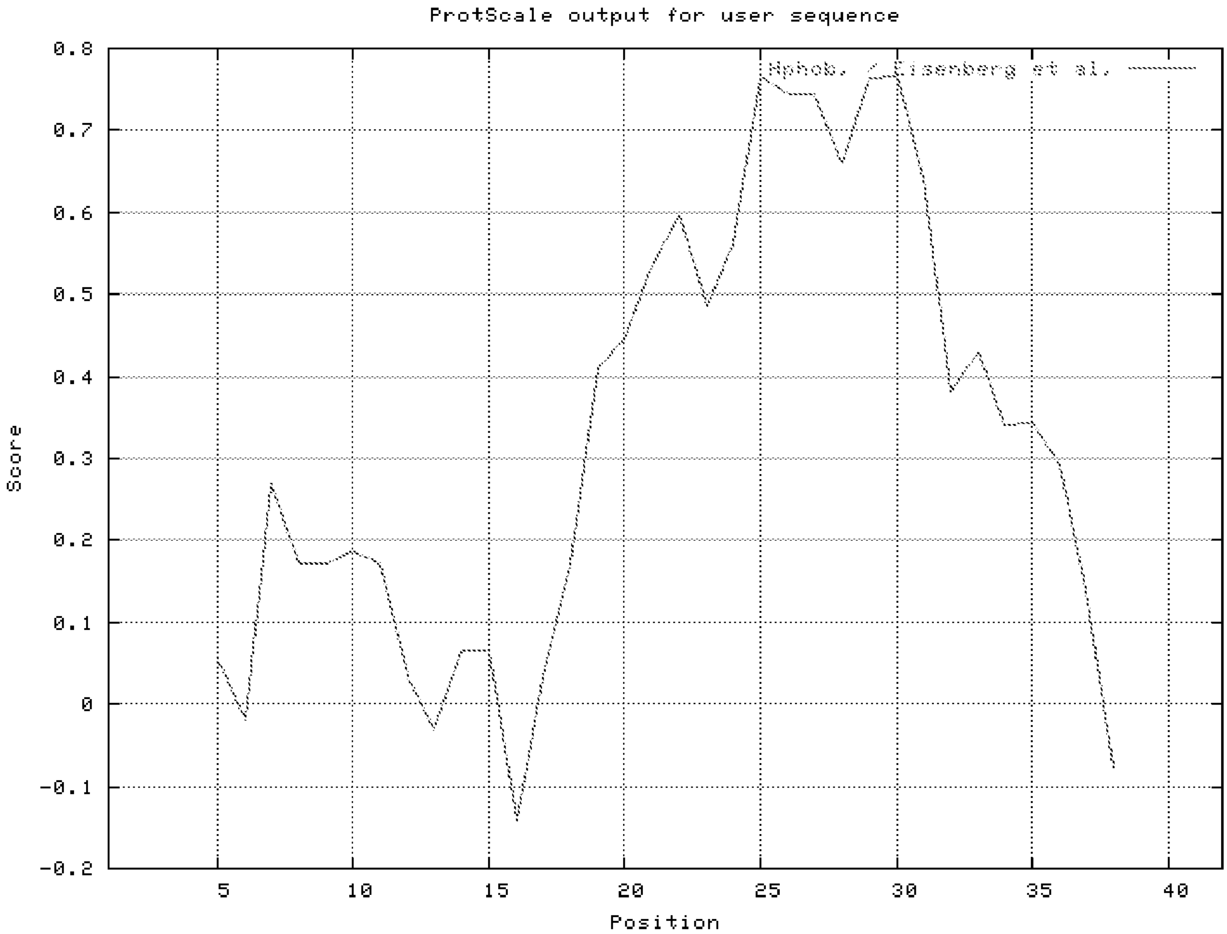
ProtScale output for user sequence
Hphob. / Eisenberg et al.
Score
Position
0.8
0.7
0.6
0.5
0.4
0.3
0.2
0.1
0
-0.1
-0.2
5
10
15
20
25
30
35
40

**Fig. 4**

**Sequence:Oryctolagus cuniculus**

```
        10         20         30         40
SKPMETLITT VDSNSSWWTN WVIPAISALI VALMYRLYMA DD
```

SEQUENCE LENGTH: 42

Using the scale **Hphob. / Eisenberg et al.**, the individual values for the 20 amino acids are:

```
Ala:  0.620  Arg: -2.530  Asn: -0.780  Asp: -0.900  Cys:  0.290
Gln: -0.850
Glu: -0.740  Gly:  0.480  His: -0.400  Ile:  1.380  Leu:  1.060
Lys: -1.500
Met:  0.640  Phe:  1.190  Pro:  0.120  Ser: -0.180  Thr: -0.050
Trp:  0.810
Tyr:  0.260  Val:  1.080  : -0.840  : -0.795  : -0.000
```

Weights for window positions 1,..,9, using **linear weight variation model:**

```
   1     2     3     4     5     6     7     8     9
 1.00  1.00  1.00  1.00  1.00  1.00  1.00  1.00  1.00
 edge                   center                   edge
```

MIN: -0.159
MAX: 0.851

**Fig. 4 cont.**

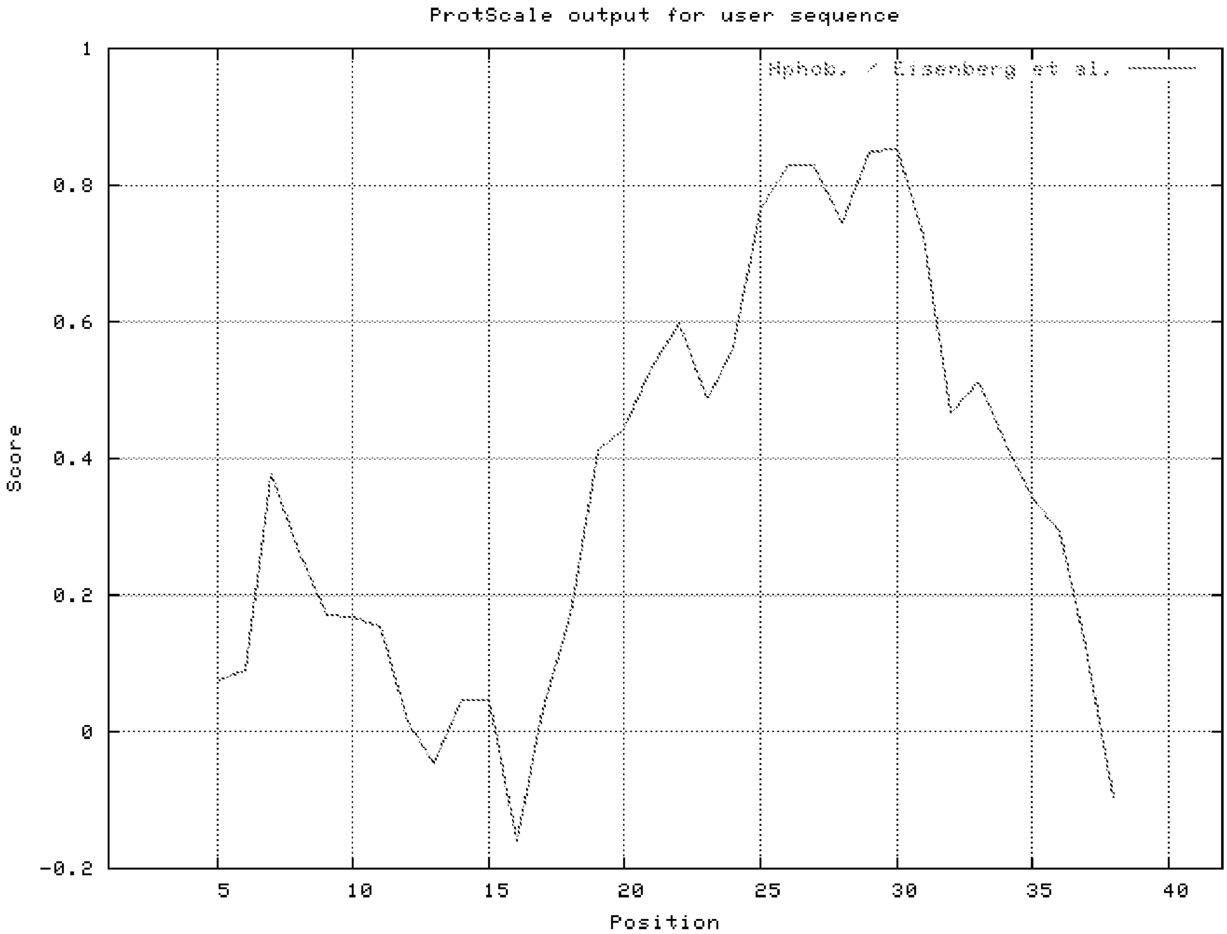
ProtScale output for user sequence
Hphob. / Eisenberg et al.
Score
Position
1
0.8
0.6
0.4
0.2
0
-0.2
5
10
15
20
25
30
35
40

**Fig. 5**

**Sequence:Gallus gallus**

```
        10         20         30         40
QKPAETLITT VQSNSSSWSN WVIPAIAAII VALMYRSYMS E
```

SEQUENCE LENGTH: 41

Using the scale **Hphob. / Eisenberg et al.**, the individual values for the 20 amino acids are:

```
Ala:  0.620  Arg: -2.530  Asn: -0.780  Asp: -0.900  Cys:  0.290
Gln: -0.850
Glu: -0.740  Gly:  0.480  His: -0.400  Ile:  1.380  Leu:  1.060
Lys: -1.500
Met:  0.640  Phe:  1.190  Pro:  0.120  Ser: -0.180  Thr: -0.050
Trp:  0.810
Tyr:  0.260  Val:  1.080  : -0.840  : -0.795  : -0.000
```

Weights for window positions 1,..,9, using **linear weight variation model:**

```
   1     2     3     4     5     6     7     8     9
 1.00  1.00  1.00  1.00  1.00  1.00  1.00  1.00  1.00
 edge                    center                  edge
```

MIN: -0.278
MAX: 0.976

**Fig. 5 cont.**

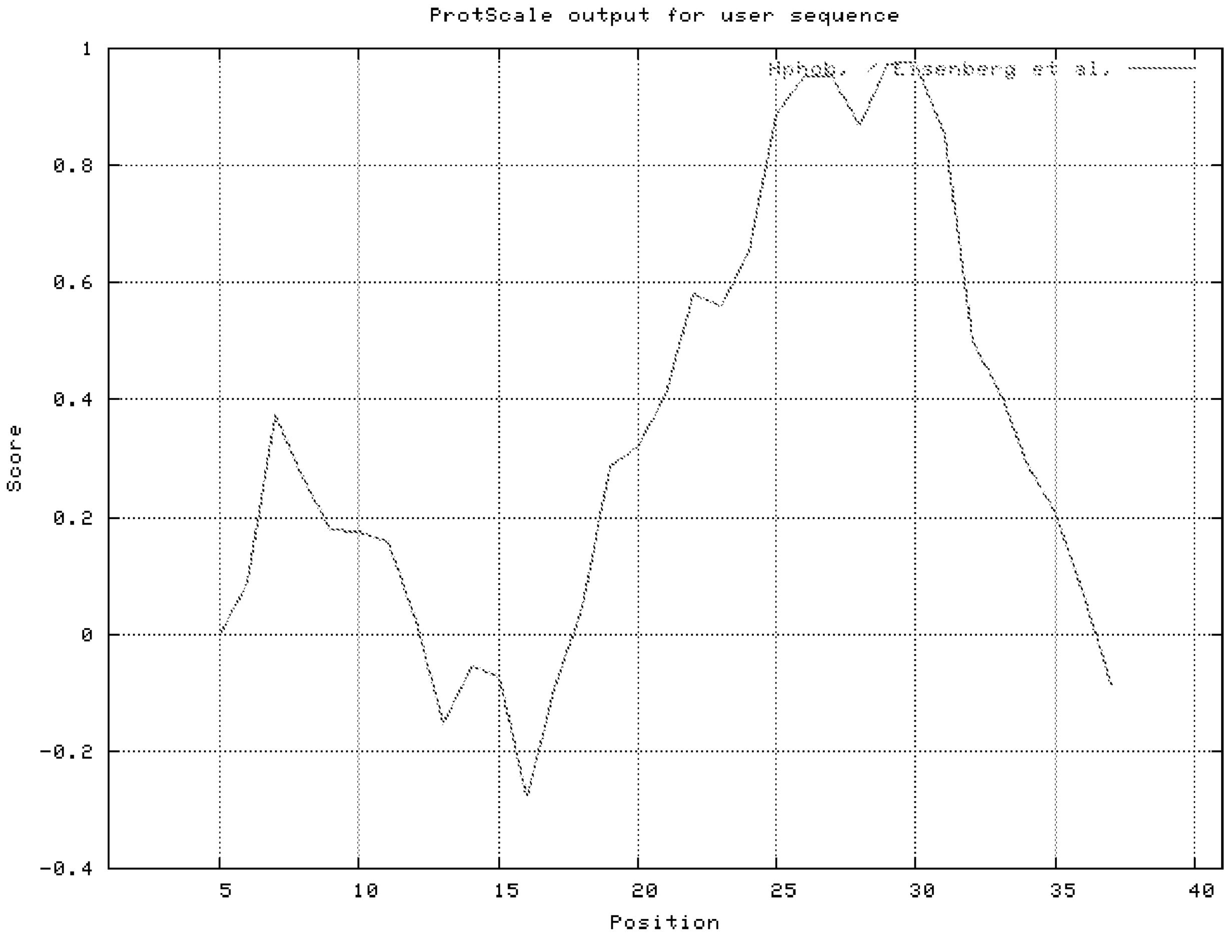
ProtScale output for user sequence
Hphob. / Eisenberg et al.
Score
Position
1
0.8
0.6
0.4
0.2
0
-0.2
-0.4
5
10
15
20
25
30
35
40

**Fig. 6**

**Sequence:**Sus scrufus

```
        10         20         30         40
AKPSETLITT VESNSSWWTN WVIPAISALV VSLMYHFYTS EN
```

SEQUENCE LENGTH: 42

---

Using the scale **Hphob. / Eisenberg et al.**, the individual values for the 20 amino acids are:

```
Ala:  0.620  Arg: -2.530  Asn: -0.780  Asp: -0.900  Cys:  0.290
Gln: -0.850
Glu: -0.740  Gly:  0.480  His: -0.400  Ile:  1.380  Leu:  1.060
Lys: -1.500
Met:  0.640  Phe:  1.190  Pro:  0.120  Ser: -0.180  Thr: -0.050
Trp:  0.810
Tyr:  0.260  Val:  1.080  : -0.840  : -0.795  : -0.000
```

---

Weights for window positions 1,..,9, using **linear weight variation model:**

```
   1     2     3     4     5     6     7     8     9
 1.00  1.00  1.00  1.00  1.00  1.00  1.00  1.00  1.00
 edge                   center                   edge
```

---

MIN: -0.141
MAX: 0.796

**Fig. 6 cont.**

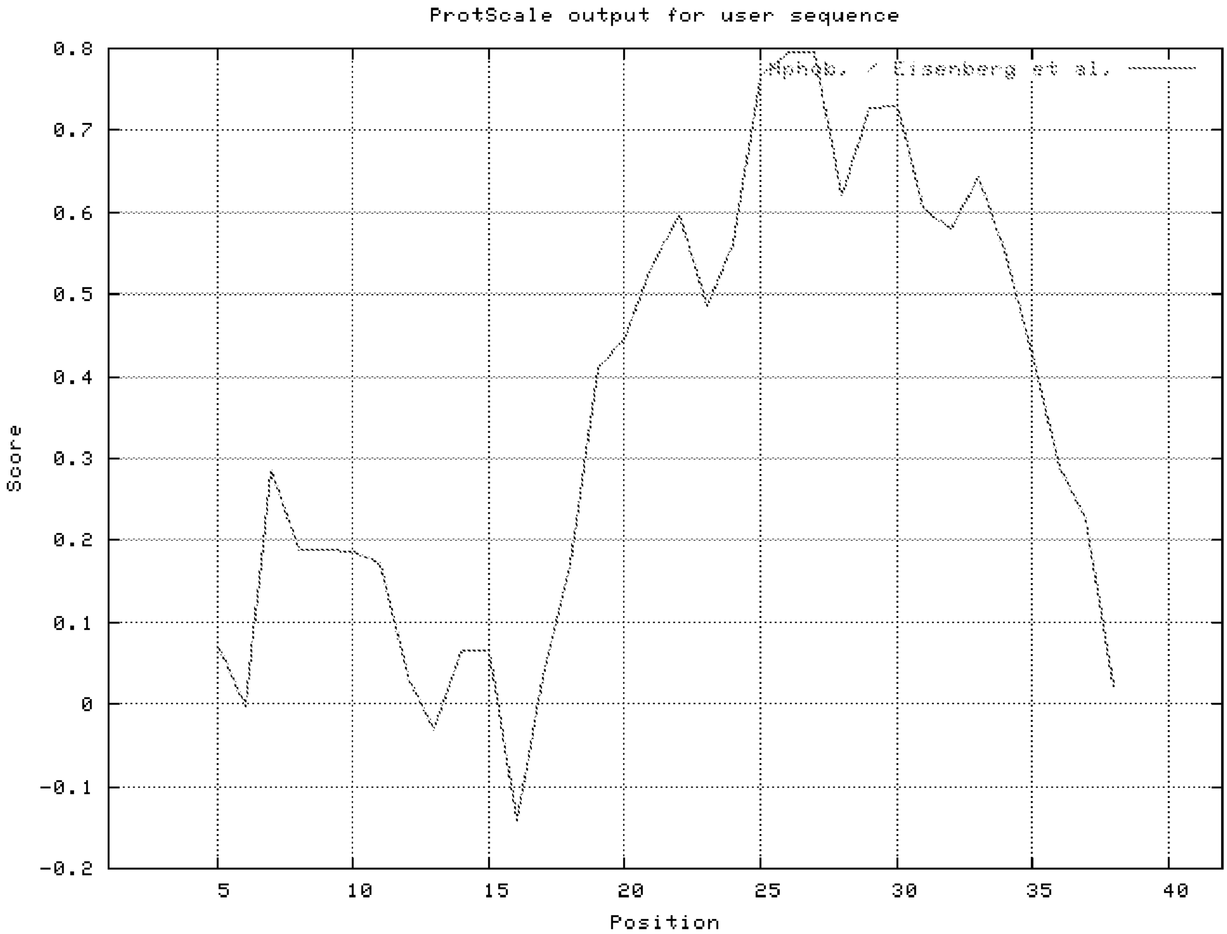
ProtScale output for user sequence
Hphob. / Eisenberg et al.
Score
0.8
0.7
0.6
0.5
0.4
0.3
0.2
0.1
0
-0.1
-0.2
5
10
15
20
25
30
35
40
Position

**Fig. 7**

**Sequence:Xenopus**

```
        10         20         30         40
QKPSETFITT TDSDSSWWSN WIIPGISAMI VALMYRFYMV SE

SEQUENCE LENGTH: 42
```

---

```
Using the scale Hphob. / Eisenberg et al., the individual values
for the 20 amino acids are:
Ala:  0.620  Arg: -2.530  Asn: -0.780  Asp: -0.900  Cys:  0.290
Gln: -0.850
Glu: -0.740  Gly:  0.480  His: -0.400  Ile:  1.380  Leu:  1.060
Lys: -1.500
Met:  0.640  Phe:  1.190  Pro:  0.120  Ser: -0.180  Thr: -0.050
Trp:  0.810
Tyr:  0.260  Val:  1.080  : -0.840  : -0.795  : -0.000
```

---

```
Weights for window positions 1,..,9, using linear weight
variation model:
   1     2     3     4     5     6     7     8     9
 1.00  1.00  1.00  1.00  1.00  1.00  1.00  1.00  1.00
 edge                   center                  edge
```

---

```
MIN: -0.187
MAX: 0.804
```

**Fig. 7 cont.**

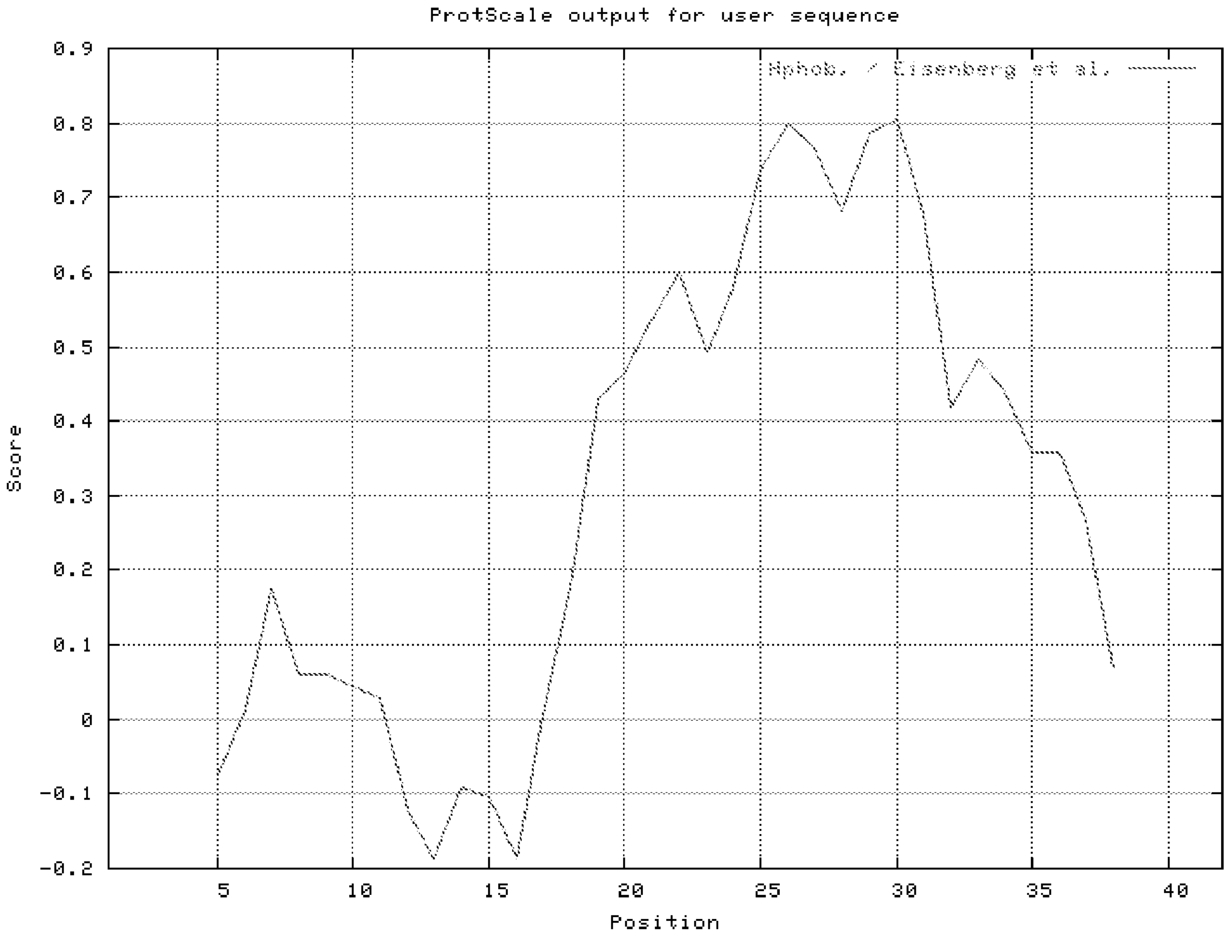
ProtScale output for user sequence
Hphob. / Eisenberg et al.
Score
0.9
0.8
0.7
0.6
0.5
0.4
0.3
0.2
0.1
0
-0.1
-0.2
5
10
15
20
25
30
35
40
Position

**Fig. 8**

**Sequence:Bombyx mori**

```
        10         20         30         40
QYSWEDTAKT SETEASFVNS WKFPVLLGLA LTLLYSYIFG
```

SEQUENCE LENGTH: 40

Using the scale **Hphob. / Eisenberg et al.**, the individual values for the 20 amino acids are:

```
Ala:  0.620  Arg: -2.530  Asn: -0.780  Asp: -0.900  Cys:  0.290
Gln: -0.850
Glu: -0.740  Gly:  0.480  His: -0.400  Ile:  1.380  Leu:  1.060
Lys: -1.500
Met:  0.640  Phe:  1.190  Pro:  0.120  Ser: -0.180  Thr: -0.050
Trp:  0.810
Tyr:  0.260  Val:  1.080  : -0.840  : -0.795  : -0.000
```

Weights for window positions 1,..,9, using **linear weight variation model:**

```
   1     2     3     4     5     6     7     8     9
 1.00  1.00  1.00  1.00  1.00  1.00  1.00  1.00  1.00
 edge                   center                  edge
```

MIN: -0.399
MAX: 0.859

**Fig. 8 cont.**

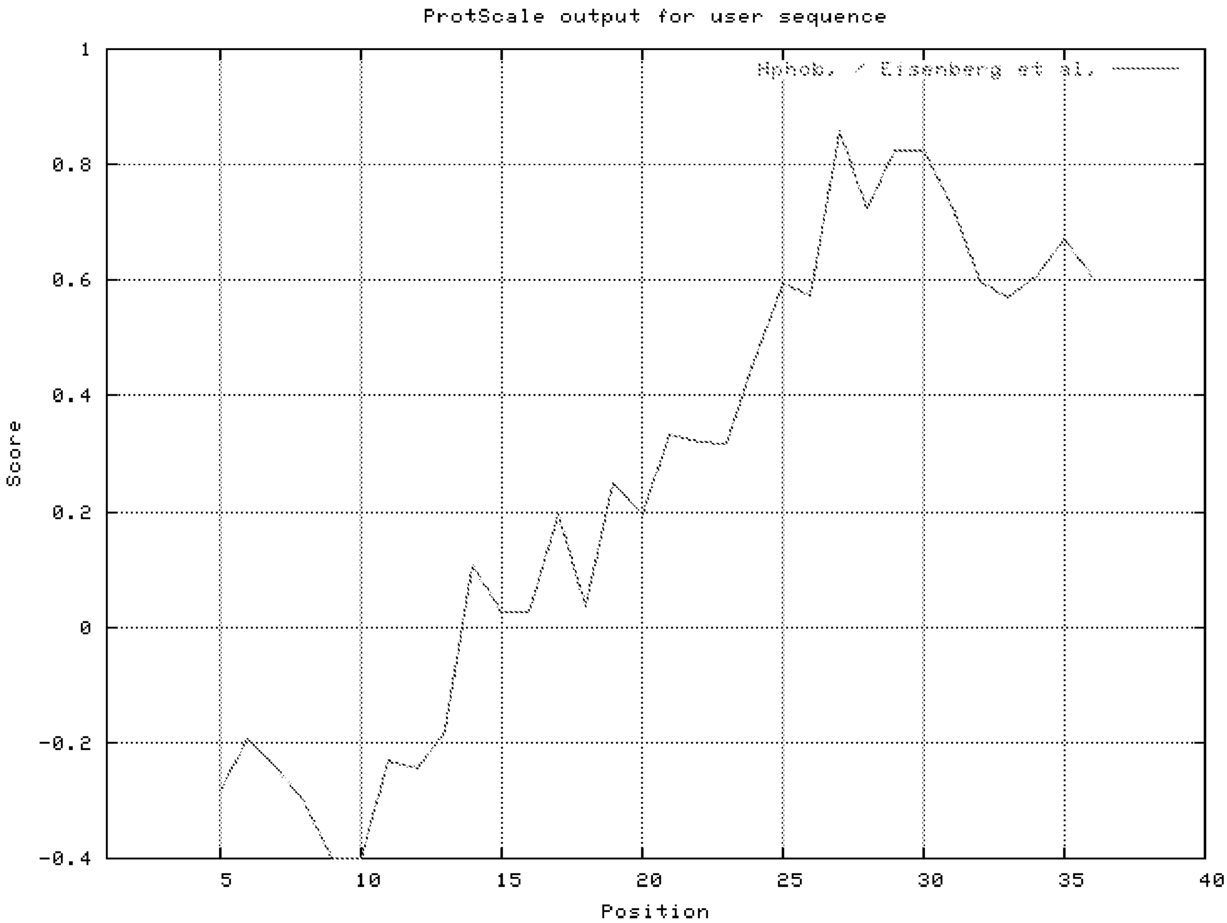
ProtScale output for user sequence
Hphob. / Eisenberg et al.
Score
Position
1
0.8
0.6
0.4
0.2
0
-0.2
-0.4
5
10
15
20
25
30
35
40

**Fig. 9**

**Sequence:Olea europaea Olive tree**

```
        10         20         30         40
KQPHYNQDKT SDFIIKLLQF LVPLFILGVA VGIHFYTKSS A
```

SEQUENCE LENGTH: 41

---

Using the scale **Hphob. / Eisenberg et al.**, the individual values for the 20 amino acids are:

```
Ala:  0.620  Arg: -2.530  Asn: -0.780  Asp: -0.900  Cys:  0.290
Gln: -0.850
Glu: -0.740  Gly:  0.480  His: -0.400  Ile:  1.380  Leu:  1.060
Lys: -1.500
Met:  0.640  Phe:  1.190  Pro:  0.120  Ser: -0.180  Thr: -0.050
Trp:  0.810
Tyr:  0.260  Val:  1.080  : -0.840  : -0.795  : -0.000
```

---

Weights for window positions 1,..,9, using **linear weight variation model:**

```
   1     2     3     4     5     6     7     8     9
 1.00  1.00  1.00  1.00  1.00  1.00  1.00  1.00  1.00
 edge                   center                  edge
```

---

MIN: -0.711
MAX: 0.972

**Fig. 9 cont.**

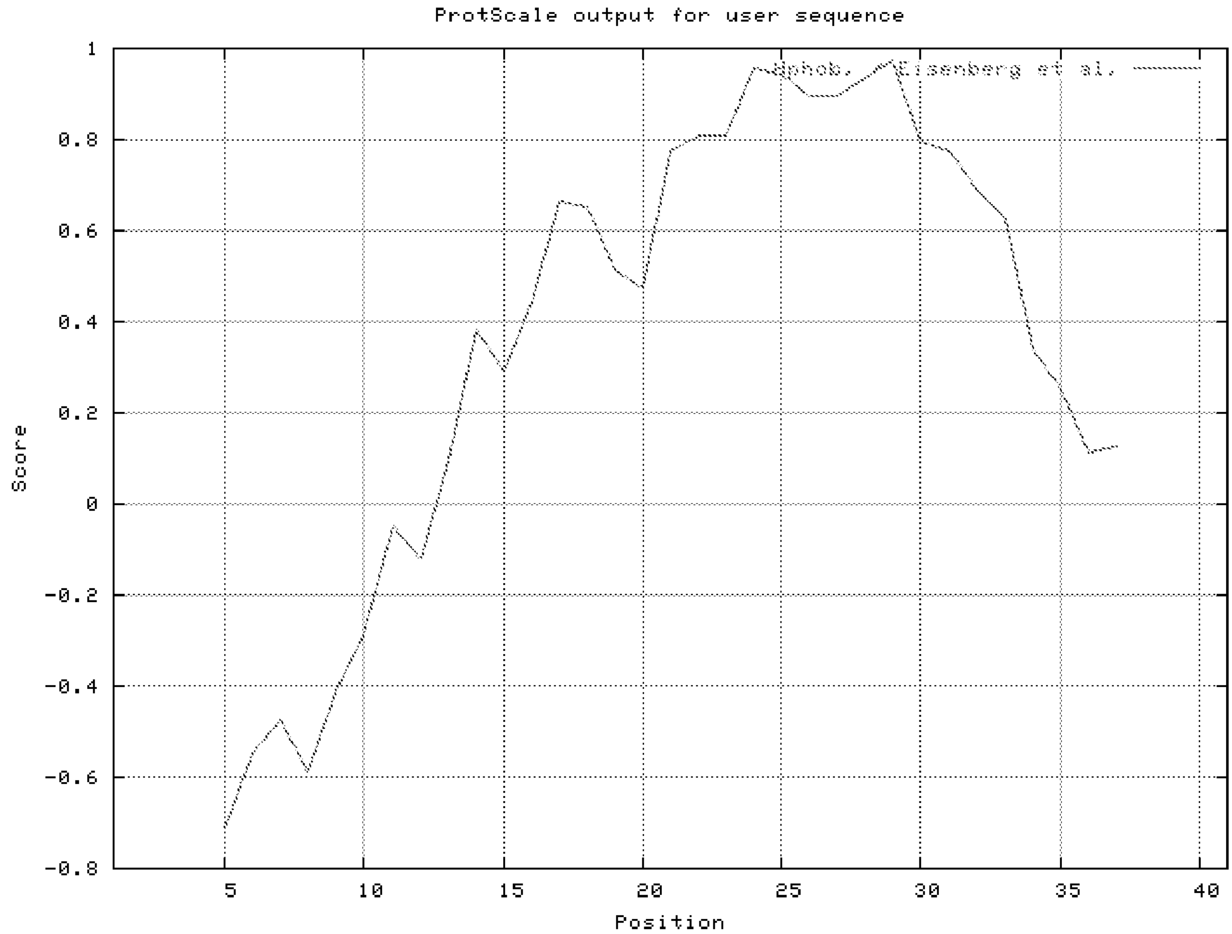
ProtScale output for user sequence
Hphob. / Eisenberg et al.
Score
Position
1
0.8
0.6
0.4
0.2
0
-0.2
-0.4
-0.6
-0.8
5
10
15
20
25
30
35
40

**Fig. 10**

**Sequence:Rhodopseudomonas palustris**

```
        10         20         30
WCGKEATEAY ATKTKGRAHT READELLPKY RIGRFAP
```

SEQUENCE LENGTH: 37

Using the scale **Hphob. / Eisenberg et al.**, the individual values for the 20 amino acids are:

```
Ala:  0.620  Arg: -2.530  Asn: -0.780  Asp: -0.900  Cys:  0.290
Gln: -0.850
Glu: -0.740  Gly:  0.480  His: -0.400  Ile:  1.380  Leu:  1.060
Lys: -1.500
Met:  0.640  Phe:  1.190  Pro:  0.120  Ser: -0.180  Thr: -0.050
Trp:  0.810
Tyr:  0.260  Val:  1.080  : -0.840  : -0.795  : -0.000
```

Weights for window positions 1,..,9, using **linear weight variation model:**

```
   1     2     3     4     5     6     7     8     9
 1.00  1.00  1.00  1.00  1.00  1.00  1.00  1.00  1.00
 edge                   center                   edge
```

MIN: -0.829
MAX: 0.000

**Fig. 10 cont.**

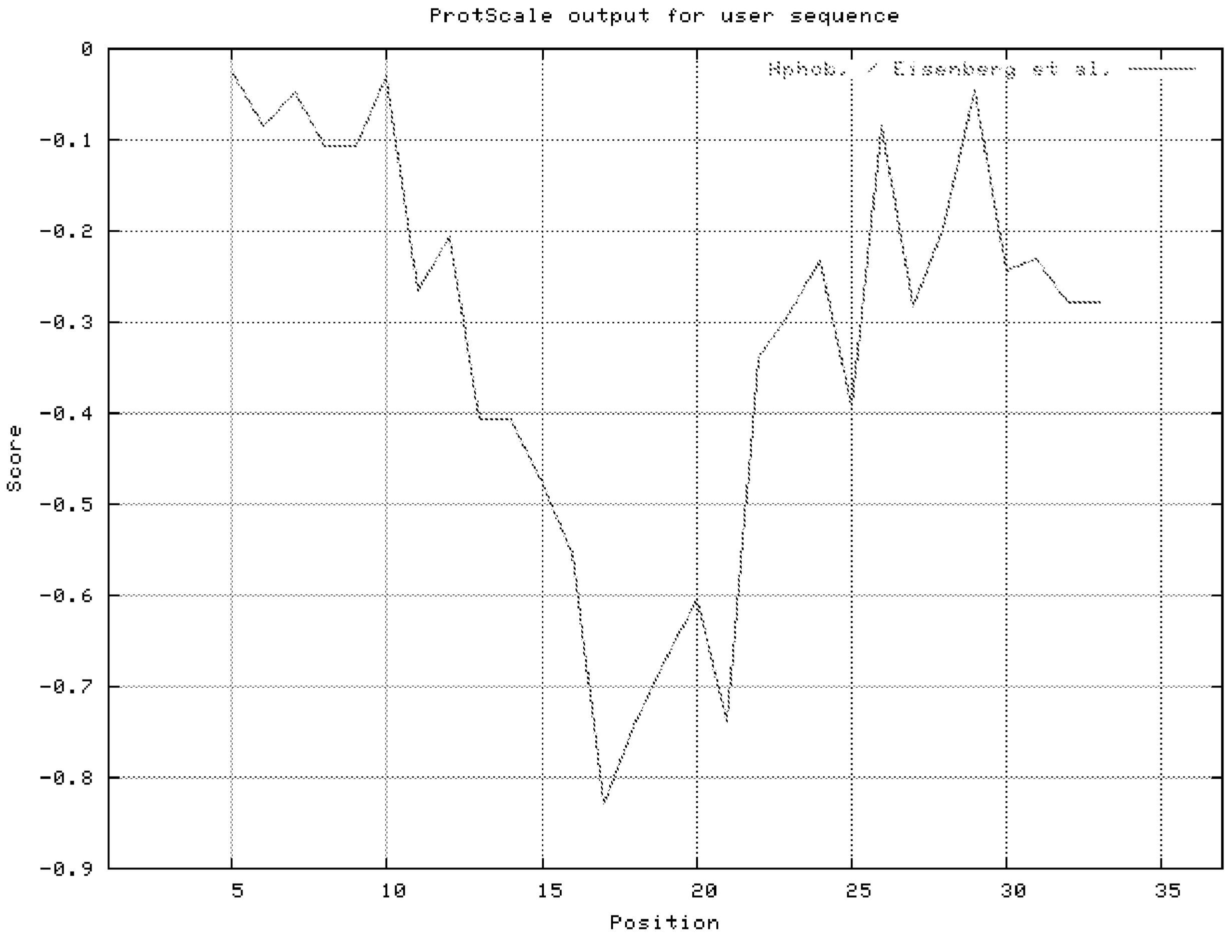
ProtScale output for user sequence
Hphob. / Eisenberg et al.
Score
Position
0
-0.1
-0.2
-0.3
-0.4
-0.5
-0.6
-0.7
-0.8
-0.9
5
10
15
20
25
30
35

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0016746 A
- US 2010226973 A
- US 7368537 B
- WO 00016746 A
- US 5767063 A, Lee
- US 6090611 A, Covacci
- US 5888530 A, Netti
- US 7561973 B
- US 7561972 B
- WO 9014837 A
- WO 9313202 A
- WO 9219265 A


### Non-patent literature cited in the description


- **FUJII et al.** *Frontiers in Bioscience,* 2008, vol. 13, 1968-1980
- **ERNST et al.** *Vaccine,* 2006, vol. 24 (24), 5158-5168
- **OLSON et al.** *Vaccine,* 2009, vol. 28 (2), 548-560
- **SHARPE et al.** *Cell,* 2010, vol. 142 (1), 158-169
- **EISENBERG et al.** *J. Mol. Biol.,* 1984, vol. 179, 125-142
- **BETAKOVA.** *Current Pharmaceutical Design,* 2007, vol. 13, 3231-3235
- **WONG.** Chemistry of Protein Conjugation and Cross-linking. CRC Press, 1991
- **BASU ; BASU.** Liposome Methods and Protocols (Methods in Molecular Biology). Humana Press, 2002
- **GREGORIADIS.** Liposome Technology. Informa HealthCare, 2006
- **CSERZO, M. ; E. WALLIN ; I. SIMON ; G. VON HEIJNE ; A. ELOFSSON.** *Protein Eng.,* 1997, vol. 10, 673-676
- **ALTSCHUL, S.F. et al.** *J. Mol. Biol.,* 1990, vol. 215, 403-410
- **KYTE ; DOOLITTLE.** *J. Mol. Biol.,* 1982, vol. 157, 105-132
- Fundamental Immunology. Raven Press, 1993
- **WILSON.** J. Immunol. Methods. 1994, vol. 175, 267-273
- **YARMUSH.** *J. Biochem. Biophys. Methods,* 1992, vol. 25, 85-97
- **WARD et al.** *Nature,* 1989, vol. 341, 544-546
- **KANN ; GOLDSTEIN.** *Proteins,* 2002, vol. 48, 367-376
- **ARSLAN et al.** *Bioinformatics,* 2001, vol. 17, 327-337
- **WOYCECHOWSKY ; RAINES.** *Curr. Opin. Chem. Biol.,* 2000, vol. 4, 533-539
- **CREIGHTON et al.** *Trends Biotechnol.,* 1995, vol. 13, 18-23
- **OLSON et al.** *Vaccine,* 2009, vol. 28, 548-560
- **SAMBROOK et al.** Molecular Cloning, A Laboratory Manual. Cold Spring Harbor Laboratory Press, 1989
- **WINNACKER.** From Genes to Clones. VCH Publishers, 1987
- **QUEEN et al.** *Immunol. Rev.,* 1986, vol. 89, 49-68
- **PUIGBO et al.** *Nucl. Acids Res.,* 2007, vol. 35 (2), W126-31
- **LORIMER et al.** *BMC Biotechnology,* 2009, vol. 9, 36
- **BATZRI et al.** *Biochem. Biophys. Acta.,* 1973, vol. 298, 1015
- **DEAMER et al.** *Biochem. Biophys. Acta.,* 1976, vol. 443, 629
- **SCHIEREN et al.** *Biochem. Biophys. Acta.,* 1978, vol. 542, 137
- **RAZIN.** *Biochem. Biophys. Acta.,* 1972, vol. 265, 24
- **MATSUMATO et al.** *J. Colloid Interface Sci.,* 1977, vol. 62, 149
- **SZOKA JR. et al.** *Proc. Natl. Acad. Sci. USA,* 1978, vol. 75, 4194
- **OLSON et al.** *Biochem. Biophys. Acta.,* 1979, vol. 557, 9
- **PICK.** *Arch. Biochem. Biophys.,* 1981, vol. 212, 186
- **STORM et al.** *PSIT,* 1998, vol. 1, 19-3
- **JEFFERY et al.** *Pharm. Res.,* 1993, vol. 10, 362
- **MCGEE et al.** *J. Microencapsul.,* 1997, vol. 14, 197
- **O'HAGAN et al.** *Vaccine,* 1993, vol. 11, 149
- Remington's Pharmaceutical Sciences. Mack Publishing Co, 1975, 1405-1412, 1461-1487

- The National Formulary XIV. American Pharmaceutical Association, 1975
- **ERNST WA ; KIM HJ ; TUMPEY TM et al.** Protection against H1, H5, H6 and H9 influenza A infection with liposomal matrix 2 epitope vaccines. *Vaccine,* 2006, vol. 24 (24), 5158-68
- **FAN J ; LIANG X ; HORTON MS et al.** Preclinical study of influenza virus A M2 peptide conjugate vaccines in mice, ferrets, and rhesus monkeys. *Vaccine,* 2004, vol. 22 (23-24), 2993-3003
- **LIU W ; LI H ; CHEN YH.** N-terminus of M2 protein could induce antibodies with inhibitory activity against influenza virus replication. *FEMS Immunol.Med Microbiol.,* 2003, vol. 35 (2), 141-6
- **NEIRYNCK S ; DEROO T ; SAELENS X ; VANLANDSCHOOT P ; JOU WM ; FIERS W.** A universal influenza A vaccine based on the extracellular domain of the M2 protein. *Nat.Med,* 1999, vol. 5 (10), 1157-63